# EUROPEAN PATENT SPECIFICATION

(11) **EP 4 441 741 B1**
(45) Date of publication and mention of the grant of the patent: **15.07.2026**
(21) Application number: 22831038.9
(22) Date of filing: 10.11.2022
(51) Int. Cl.: G16B 20/10, G16B 30/00

(54) **SYSTEMS AND METHODS FOR IDENTIFYING REGIONS OF ANEUPLOIDY IN A TISSUE**
SYSTEME UND VERFAHREN ZUR IDENTIFIZIERUNG VON ANEUPLOIDIEREGIONEN IN EINEM GEWEBE
SYSTÈMES ET PROCÉDÉS D'IDENTIFICATION DE RÉGIONS D'ANEUPLOÏDIE DANS UN TISSU

(30) Priority: 30.11.2021 US 202163284560 P
(43) Date of publication of application: 09.10.2024
(73) Proprietor: 10X Genomics, Inc., Pleasanton, CA 94588-3260 (US)
(72) Inventor: WILLIAMS, Stephen, R., Pleasanton, CA 94588-3260 (US); ROMERO RIOJAS, Juan, Pablo, Pleasanton, CA 94588-3260 (US)
(74) Representative: Grund, Martin
(86) International application number: PCT/US2022/079662
(87) International publication number: WO 2023/102313

(56) References cited:
- WO-A1-2017/161201
- WO-A1-2020/206285
- WO-A1-2021/168146
- US-A1- 2021 062 272

## Description

### TECHNICAL FIELD

This specification describes technologies relating to determining copy number variation using spatial array-based datasets.

### BACKGROUND

Cells within a tissue have differences in cell morphology and/or function due to varied analyte levels *(e.g.,* gene and/or protein expression) within the different cells. The specific position of a cell within a tissue *(e.g.,* the cell's position relative to neighboring cells or the cell's position relative to the tissue microenvironment) can affect, for instance, the cell's morphology, differentiation, fate, viability, proliferation, behavior, and signaling and cross-talk with other cells in the tissue. Variant detection in tissues containing heterogenous cell types is of interest due to its importance as a basis for understanding disease pathology and treatment.

Spatial heterogeneity has been previously studied using techniques that typically provide data for a handful of analytes in the context of intact tissue or a portion of a tissue (e.g., tissue section) or provide substantial analyte data from individual, single cells, but these techniques fail to provide information regarding the position of the single cells from the originating biological sample (e.g., tissue). There remains a need for variant detection that can provide information on the location and composition of heterogenous cell types within tissue samples and thus allow further characterization of biological conditions. US 2021/062272 A1 discloses a method of spatial mapping of haplotypes in a tissue sample, by obtaining sequence reads that comprise a molecular identifier as well as a spatial barcode that represents their position in the tissue sample. US 2021/062272 A1 also discloses counting reads per location for different loci, but does not disclose performing counts for bins that comprise multiple genomic regions.

### SUMMARY OF THE INVENTION

The invention is set out in the appended claims.

### SUMMARY OF RELATED DISCLOSURE

One aspect of the present disclosure provides a method of delineating a tissue sample of a subject into one or more regions that are characterized by an aneuploid state and one or more regions that are characterized by a diploid state, at a computer system comprising at least one processor and a memory storing at least one program for execution by the at least one processor. The method includes obtaining a plurality of nucleic acid sequence reads (e.g., comprising 10,000 or more sequence reads), in electronic form. Each respective sequence read includes (i) a corresponding spatial barcode associating the respective sequence read with a feature in a two-dimensional array of features (e.g., comprising at least 500 features) on a substrate that is in contact with the tissue sample for a period of time prior to obtaining the plurality of sequence reads and (ii) a unique molecular identifier (UMI). The plurality of sequence reads comprises sequence reads of all or portions of a plurality of nucleic acids representing 1000 or more different genomic regions in the genome of the subject across five or more different chromosomes.

The method includes using the plurality of sequence reads to determine a count data structure comprising, for each different genomic region represented by the plurality of nucleic acids, a respective UMI count for each feature in the two-dimensional array of features on the substrate having a positive UMI count. For each respective feature in the two-dimensional array of features, a respective bin count is determined for each respective bin in a plurality of bins spanning all or a portion of the genome of the subject corresponding to the respective feature. A respective copy number state of each respective feature in the two-dimensional array of features is determined using the respective bin count for each respective bin in the respective plurality of bins corresponding to the respective feature. The respective copy number state of each respective feature in the two-dimensional array of features is used to identify the one or more regions of the tissue sample that are characterized by an aneuploid state and the one or more regions of the tissue sample that are characterized by the diploid state.

In some embodiments, the obtaining the plurality of nucleic acid sequence reads comprises sequencing of the two-dimensional array of features on the substrate. In some embodiments, the obtaining the plurality of nucleic acid sequence reads comprises high-throughput sequencing.

In some embodiments, the plurality of nucleic acids represent 2000 or more different genomic regions, or between 2000 and 10,000 genomic regions.

In some embodiments, the plurality of sequence reads comprises 50,000 or more sequence reads, 100,000 or more sequence reads, or 1 x 10⁶ or more sequence reads.

In some embodiments, the corresponding spatial barcode encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x 10¹²}. In some embodiments, the corresponding spatial barcode in the respective sequence read is localized to a contiguous set of oligonucleotides within the respective sequencing read. In some such embodiments, the contiguous set of oligonucleotides is an N-mer, wherein N is an integer selected from the set {4, ..., 20}.

In some embodiments, the using the plurality of sequence reads to determine a count data structure comprises aligning each sequence read in the plurality of sequence reads to a genome of the subject. In some such embodiments, the aligning is a local alignment that aligns the respective sequence read to the genome of the subject using a scoring system that (i) penalizes a mismatch between a nucleotide in the respective sequence read and a corresponding nucleotide in the reference sequence in accordance with a substitution matrix and (ii) penalizes a gap introduced into an alignment of the sequence read and the reference sequence. In some embodiments, the local alignment is a Smith-Waterman alignment.

In some embodiments, each respective feature includes 10 or more capture probes, 20 or more capture probes, 50 or more capture probes, 100 or more capture probes, 1000 or more capture probes, 2000 or more capture probes, 10,000 or more capture probes, 100,000 or more capture probes, or 1,000,000 or more capture probes.

In some embodiments, each respective capture probe in the respective feature includes a poly-A sequence or a poly-T sequence and the corresponding spatial barcode for the respective feature that is incorporated into sequence reads in the plurality of sequence reads associated with the respective feature. In some such embodiments, each respective capture probe in the respective feature includes the same spatial barcode. In some embodiments, each respective capture probe in the respective feature includes a unique molecule identifier that is incorporated into sequence reads in the plurality of sequence reads associated with the respective capture probe.

In some embodiments, the tissue sample is a sectioned tissue sample having a depth of 100 microns or less, 50 microns on less, 20 microns or less, or 10 microns or less.

In some embodiments, the obtaining the plurality of nucleic acid sequence reads comprises genome-wide transcript coverage obtained from a gene expression workflow.

In some embodiments, the method further comprises, prior to the determining a respective bin count, transforming the count data structure using a log-Freeman-Tukey transform.

In some embodiments, the method further comprises i) clustering the count data structure across the plurality of bins to arrive at a plurality of clusters of features in the two-dimensional array of features, ii) determining a corresponding cluster consensus profile across the 1000 or more different genomic regions in the genome of the subject for each cluster in the plurality of clusters, iii) identifying a confident normal cluster in the plurality of clusters of features as a ground-state copy number based on a variance with respect to the corresponding consensus profile for the first cluster as compared to a variance with respect to the corresponding consensus profile for each other cluster in the plurality of clusters, iv) performing copy number evaluation for each respective cluster in the plurality of clusters using the corresponding consensus profile of the respective cluster, v) clustering the plurality of features in the two-dimensional array of features into a first cluster and a second cluster, vi) identifying each feature in the first cluster as one of aneuploid or diploid and each feature in the second cluster as one of aneuploid or diploid based on an enrichment within the first cluster or the second cluster of features in the confident normal cluster, and vii) marking each feature in the two-dimensional array of features as one of aneuploid or diploid based on the identifying vi).

In some embodiments, the determining the respective copy number state calculates, for each respective feature in the two-dimensional array of features, the respective copy number state, across the corresponding plurality of bins of the respective feature, using a stochastic modeling algorithm and the respective bin count for each respective bin in the respective plurality of bins corresponding to the respective feature. In some such embodiments, the stochastic modeling algorithm is a Hidden Markov Model algorithm.

In some embodiments, the determining the respective copy number state calculates, for each respective feature in the two-dimensional array of features, the respective copy number state, across the corresponding plurality of bins of the respective feature, using a circular binary segmentation algorithm and the respective bin count for each respective bin in the respective plurality of bins corresponding to the respective feature.

In some embodiments, the method further comprises merging together adjacent bins that have the same copy number state for a respective feature.

In some embodiments, the method further comprises identifying a region in the one or more regions of the tissue sample that are characterized by the aneuploid state as tumor.

In some embodiments, the method further comprises using the one or more regions of the tissue sample that are characterized by an aneuploid state and the one or more regions of the tissue sample that are characterized by the diploid state to identify a stage of a cancer in the subject.

In some embodiments, the plurality of sequence reads comprises more than 50 sequence reads for all or portions of a plurality of nucleic acids representing 5000 or more different genomic regions in the genome of the subject across ten or more different chromosomes.

Another aspect of the present disclosure provides a computer system for delineating a tissue sample of a subject into one or more regions that are characterized by an aneuploid state and one or more regions that are characterized by a diploid state, the computer system comprising one or more processors and memory addressable by the one or more processors, the memory storing at least one program for execution by the one or more processors, the at least one program comprising instructions for performing any of the methods disclosed above.

Another aspect of the present disclosure provides a non-transitory computer readable storage medium, where the non-transitory computer readable storage medium stores instructions, which when executed by a computer system, cause the computer system to perform any of the methods disclosed above.

Various embodiments of the features of this disclosure are described herein. However, it should be understood that such embodiments are provided merely by way of example, and numerous variations, changes, and substitutions can occur to those skilled in the art without departing from the scope of this disclosure. It should also be understood that various alternatives to the specific embodiments described herein are also within the scope of this disclosure.

### DESCRIPTION OF DRAWINGS

The following drawings illustrate certain embodiments of the features and advantages of this disclosure. These embodiments are not intended to limit the scope of the appended claims in any manner. Like reference symbols in the drawings indicate like elements.
**FIG. 1** is an example block diagram illustrating a computing device in accordance with some embodiments of the present disclosure.
**FIGS. 2A****,** **2B****,** **2C****,** **2D****,** **2E****,** **2F****, and** **2G** collectively illustrate non-limiting methods for delineating a tissue sample of a subject into one or more regions that are characterized by an aneuploid state and one or more regions that are characterized by a diploid state in accordance with some embodiments of the present disclosure, in which optional steps are illustrated by dashed line boxes.
**FIG. 3** shows an exemplary count data structure, in accordance with some embodiments of the present disclosure.
**FIG. 4** shows an exemplary tissue sample of a subject that is delineated into one or more regions that are characterized by an aneuploid state and one or more regions that are characterized by a diploid state, in accordance with an embodiment of the present disclosure.
**FIGS. 5A****,** **5B****,** **5C****,** **5D****,** **5E****, and** **5F** show exemplary tissue samples that are delineated into one or more regions that are characterized by an aneuploid state and one or more regions that are characterized by a diploid state, in accordance with some embodiments of the present disclosure.
**FIGS. 6A****,** **6B****,** **6C** **and** **6D** show an exemplary tissue sample that is delineated into one or more regions characterized by an aneuploid state and one or more regions characterized by a diploid state, in accordance with some embodiments of the present disclosure.
**FIGS. 7A****,** **7B****,** **7C** **and** **7D** show an exemplary tissue sample that is delineated into one or more regions characterized by an aneuploid state and one or more regions characterized by a diploid state, in accordance with some embodiments of the present disclosure.

### DETAILED DESCRIPTION

### Introduction.

Methods for studying spatial heterogeneity in developing systems include RNA hybridization, immunohistochemistry, fluorescent reporters, purification or induction of pre-defined subpopulations, and subsequent genomic profiling (e.g., RNA-seq). Such approaches, however, can rely on a relatively small set of pre-defined markers, introducing selection bias and limiting discovery. For instance, traditional spatial RNA assays may rely on staining for a limited number of RNA species. Other methods, such as single-cell RNA-sequencing, allow for deep profiling of cellular gene expression but result in a separation of cells from their native spatial context.

Accordingly, the systems and methods disclosed herein can provide spatial analyte data for a large number and/or variety of analytes within a biological sample at high spatial resolution, while retaining native spatial context. Spatial analysis systems and methods can include, for instance, the use of a capture probe including a barcode *(e.g.,* a nucleic acid sequence that provides information as to the location or position of an analyte within a cell or a tissue sample) and a capture domain that is capable of binding to an analyte *(e.g.,* a protein and/or a nucleic acid) produced by and/or present in a cell. Spatial analysis systems and methods can also include the use of a capture probe having a capture domain that captures an intermediate agent for indirect detection of an analyte. For example, the intermediate agent can include a nucleic acid sequence *(e.g.,* a barcode) associated with the intermediate agent. Detection of the intermediate agent is therefore indicative of the analyte in the cell or tissue sample.

Useful applications for spatial analysis include, for instance, evaluating whether lymphocytes have successfully infiltrated a tumor. The morphological pattern exhibited by lymphocyte infiltration into the tumor would generally be associated with a favorable prognosis whereas the inability of lymphocytes to infiltrate the tumor would generally be associated with an unfavorable prognosis. Other applications include assessing tumor metastasis or determining the overall extent of a tumor within a normal healthy tissue, particularly in cases where the tumor is small and/or difficult to discern by conventional visual methods. In some implementations, cancerous cells can be discriminated from healthy cells within a tissue sample based on their respective analyte data *(e.g.,* gene expression profiles), where analyte data can be clustered and visualized according to their original positions within the tissue. In this way, the spatial relationship (e.g., morphological pattern) of cell types in heterogeneous tissue can be used to analyze tissue samples.

Specifically, spatial patterns of genomic variations within biological samples are of particular interest with respect to their role in disease susceptibility. For instance, copy number variation (CNV) is a form of structural variation of a DNA sequence that includes multiplication and deletions of segments of DNA. CNVs are increasingly considered to be relevant in a range of clinical conditions, including Parkinson's disease, Hirschsprung, diabetes melitus, autism, Alzheimer's disease, schizophrenia, neurological disorders, and cancer. Somatic CNV plays a significant role in cancer, since oncogene activation is often attributed to chromosomal copy number amplification, and tumor suppressor gene inactivation is often causally associated with genomic deletions. Thus, identification of CNVs and detection of aneuploidy in somatic cells can have an important role in cancer prognosis and treatment improvement.

However, tumors are often characterized by high levels of heterogeneity and complexity, including the presence of normal tissue and/or multiple clonal populations. These complications hinder the detection of cancer-specific CNVs. Given the above, there is a need in the art for improved systems and methods for identifying regions of aneuploidy and diploidy in biological samples.

Accordingly, the present disclosure provides systems and methods of delineating tissue samples into regions that are characterized by an aneuploid state and regions that are characterized by a diploid state. In an exemplary embodiment, a plurality of nucleic acid sequence reads is obtained from the capture of nucleic acid analytes and/or intermediate agents by a plurality of capture probes. Each respective sequence read includes (i) a corresponding spatial barcode associating the respective sequence read with a feature in a two-dimensional array of features on a substrate (e.g., a capture spot array) and (ii) a unique molecular identifier (UMI). The plurality of sequence reads comprises sequence reads of all or portions of a plurality of nucleic acids representing different genomic regions in a genome.

The plurality of sequence reads is used to determine a count data structure comprising, for each different genomic region represented by the plurality of nucleic acids, a respective UMI count for each feature in the two-dimensional array of features on the substrate having a positive UMI count. For each respective feature in the two-dimensional array of features, a respective bin count is determined for each respective bin in a plurality of bins spanning all or a portion of the genome corresponding to the respective feature, where each bin represents multiple genomic regions, and the bin count is a sum of the UMI counts for each genomic region in the bin.

A respective copy number state of each respective feature in the two-dimensional array of features is determined using the respective bin count for each respective bin in the respective plurality of bins corresponding to the respective feature. Specifically, a baseline ploidy state is determined using the respective bin counts across the plurality of features, and features that differ from the baseline ploidy state are determined to be aneuploid. Thus, the respective copy number state of each respective feature in the two-dimensional array of features is used to identify the one or more regions of the tissue sample that are characterized by an aneuploid state and the one or more regions of the tissue sample that are characterized by the diploid state.

Advantageously, the systems and methods provided herein can be used to detect spatial patterns of genomic variations in tissues, which can be further applied to determine clinical conditions. Such applications are useful for performing digital pathology independent of or concurrent with analysis by an expert pathologist, reducing labor and training requirements and improving workflow. Moreover, CNVs can be used for detection of genomic variations even in cases where sequencing or genotyping of the genome *(e.g.,* SNP identification) cannot be performed, such as when analyzing counts of sequence reads and/or when using intermediate agents to indirectly capture nucleic acid analytes.

### Definitions.

Specific terminology is used throughout this disclosure to explain various aspects of the apparatus, systems, methods, and compositions that are described. This sub-section includes explanations of certain terms that appear in later sections of the disclosure. To the extent that the descriptions in this section are in apparent conflict with usage in other sections of this disclosure, the definitions in this section will control.

The terminology used in the present disclosure is for the purpose of describing particular embodiments only and is not intended to be limiting of the invention. It will also be understood that the term "and/or" as used herein refers to and encompasses any and all possible combinations of one or more of the associated listed items. It will be further understood that the terms "comprises" and/or "comprising," when used in this specification, specify the presence of stated integers, steps, operations, elements, and/or components, but do not preclude the presence or addition of one or more other integers, steps, operations, elements, components, and/or groups thereof.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting (the stated condition or event)" or "in response to detecting (the stated condition or event)," depending on the context.

The term "each," when used in reference to a collection of items, is intended to identify an individual item in the collection but does not necessarily refer to every item in the collection, unless expressly stated otherwise, or unless the context of the usage clearly indicates otherwise.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

### Analytes

As used herein, the term "analyte" refers to any biological substance, structure, moiety, or component to be analyzed. In some embodiments, the apparatus, systems, methods, and compositions described in this disclosure can be used to detect and analyze a wide variety of different analytes.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, *etc.),* extracellular and intracellular proteins, antibodies, and antigen binding fragments. In some embodiments, the analyte is an organelle (e.g., nuclei or mitochondria). In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, *etc.* In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a connected probe *(e.g.,* a ligation product) or an analyte capture agent *(e.g.,* an oligonucleotide-conjugated antibody), such as those described herein. In some embodiments, analytes can include one or more intermediate agents, e.g., connected probes or analyte capture agents that bind to nucleic acid, protein, or peptide analytes in a sample.

Cell surface elements corresponding to analytes can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, an extracellular matrix protein, a posttranslational modification (e.g., phosphorylation, glycosylation, ubiquitination, nitrosylation, methylation, acetylation or lipidation) state of a cell surface protein, a gap junction, and an adherens junction.

Analytes can be derived from a specific type of cell and/or a specific sub-cellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis. Examples of nucleic acid analytes include DNA analytes such as genomic DNA, methylated DNA, specific methylated DNA sequences, fragmented DNA, mitochondrial DNA, in situ synthesized PCR products, and RNA/DNA hybrids.

Examples of nucleic acid analytes also include RNA analytes such as various types of coding and non-coding RNA. Examples of the different types of RNA analytes include messenger RNA (mRNA), ribosomal RNA (rRNA), transfer RNA (tRNA), microRNA (miRNA), and viral RNA. The RNA can be a transcript (e.g., present in a tissue section). The RNA can be small *(e.g.,* less than 200 nucleic acid bases in length) or large *(e.g.,* RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be double-stranded RNA or single-stranded RNA. The RNA can be circular RNA. The RNA can be a bacterial rRNA *(e.g.,* 16s rRNA or 23s rRNA).

Additional examples of analytes include mRNA and cell surface elements (e.g., using the labelling agents described herein), mRNA and intracellular proteins (e.g., transcription factors), mRNA and cell methylation status, mRNA and accessible chromatin *(e.g.,* ATAC-seq, DNase-seq, and/or MNase-seq), mRNA and metabolites *(e.g.,* using the labelling agents described herein), a barcoded labelling agent *(e.g.,* the oligonucleotide tagged antibodies described herein) and a V(D)J sequence of an immune cell receptor *(e.g.,* a T-cell or B-cell receptor), mRNA and a perturbation agent *(e.g.,* a CRISPR crRNA/sgRNA, TALEN, zinc finger nuclease, and/or antisense oligonucleotide as described herein). In some embodiments, a perturbation agent is a small molecule, an antibody, a drug, an aptamer, a miRNA, a physical environmental (e.g., temperature change), or any other known perturbation agents.

In certain embodiments, an analyte is extracted from a live cell. Processing conditions can be adjusted to ensure that a biological sample remains live during analysis, and analytes are extracted from (or released from) live cells of the sample. Live cell-derived analytes can be obtained only once from the sample or can be obtained at intervals from a sample that continues to remain in viable condition.

In general, the systems, apparatus, methods, and compositions can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000 or more analytes present in a region of the sample or within an individual capture spot of the substrate.

In some embodiments, multiplexed assays are performed to analyze two or more different analytes. In some embodiments, more than one analyte type *(e.g.,* nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe *(e.g.,* a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) a capture handle sequence. As used herein, the term "analyte binding moiety barcode" refers to a barcode that is associated with or otherwise identifies the analyte binding moiety. As used herein, the term "analyte capture sequence" or "capture handle sequence" refers to a region or moiety configured to hybridize to, bind to, couple to, or otherwise interact with a capture domain of a capture probe. In some embodiments, a capture handle sequence is complementary to a capture domain of a capture probe. In some cases, an analyte binding moiety barcode (or portion thereof) may be removable (e.g., cleaved) from the analyte capture agent.

In some embodiments, detection of one or more analytes (e.g., protein analytes) can be performed using one or more intermediate agents. In some embodiments, the one or more intermediate agents include one or more probes. For example, in some embodiments, a respective analyte is detected indirectly by hybridizing one or more probes to the respective analyte and subsequently detecting the one or more probes after hybridization. In some embodiments, the one or more intermediate agents is a plurality of probes, and detection of analytes is performed by detecting a ligation product obtained from the plurality of probes. In some embodiments, the detection of analytes is performed using RNA-templated ligation. For instance, in some embodiments, RNA-templated ligation comprises hybridization of a set of probes to a target analyte. Each probe in the set of probes hybridizes to a sequence in the analyte that is specific to the analyte, and, upon hybridization, the set of probes is ligated to form a ligation product. In some embodiments, all or a portion of the ligation product is complementary to a capture domain of a capture probe.

In some instances, the one or more intermediate agents for a respective analyte is a pair of probes that is specific to the respective analyte. In some instances, the one or more intermediate agents for a respective analyte is a set of probes that is specific to the respective analyte. In some embodiments, each respective probe in a respective set of probes is an oligonucleotide probe.

In some embodiments, probes can be designed so that one of the probes of a pair is a probe that hybridizes to a specific sequence. Then, the other probe can be designed to detect a mutation of interest. Accordingly, in some instances, multiple second probes can be designed and can vary so that each probe binds to a specific sequence. For example, one second probe can be designed to hybridize to a wild-type sequence, and another second probe can be designed to detect a mutated sequence. Thus, in some instances, a set of probes can include one first probe and two second probes (or vice versa).

In some instances, probes can be designed so that they cover conserved regions of an analyte. Thus, in some instances, a probe (or probe pair) can hybridize to similar analytes in a biological sample *(e.g.,* to detect conserved or similar analytes) or in different biological samples (e.g., across different species).

In some embodiments, the one or more intermediate agents comprises a plurality of probe sets that covers all or nearly all of a genome (e.g., human genome). In instances where the plurality of probe sets are designed to cover an entire genome *(e.g.,* the human genome), the methods disclosed herein can detect analytes in an unbiased manner. In some instances, one probe pair *(e.g.,* oligonucleotide pair) is designed to cover one analyte *(e.g.,* transcript). In some instances, more than one probe pair *(e.g.,* a probe pair comprising a first probe and a second probe) is designed to cover one analyte (e.g., transcript). For example, at least two, three, four, five, six, seven, eight, nine, ten, or more probe sets can be used to hybridize to a single analyte. Factors to consider when designing probes is presence of variants (e.g., SNPs, mutations) or multiple isoforms expressed by a single gene. In some instances, the probe pair does not hybridize to the entire analyte *(e.g.,* a transcript), but instead the probe pair hybridizes to a portion of the entire analyte (e.g., transcript).

In some instances, the plurality of sets of probes comprises about 5000, 10,000, 15,000, 20,000, or more probe pairs *(e.g.,* a probe pair comprising a first probe and a second probe). In some instances, the plurality of sets of probes comprises about 20,000 probe pairs.

In some instances, analyte capture is performed using targeted RNA capture. Targeted RNA capture allows for examination of a subset of RNA analytes from the entire transcriptome. In some embodiments, the subset of analytes includes an individual target RNA. In some embodiments, the subset of analytes includes two or more targeted RNAs. In some embodiments, the subset of analytes includes one or more mRNAs transcribed by one or more targeted genes. In some embodiments, the subset of analytes includes one or more mRNA splice variants of one or more targeted genes. In some embodiments, the subset of analytes includes non-polyadenylated RNAs in a biological sample. In some embodiments, the subset of analytes includes detection of mRNAs having one or more single nucleotide polymorphisms (SNPs) in a biological sample.

In some embodiments, the subset of analytes includes mRNAs that mediate expression of a set of genes of interest. In some embodiments, the subset of analytes includes mRNAs that share identical or substantially similar sequences, which mRNAs are translated into polypeptides having similar functional groups or protein domains. In some embodiments, the subset of analytes includes mRNAs that do not share identical or substantially similar sequences, which mRNAs are translated into proteins that do not share similar functional groups or protein domains. In some embodiments, the subset of analytes includes mRNAs that are translated into proteins that function in the same or similar biological pathways. In some embodiments, the biological pathways are associated with a pathologic disease. For example, targeted RNA capture can detect genes that are overexpressed or underexpressed in cancer.

In some embodiments, the subset of analytes includes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, about 55, about 60, about 65, about 70, about 75, about 80, about 85, about 90, about 95, about 100, about 110, about 120, about 130, about 140, about 150, about 160, about 170, about 180, about 190, about 200, about 225, about 250, about 275, about 300, about 325, about 350, about 375, about 400, about 425, about 450, about 475, about 500, about 600, about 700, about 800, about 900, or about 1000 analytes.

In some instances, the methods disclosed herein can detect the abundance and location of at least 5,000, 10,000, 15,000, 20,000, or more different analytes.

In some embodiments, the subset of analytes detected by targeted RNA capture methods provided herein includes a large proportion of the transcriptome of one or more cells. For example, the subset of analytes detected by targeted RNA capture methods provided herein can include at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more of the mRNAs present in the transcriptome of one or more cells.

In some instances, the probes are DNA probes. In some instances, the probes are diribo-containing probes.

Additional examples of analytes suitable for use in the present disclosure are described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

Examples of RNA-templated ligation suitable for use in the present disclosure are described in U.S. Provisional Patent Application No. 62/952,736, filed December 23, 5 2019; U.S. Provisional Patent Application No. 62/969,458, filed February 3, 2020; U.S. Provisional Patent Application No. 63/087,061, filed October 2, 2020; U.S. Provisional Patent Application No. 63/108,088, filed October 30, 2020; PCT Application No. PCT/US2020/066720, filed December 22, 2020; and U.S. Patent Application No. 17/220,534, filed April 1, 2021.

### Barcodes

As used herein, the term "barcode" refers to a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes.

Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

Barcodes can spatially-resolve molecular components found in biological samples, for example, a barcode can be or can include a "spatial barcode". In some embodiments, a barcode includes both a UMI and a spatial barcode. In some embodiments the UMI and barcode are separate entities. In some embodiments, a barcode includes two or more sub-barcodes that together function as a single barcode. For example, a polynucleotide barcode can include two or more polynucleotide sequences (e.g., sub-barcodes) that are separated by one or more non-barcode sequences.

Barcodes suitable for use in the present disclosure are further described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Biological samples

As used herein, the term "sample" or "biological sample" refers to any material obtained from a subject for analysis using any of a variety of techniques including, but not limited to, biopsy, surgery, and laser capture microscopy (LCM), and generally includes cells and/or other biological material from the subject. In addition to the subjects described above, a biological sample can also be obtained from non-mammalian organisms (e.g., plants, insects, arachnids, nematodes, fungi, amphibians, and fish. A biological sample can be obtained from a prokaryote such as a bacterium, *e.g., Escherichia coli, Staphylococci* or *Mycoplasma pneumoniae;* archaea; a virus such as Hepatitis C virus or human immunodeficiency virus; or a viroid. A biological sample can also be obtained from a eukaryote, such as a patient derived organoid (PDO) or patient derived xenograft (PDX). The biological sample can include organoids, a miniaturized and simplified version of an organ produced *in vitro* in three dimensions that shows realistic micro-anatomy. Organoids can be generated from one or more cells from a tissue, embryonic stem cells, and/or induced pluripotent stem cells, which can self-organize in three-dimensional culture owing to their self-renewal and differentiation capacities. In some embodiments, an organoid is a cerebral organoid, an intestinal organoid, a stomach organoid, a lingual organoid, a thyroid organoid, a thymic organoid, a testicular organoid, a hepatic organoid, a pancreatic organoid, an epithelial organoid, a lung organoid, a kidney organoid, a gastruloid, a cardiac organoid, or a retinal organoid. Subjects from which biological samples can be obtained can be healthy or asymptomatic individuals, individuals that have or are suspected of having a disease (e.g., cancer) or a pre-disposition to a disease, and/or individuals that are in need of therapy or suspected of needing therapy.

The biological sample can include any number of macromolecules, for example, cellular macromolecules and organelles (e.g., mitochondria and nuclei). The biological sample can be a nucleic acid sample and/or protein sample. The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions and/or disaggregated cells.

Cell-free biological samples can include extracellular polynucleotides. Extracellular polynucleotides can be isolated from a bodily sample, e.g., blood, plasma, serum, urine, saliva, mucosal excretions, sputum, stool, and tears.

Biological samples can be derived from a homogeneous culture or population of the subjects or organisms mentioned herein or alternatively from a collection of several different organisms, for example, in a community or ecosystem.

Biological samples can include one or more diseased cells. A diseased cell can have altered metabolic properties, gene expression, protein expression, and/or morphologic characteristics. Examples of diseases include inflammatory disorders, metabolic disorders, nervous system disorders, and cancer. Cancer cells can be derived from solid tumors, hematological malignancies, cell lines, or obtained as circulating tumor cells.

Biological samples can also include fetal cells. For example, a procedure such as amniocentesis can be performed to obtain a fetal cell sample from maternal circulation. Sequencing of fetal cells can be used to identify any of a number of genetic disorders, including, e.g., aneuploidy such as Down's syndrome, Edwards syndrome, and Patau syndrome. Further, cell surface elements of fetal cells can be used to identify any of a number of disorders or diseases.

Biological samples can also include immune cells. Sequence analysis of the immune repertoire of such cells, including genomic, proteomic, and cell surface elements, can provide a wealth of information to facilitate an understanding the status and function of the immune system. Examples of immune cells in a biological sample include, but are not limited to, B cells, T cells (e.g., cytotoxic T cells, natural killer T cells, regulatory T cells, and T helper cells), natural killer cells, cytokine induced killer (CIK) cells, myeloid cells, such as granulocytes (basophil granulocytes, eosinophil granulocytes, neutrophil granulocytes/hyper-segmented neutrophils), monocytes/macrophages, mast cells, thrombocytes/megakaryocytes, and dendritic cells.

As discussed above, a biological sample can include a single analyte of interest, or more than one analyte of interest.

A variety of steps can be performed to prepare a biological sample for analysis. Except where indicated otherwise, the preparative steps for biological samples can generally be combined in any manner to appropriately prepare a particular sample for analysis.

For instance, in some embodiments, the biological sample is a tissue section. In some embodiments, the biological sample is prepared using tissue sectioning. A biological sample can be harvested from a subject (e.g., via surgical biopsy, whole subject sectioning, grown *in vitro (e.g.,* patient derived tumor(s) or patient derived organoid(s)) on a growth substrate or culture dish as a population of cells, or prepared for analysis as a tissue slice or tissue section). Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material. The thickness of the tissue section can be a fraction of *(e.g.,* less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, *e.g.,* 10-20 micrometers thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 11, 12, 13, 14, 15, 20, 30, 40, or 50 micrometers. Thicker sections can also be used if desired or convenient, e.g., at least 70, 80, 90, or 100 micrometers or more. Typically, the thickness of a tissue section is between 1-100 micrometers, 1-50 micrometers, 1-30 micrometers, 1-25 micrometers, 1-20 micrometers, 1-15 micrometers, 1-10 micrometers, 2-8 micrometers, 3-7 micrometers, or 4-6 micrometers, but as mentioned above, sections with thicknesses larger or smaller than these ranges can also be analyzed.

In some embodiments, a tissue section is a similar size and shape to a substrate *(e.g.,* the first substrate and/or the second substrate). In some embodiments, a tissue section is a different size and shape from a substrate. In some embodiments, a tissue section is on all or a portion of the substrate. In some embodiments, several biological samples from a subject are concurrently analyzed. For instance, in some embodiments several different sections of a tissue are concurrently analyzed. In some embodiments, 1, 2, 3, 4, 5, 6, 7, 8, 9, 10, 11, 12, 13, 14, 15, 16, 17, 18, 19, or 20 different biological samples from a subject are concurrently analyzed. For example, in some embodiments, 1, 2, 3, 4, 5, 6, 7, 8, 9, 10, 11, 12, 13, 14, 15, 16, 17, 18, 19, or 20 different tissue sections from a single biological sample from a single subject are concurrently analyzed. In some embodiments, one or more images are acquired of each such tissue section.

In some embodiments, a tissue section on a substrate is a single uniform tissue section. In some embodiments, multiple tissue sections are on a substrate. In some such embodiments, a single capture area can contain multiple tissue sections, where each tissue section is obtained from either the same biological sample and/or subject or from different biological samples and/or subjects. In some embodiments, a tissue section is a single tissue section that comprises one or more regions where no cells are present (e.g., holes, tears, or gaps in the tissue). Thus, in some embodiments, such as the above, an image of a tissue section on a substrate can contain regions where tissue is present and regions where tissue is not present.

Additional examples of tissue samples are catalogued, for example, in 10X, 2019, "Visium Spatial Gene Expression Solution," and in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analyzed successively to obtain three-dimensional information about the biological sample.

In some embodiments, a biological sample is prepared using one or more steps including, but not limited to, freezing, fixation, embedding, formalin fixation and paraffin embedding, hydrogel embedding, biological sample transfer, isometric expansion, cell disaggregation, cell suspension, cell adhesion, permeabilization, lysis, protease digestion, selective permeabilization, selective lysis, selective enrichment, enzyme treatment, library preparation, and/or sequencing pre-processing. Methods for biological sample preparation that are contemplated in the present disclosure are described in further detail in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

In some embodiments, a biological sample is prepared by staining. To facilitate visualization, biological samples can be stained using a wide variety of stains and staining techniques. In some embodiments, for example, a sample can be stained using any number of biological stains, including but not limited to, acridine orange, Bismarck brown, carmine, Coomassie blue, cresyl violet, DAPI, eosin, ethidium bromide, acid fuchsine, hematoxylin, Hoechst stains, iodine, methyl green, methylene blue, neutral red, Nile blue, Nile red, osmium tetroxide, propidium iodide, rhodamine, safranin, or a combination thereof.

The sample can be stained using known staining techniques, including Can-Grunwald, Giemsa, hematoxylin and eosin (H&E), Jenner's, Leishman, Masson's trichrome, Papanicolaou, Romanowsky, silver, Sudan, Wright's, and/or Periodic Acid Schiff (PAS) staining techniques. PAS staining is typically performed after formalin or acetone fixation.

In some embodiments, the sample is stained using a detectable label *(e.g.,* radioisotopes, fluorophores, chemiluminescent compounds, bioluminescent compounds, and dyes). In some embodiments, a biological sample is stained using only one type of stain or one technique. In some embodiments, staining includes biological staining techniques such as H&E staining. In some embodiments, staining includes identifying analytes using fluorescently-labeled antibodies. In some embodiments, a biological sample is stained using two or more different types of stains, or two or more different staining techniques. For example, a biological sample can be prepared by staining and imaging using one technique *(e.g.,* H&E staining and bright-field imaging), followed by staining and imaging using another technique (e.g., IHC/IF staining and fluorescence microscopy) for the same biological sample.

In some embodiments, biological samples can be destained. Methods of destaining or discoloring a biological sample are known in the art, and generally depend on the nature of the stain(s) applied to the sample. For example, H&E staining can be destained by washing the sample in HCl, or any other low pH acid *(e.g.,* selenic acid, sulfuric acid, hydroiodic acid, benzoic acid, carbonic acid, malic acid, phosphoric acid, oxalic acid, succinic acid, salicylic acid, tartaric acid, sulfurous acid, trichloroacetic acid, hydrobromic acid, hydrochloric acid, nitric acid, orthophosphoric acid, arsenic acid, selenous acid, chromic acid, citric acid, hydrofluoric acid, nitrous acid, isocyanic acid, formic acid, hydrogen selenide, molybdic acid, lactic acid, acetic acid, carbonic acid, hydrogen sulfide, or combinations thereof). In some embodiments, destaining can include 1, 2, 3, 4, 5, or more washes in a low pH acid *(e.g.,* HCl). In some embodiments, destaining can include adding HCl to a downstream solution (e.g., permeabilization solution). In some embodiments, destaining can include dissolving an enzyme used in the disclosed methods (e.g., pepsin) in a low pH acid *(e.g.,* HCl) solution. In some embodiments, after destaining hematoxylin with a low pH acid, other reagents can be added to the destaining solution to raise the pH for use in other applications. For example, SDS can be added to a low pH acid destaining solution in order to raise the pH as compared to the low pH acid destaining solution alone. As another example, in some embodiments, one or more immunofluorescence stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., 2017, J. Histochem. Cytochem. 65(8): 431-444, Lin et al., 2015, Nat Commun. 6:8390, Pirici et al., 2009, J. Histochem. Cytochem. 57:567-75, and Glass et al., 2009, J. Histochem. Cytochem. 57 899-905.

In some embodiments, the biological sample can be attached to a substrate (e.g., a slide and/or a chip). Examples of substrates suitable for this purpose are described in detail elsewhere herein *(see,* for example, the section entitled "Definitions: Substrates," below). Attachment of the biological sample can be irreversible or reversible, depending upon the nature of the sample and subsequent steps in the analytical method.

In certain embodiments, the sample can be attached to the substrate reversibly by applying a suitable polymer coating to the substrate and contacting the sample to the polymer coating. The sample can then be detached from the substrate using an organic solvent that at least partially dissolves the polymer coating. Hydrogels are examples of polymers that are suitable for this purpose. More generally, in some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

Biological samples contemplated for use in the present disclosure are further described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Capture probes

A "capture probe," also interchangeably referred to herein as a "probe," refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe is a conjugate (e.g., an oligonucleotide-antibody conjugate). In some embodiments, the capture probe includes a barcode *(e.g.,* a spatial barcode and/or a unique molecular identifier (UMI)) and a capture domain.

In some embodiments, the capture probe is optionally coupled to a capture spot *(e.g.,* a probe spot and/or a feature 142), for instance, by a cleavage domain, such as a disulfide linker.

The capture probe can include functional sequences that are useful for subsequent processing, which can include a sequencer specific flow cell attachment sequence, *e.g.,* a P5 sequence, and/or sequencing primer sequences, e.g., an R1 primer binding site, an R2 primer binding site. In some embodiments, a sequencer specific flow cell attachment sequence is a P7 sequence and sequencing primer sequence is a R2 primer binding site.

A barcode *(e.g.,* a spatial barcode 124) can be included within the capture probe for use in barcoding the target analyte. The functional sequences can be selected for compatibility with a variety of different sequencing systems, e.g., 454 Sequencing, Ion Torrent Proton or PGM, Illumina sequencing instruments, PacBio, Oxford Nanopore, *etc.,* and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the barcode and/or functional sequences (e.g., flow cell attachment sequence and/or sequencing primer sequences) can be common to all of the probes attached to a given capture spot. The barcode can also include a capture domain to facilitate capture of a target analyte.

The capture probe can include one or more (e.g., two or more, three or more, four or more, five or more) unique molecular identifiers (UMIs). A unique molecular identifier is a contiguous nucleic acid segment or two or more non-contiguous nucleic acid segments that function as a label or identifier for a particular analyte, or for a capture probe that binds a particular analyte (e.g., via the capture domain). A UMI can be unique. A UMI can include one or more specific polynucleotides sequences, one or more random nucleic acid and/or amino acid sequences, and/or one or more synthetic nucleic acid and/or amino acid sequences. In some embodiments, the UMI is a nucleic acid sequence that does not substantially hybridize to analyte nucleic acid molecules in a biological sample. In some embodiments, the UMI has less than 80% sequence identity (e.g., less than 70%, 60%, 50%, or less than 40% sequence identity) to the nucleic acid sequences across a substantial portion *(e.g.,* 80% or more) of the nucleic acid molecules in the biological sample. In some embodiments, a UMI is attached to an analyte in a reversible or irreversible manner. In some embodiments, a UMI is added to, for example, a fragment of a DNA or RNA sample before, during, and/or after sequencing of the analyte. In some embodiments, a UMI allows for identification and/or quantification of individual sequencing-reads.

In some embodiments, after analytes from the sample have hybridized or otherwise been associated with capture probes, analyte capture agents, or other barcoded oligonucleotide sequences according to any of the methods described herein in connection with the general spatial cell-based analytical methodology, the barcoded constructs that result from hybridization/association are analyzed via sequencing to identify the analytes.

In some embodiments, where a tissue sample is barcoded directly via hybridization with capture probes or analyte capture agents hybridized, bound, or associated with either cell surfaces, or introduced into cells of the tissue sample, as described above, sequencing can be performed on the tissue sample.

A wide variety of different sequencing methods can be used to analyze barcoded analyte constructs. In general, sequenced polynucleotides can be, for example, nucleic acid molecules such as deoxyribonucleic acid (DNA) or ribonucleic acid (RNA), including variants or derivatives thereof (e.g., single stranded DNA or DNA/RNA hybrids, and nucleic acid molecules with a nucleotide analog).

Sequencing of polynucleotides can be performed by various commercial systems. More generally, sequencing can be performed using nucleic acid amplification, polymerase chain reaction (PCR) *(e.g.,* digital PCR and droplet digital PCR (ddPCR), quantitative PCR, real time PCR, multiplex PCR, PCR-based singleplex methods, emulsion PCR), and/or isothermal amplification.

Other examples of methods for sequencing genetic material include, but are not limited to, DNA hybridization methods (e.g., Southern blotting), restriction enzyme digestion methods, Sanger sequencing methods, next-generation sequencing methods (e.g., single-molecule real-time sequencing, nanopore sequencing, and Polony sequencing), ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and any combinations thereof.

Sequence analysis of the nucleic acid molecules (including barcoded nucleic acid molecules or derivatives thereof) can be direct or indirect. Thus, the sequence analysis substrate (which can be viewed as the molecule which is subjected to the sequence analysis step or process) can directly be the barcoded nucleic acid molecule or it can be a molecule which is derived therefrom *(e.g.,* a complement thereof). Thus, for example, in the sequence analysis step of a sequencing reaction, the sequencing template can be the barcoded nucleic acid molecule, or it can be a molecule derived therefrom. For example, a first and/or second strand DNA molecule can be directly subjected to sequence analysis (e.g., sequencing), i.e., can directly take part in the sequence analysis reaction or process *(e.g.,* the sequencing reaction or sequencing process, or be the molecule which is sequenced or otherwise identified). Alternatively, the barcoded nucleic acid molecule can be subjected to a step of second strand synthesis, or amplification, before sequence analysis (e.g., sequencing or identification by another technique). The sequence analysis substrate (e.g., template) can thus be an amplicon or a second strand of a barcoded nucleic acid molecule.

In some embodiments, the sequencing of the nucleic acid molecule sequences a captured analyte capture moiety, such as an intermediate agent. In some embodiments, the sequencing determines a sequence for a captured ligation product derived from a set of probes upon hybridization of the set of probes to a target analyte. In some embodiments, the sequencing determines a sequence for a ligation product obtained from RNA-templated ligation.

Examples of RNA-templated ligation suitable for use in the present disclosure are described in U.S. Provisional Patent Application No. 62/952,736, filed December 23, 5 2019; U.S. Provisional Patent Application No. 62/969,458, filed February 3, 2020; U.S. Provisional Patent Application No. 63/087,061, filed October 2, 2020; U.S. Provisional Patent Application No. 63/108,088, filed October 30, 2020; PCT Application No. PCT/US2020/066720, filed December 22, 2020; and U.S. Patent Application No. 17/220,534, filed April 1, 2021.

Other aspects of capture probes contemplated for use in the present disclosure are known in the art. For instance, example suitable cleavage domains are described in further detail in PCT publication 202020176788A1, entitled "Profiling of biological analytes with spatially barcoded oligonucleotide arrays. Example suitable functional domains are described in further detail in U.S. Patent Application No. 16/992,569, entitled "Systems and Methods for Using the Spatial Distribution of Haplotypes to Determine a Biological Condition," filed August 13, 2020, as well as PCT publication 202020176788A1, entitled "Profiling of biological analytes with spatially barcoded oligonucleotide arrays. Example suitable spatial barcodes and unique molecular identifiers are described in further detail in U.S. Patent Application No. 16/992,569, entitled "Systems and Methods for Using the Spatial Distribution of Haplotypes to Determine a Biological Condition," filed August 13, 2020, and PCT publication 202020176788A1, entitled "Profiling of biological analytes with spatially barcoded oligonucleotide arrays.

Capture probes contemplated for use in the present disclosure are further described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Capture spots

As used interchangeably herein, the terms "capture spot," "probe spot," "capture feature," "feature," or "capture probe plurality" refer to an entity that acts as a support or repository for various molecular entities used in sample analysis. Examples of capture spots include, but are not limited to, a bead, a spot of any two- or three-dimensional geometry (e.g., an ink jet spot, a masked spot, a square on a grid), a well, and a hydrogel pad. In some embodiments, a capture spot is an area on a substrate at which capture probes with spatial barcodes are clustered. Specific non-limiting embodiments of capture spots and substrates are further described below in the present disclosure.

In some embodiments, capture spots are directly or indirectly attached or fixed to a substrate (e.g., of a chip or a slide). In some embodiments, the capture spots are not directly or indirectly attached or fixed to a substrate, but instead, for example, are disposed within an enclosed or partially enclosed three dimensional space (e.g., wells or divots). In some embodiments, some or all capture spots in an array include a capture probe.

In some embodiments, a capture spot includes different types of capture probes attached to the capture spot. For example, the capture spot can include a first type of capture probe with a capture domain designed to bind to one type of analyte, and a second type of capture probe with a capture domain designed to bind to a second type of analyte. In general, capture spots can include one or more (e.g., two or more, three or more, four or more, five or more, six or more, eight or more, ten or more, 12 or more, 15 or more, 20 or more, 30 or more, 50 or more) different types of capture probes attached to a single capture spot.

In some embodiments, each respective probe spot in a plurality of probe spots is a physical probe spot *(e.g.,* on a substrate). In some embodiments, a respective probe spot in a plurality of probe spots is a visual representation of a physical probe spot, such as an image of the probe spot and/or a two-dimensional position of the respective probe spot in a two-dimensional spatial arrangement of the plurality of probe spots.

In some embodiments, each respective probe at each respective probe spot is associated with a unique corresponding barcode. In some embodiments, each probe spot in the plurality of probe spots has a corresponding respective barcode, where each barcode is uniquely identifiable. The location of each barcode is known with regard to each other barcode (e.g., barcodes are spatially coded). An example of such measurement techniques for spatial probe spot based sequencing is disclosed in United States Patent Application Nos. 16/992,569, entitled "Systems and Methods for Using the Spatial Distribution of Haplotypes to Determine a Biological Condition," filed August 13, 2020, and 16/951,864, entitled "Pipeline for Analysis of Analytes," filed November 18, 2020. In some embodiments, each respective probe spot comprises a plurality of corresponding probes with different corresponding barcodes.

In some embodiments, a capture spot on the array includes a bead. In some embodiments, two or more beads are dispersed onto a substrate to create an array, where each bead is a capture spot on the array.

Further details and non-limiting embodiments relating to capture spots are described in U.S. Patent Application No. 16/992,569, U.S. Patent Publication No. 20110059865A1, U.S. Provisional Application No. 62/839,346, U.S. Patent No. 9,012,022, and PCT publication 202020176788A1, entitled "Profiling of biological analytes with spatially barcoded oligonucleotide arrays"; U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Capture spot arrays

In some embodiments, capture spots are collectively positioned on a substrate. As used herein, the term "capture spot array" or "array" refers to a specific arrangement of a plurality of capture spots (also termed "features") that is either irregular or forms a regular pattern. Individual capture spots in the array differ from one another based on their relative spatial locations. In general, at least two of the plurality of capture spots in the array include a distinct capture probe *(e.g.,* any of the examples of capture probes described herein).

Arrays can be used to measure large numbers of analytes simultaneously. In some embodiments, oligonucleotides are used, at least in part, to create an array. For example, one or more copies of a single species of oligonucleotide (e.g., capture probe) can correspond to or be directly or indirectly attached to a given capture spot in the array. In some embodiments, a given capture spot in the array includes two or more species of oligonucleotides (e.g., capture probes). In some embodiments, the two or more species of oligonucleotides (e.g., capture probes) attached directly or indirectly to a given capture spot on the array include a common (e.g., identical) spatial barcode.

In some embodiments, a substrate and/or an array (e.g., two-dimensional array) comprises a plurality of capture spots. In some embodiments, a substrate and/or an array includes between 4000 and 10,000 capture spots, or any range within 4000 to 6000 capture spots. For example, a substrate and/or an array includes between 4,000 to 4,400 capture spots, 4,000 to 4,800 capture spots, 4,000 to 5,200 capture spots, 4,000 to 5,600 capture spots, 5,600 to 6,000 capture spots, 5,200 to 6,000 capture spots, 4,800 to 6,000 capture spots, or 4,400 to 6,000 capture spots. In some embodiments, the substrate and/or array includes between 4,100 and 5,900 capture spots, between 4,200 and 5,800 capture spots, between 4,300 and 5,700 capture spots, between 4,400 and 5,600 capture spots, between 4,500 and 5,500 capture spots, between 4,600 and 5,400 capture spots, between 4,700 and 5,300 capture spots, between 4,800 and 5,200 capture spots, between 4,900 and 5,100 capture spots, or any range within the disclosed sub-ranges. For example, the substrate and/or array can include about 4,000 capture spots, about 4,200 capture spots, about 4,400 capture spots, about 4,800 capture spots, about 5,000 capture spots, about 5,200 capture spots, about 5,400 capture spots, about 5,600 capture spots, or about 6,000 capture spots. In some embodiments, the substrate and/or array comprises at least 4,000 capture spots. In some embodiments, the substrate and/or array includes approximately 5,000 capture spots.

Arrays suitable for use in the present disclosure are further described in PCT publication 202020176788A1, entitled "Profiling of biological analytes with spatially barcoded oligonucleotide arrays"; U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Contact

As used herein, the terms "contact," "contacted," and/ or "contacting" of a biological sample with a substrate comprising capture spots refers to any contact (e.g., direct or indirect) such that capture probes can interact (e.g., capture) with analytes from the biological sample. For example, the substrate may be near or adjacent to the biological sample without direct physical contact, yet capable of capturing analytes from the biological sample. In some embodiments the biological sample is in direct physical contact with the substrate. In some embodiments, the biological sample is in indirect physical contact with the substrate. For example, a liquid layer may be between the biological sample and the substrate. In some embodiments, the analytes diffuse through the liquid layer. In some embodiments the capture probes diffuse through the liquid layer. In some embodiments, reagents may be delivered via the liquid layer between the biological sample and the substrate. In some embodiments, indirect physical contact may be the presence of a second substrate (e.g., a hydrogel, a film, a porous membrane) between the biological sample and the first substrate comprising capture spots with capture probes. In some embodiments, reagents are delivered by the second substrate to the biological sample.

Generally, analytes can be captured when contacting a biological sample with, *e.g.,* a substrate comprising capture probes *(e.g.,* substrate with capture probes embedded, spotted, printed on the substrate or a substrate with capture spots (e.g., beads, wells) comprising capture probes). Capture can be performed using passive capture methods (e.g., gravity or diffusion) and/or active capture methods (e.g., electrophoresis).

In some embodiments, capture of analytes is facilitated by treating the biological sample with permeabilization reagents. If a biological sample is not permeabilized sufficiently, the amount of analyte captured on the substrate can be too low to enable adequate analysis. Conversely, if the biological sample is too permeable, the analyte can diffuse away from its origin in the biological sample, such that the relative spatial relationship of the analytes within the biological sample is lost. Hence, a balance between permeabilizing the biological sample enough to obtain good signal intensity while still maintaining the spatial resolution of the analyte distribution in the biological sample is desired. Methods of preparing biological samples to facilitate capture are known in the art and can be modified depending on the biological sample and how the biological sample is prepared *(e.g.,* fresh frozen, FFPE, PFA, *etc.).* Examples of analyte capture suitable for use in the present disclosure are further described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Fiducials

As used interchangeably herein, the terms "fiducial," "spatial fiducial," "fiducial marker," and "fiducial spot" generally refers to a point of reference or measurement scale. In some embodiments, imaging is performed using one or more fiducial markers, *i.e.,* objects placed in the field of view of an imaging system that appear in the image produced. Fiducial markers can include, but are not limited to, detectable labels such as fluorescent, radioactive, chemiluminescent, calorimetric, and colorimetric labels. The use of fiducial markers to stabilize and orient biological samples is described, for example, in Carter et al., Applied Optics 46:421-427, 2007).

In some embodiments, a fiducial marker can be present on a substrate to provide orientation of the biological sample. In some embodiments, a microsphere can be coupled to a substrate to aid in orientation of the biological sample. In some examples, a microsphere coupled to a substrate can produce an optical signal (e.g., fluorescence). In another example, a microsphere can be attached to a portion (e.g., corner) of an array in a specific pattern or design (e.g., hexagonal design) to aid in orientation of a biological sample on an array of capture spots on the substrate. In some embodiments, a fiducial marker can be an immobilized molecule with which a detectable signal molecule can interact to generate a signal. For example, a marker nucleic acid can be linked or coupled to a chemical moiety capable of fluorescing when subjected to light of a specific wavelength (or range of wavelengths). Such a marker nucleic acid molecule can be contacted with an array before, contemporaneously with, or after the tissue sample is stained to visualize or image the tissue section. In some embodiments, it can be advantageous to use a marker that can be detected using the same conditions (e.g., imaging conditions) used to detect an analyte of interest.

In some embodiments, fiducial markers are included to facilitate the orientation of a tissue sample or an image thereof in relation to an immobilized capture probes on a substrate. Any number of methods for marking an array can be used such that a marker is detectable only when a tissue section is imaged. For instance, a molecule, *e.g.,* a fluorescent molecule that generates a signal, can be immobilized directly or indirectly on the surface of a substrate. Markers can be provided on a substrate in a pattern (e.g., an edge, one or more rows, one or more lines, *etc.).*

In some embodiments, a fiducial marker can be stamped, attached, or synthesized on the substrate and contacted with a biological sample. Typically, an image (e.g., brightfield or fluorescence image) of the sample and the fiducial marker is taken, and the position of the fiducial marker on the substrate can be confirmed by viewing the image.

In some examples, fiducial markers can surround the array. In some embodiments the fiducial markers allow for detection of, e.g., mirroring. In some embodiments, the fiducial markers may completely surround the array (e.g., creating a fiducial board or frame). In some embodiments, the fiducial markers may not completely surround the array. In some embodiments, the fiducial markers identify the corners of the array. In some embodiments, one or more fiducial markers identify the center of the array.

Example fiducial markers suitable for use in the present disclosure are further described in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Genome

A "genome" generally refers to genomic information from a subject, which can be, for example, at least a portion of, or the entirety of, the subject's gene-encoded hereditary information. A genome can include coding regions (e.g., that code for proteins) as well as non-coding regions. A genome can include the sequences of some or all of the subject's chromosomes. For example, the human genome ordinarily has a total of 46 chromosomes. The sequences of some or all of these can constitute the genome.

### Hybridizing, Hybridize, Annealing, and Anneal

The terms "hybridizing," "hybridize," "annealing," and "anneal" are used interchangeably in this disclosure and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 80% of their individual bases are complementary to one another.

### Nucleic acid and Nucleotide

As used herein, the terms "nucleic acid" and "nucleotide" are intended to be consistent with their use in the art and to include naturally-occurring species or functional analogs thereof. Particularly useful functional analogs of nucleic acids are capable of hybridizing to a nucleic acid in a sequence-specific fashion (e.g., capable of hybridizing to two nucleic acids such that ligation can occur between the two hybridized nucleic acids) or are capable of being used as a template for replication of a particular nucleotide sequence. Naturally-occurring nucleic acids generally have a backbone containing phosphodiester bonds. An analog structure can have an alternate backbone linkage including any of a variety of those known in the art. Naturally-occurring nucleic acids generally have a deoxyribose sugar *(e.g.,* found in deoxyribonucleic acid (DNA)) or a ribose sugar *(e.g.,* found in ribonucleic acid (RNA)).

A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine (A), thymine (T), cytosine (C), or guanine (G), and a ribonucleic acid can have one or more bases selected from the group consisting of uracil (U), adenine (A), cytosine (C), or guanine (G). Useful non-native bases that can be included in a nucleic acid or nucleotide are known in the art.

### Primer

As used herein, a "primer" refers to a single-stranded nucleic acid sequence having a 3' end that can be used as a substrate for a nucleic acid polymerase in a nucleic acid extension reaction. RNA primers are formed of RNA nucleotides, and are used in RNA synthesis, while DNA primers are formed of DNA nucleotides and used in DNA synthesis. In general, primers are relatively short nucleic acid sequences, and typically include up to about 25 bases.

### Primer extension

A "primer extension" refers to any method where two nucleic acid sequences *(e.g.,* a constant region from each of two distinct capture probes) become linked *(e.g.,* hybridized) by an overlap of their respective terminal complementary nucleic acid sequences *(i.e.,* for example, 3' termini). Such linking can be followed by nucleic acid extension *(e.g.,* an enzymatic extension) of one, or both termini using the other nucleic acid sequence as a template for extension. Enzymatic extension can be performed by an enzyme including, but not limited to, a polymerase and/or a reverse transcriptase.

### Proximity ligation

A "proximity ligation" is a method of ligating two (or more) nucleic acid sequences that are in proximity with each other through enzymatic means *(e.g.,* a ligase). In some embodiments, proximity ligation can include a "gap-filling" step that involves incorporation of one or more nucleic acids by a polymerase, based on the nucleic acid sequence of a template nucleic acid molecule, spanning a distance between two nucleic acid molecules of interest *(see, e.g.,* U.S. Patent No. 7,264,929).

A wide variety of different methods can be used for proximity ligating nucleic acid molecules, including (but not limited to) "sticky-end" and "blunt-end" ligations. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation.

### Subject

As used herein, the term "subject" refers to an animal, such as a mammal (e.g., human or a non-human simian), avian (e.g., bird), or other organism, such as a plant. Examples of subjects include, but are not limited to, a mammal such as a rodent, mouse, rat, rabbit, guinea pig, ungulate, horse, sheep, pig, goat, cow, cat, dog, primate (e.g., human or non-human primate); a plant such as *Arabidopsis thaliana,* corn, sorghum, oat, wheat, rice, canola, or soybean; an algae such as *Chlamydomonas reinhardtii;* a nematode such as *Caenorhabditis elegans;* an insect such as *Drosophila melanogaster,* mosquito, fruit fly, honey bee or spider; a fish such as zebrafish; a reptile; an amphibian such as a frog or *Xenopus laevis; a Dictyostelium discoideum;* a fungi such as *Pneumocystis carinii, Takifugu rubripes,* yeast, *Saccharamoyces cerevisiae* or *Schizosaccharomyces pombe;* or a *Plasmodium falciparum.*

### Substrates

As used herein, a "substrate" refers to a support that is insoluble in aqueous liquid and that allows for positioning of biological samples, analytes, capture spots, and/or capture probes on the substrate. For instance, a substrate can be any surface onto which a sample and/or capture probes can be affixed *(e.g.,* a chip, solid array, a bead, a slide, a coverslip, a wafer, *etc.).* For the spatial analytical methods described in this section, a substrate is used to provide support to a biological sample, particularly, for example, a tissue section. In addition, in some embodiments, a substrate (e.g., the same substrate or a different substrate) functions as a support for direct or indirect attachment of capture probes to capture spots of the array.

A wide variety of different substrates can be used for the foregoing purposes. In general, a substrate can be any suitable support material. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides, *etc.),* nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate.

The substrate can also correspond to a flow cell. Flow cells can be formed of any of the foregoing materials, and can include channels that permit reagents, solvents, capture spots, and molecules to pass through the flow cell.

The substrate can generally have any suitable form or format. For example, the substrate can be flat, curved, e.g., convexly or concavely curved towards the area where the interaction between a biological sample, e.g., tissue sample, and the substrate takes place. In some embodiments, the substrate is a flat, *e.g.,* planar, chip or slide. The substrate can contain one or more patterned surfaces within the substrate (e.g., channels, wells, projections, ridges, divots, *etc.).* A substrate can be of any desired shape. For example, a substrate can be typically a flat shape *(e.g.,* a square or a rectangle). In some embodiments, a substrate structure has rounded corners *(e.g.,* for increased safety or robustness). In some embodiments, a substrate structure has one or more cut-off corners *(e.g.,* for use with a slide clamp or cross-table). In some embodiments, where a substrate structure is flat, the substrate structure can be any appropriate type of support having a flat surface *(e.g.,* a chip or a slide such as a microscope slide).

In some embodiments, a substrate includes one or more markings on a surface of the substrate, e.g., to provide guidance for correlating spatial information with the characterization of the analyte of interest. For example, a substrate can be marked with a grid of lines *(e.g.,* to allow the size of objects seen under magnification to be easily estimated and/or to provide reference areas for counting objects). In some embodiments, fiducials *(e.g.,* fiducial markers, fiducial spots, or fiducial patterns) can be included on the substrate. Fiducials can be made using techniques including, but not limited to, printing, sand-blasting, and depositing on the surface. In some embodiments, the substrate (e.g., or a bead or a capture spot on an array) includes a plurality of oligonucleotide molecules (e.g., capture probes). In some embodiments, the substrate includes tens to hundreds of thousands or millions of individual oligonucleotide molecules (e.g., at least about 10,000, 50,000, 100,000, 500,000, 1,000,000, 10,000,000, 100,000,000, 1,000,000,000 or 10,000,000,000 oligonucleotide molecules). In some embodiments, a substrate can include a substrate identifier, such as a serial number.

Further examples of substrates, including for example fiducial markers on such substrates, are disclosed in PCT publication 202020176788A1, entitled "Profiling of biological analytes with spatially barcoded oligonucleotide arrays"; U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Spatial analyte data

As used herein, "spatial analyte data" refers to any data measured, either directly, from the capture of analytes on capture probes, or indirectly, through intermediate agents disclosed herein that bind to analytes in a sample, e.g., connected probes disclosed herein, analyte capture agents or portions thereof (such as, e.g., analyte binding moieties and their associated analyte binding moiety barcodes). Spatial analyte data thus may, in some aspects, include two different labels from two different classes of barcodes. One class of barcode identifies the analyte, while the other class of barcodes identifies the specific capture probe in which an analyte was detected.

### Template switching oligonucleotide

As used herein, the term "template switching oligonucleotide" refers to an oligonucleotide that hybridizes to untemplated poly(C) nucleotides added by a reverse transcriptase (e.g., enzyme with terminal transferase activity) during reverse transcription. **In** some embodiments, the template switching oligonucleotide adds a common 5' sequence to full-length cDNA that is used for cDNA amplification. In some embodiments, a template switching oligonucleotide is added before, contemporaneously with, or after a reverse transcription, or other terminal transferase-based reaction. In some embodiments, a template switching oligonucleotide is included in the capture probe. In certain embodiments, methods of sample analysis using template switching oligonucleotides can involve the generation of nucleic acid products from analytes of the tissue sample, followed by further processing of the nucleic acid products with the template switching oligonucleotide.

Template switching oligonucleotides can include a hybridization region and a template region. The hybridization region can include any sequence capable of hybridizing to the target. In some embodiments, the hybridization region includes a series of G bases to complement the overhanging C bases at the 3' end of a cDNA molecule. The series of G bases can include 1 G base, 2 G bases, 3 G bases, 4 G bases, 5 G bases, or more than 5 G bases. The template sequence can include any sequence to be incorporated into the cDNA. In some embodiments, the template region includes at least 1 *(e.g.,* at least 2, 3, 4, 5 or more) tag sequences and/or functional sequences. In some embodiments, the template region and hybridization region are separated by a spacer.

In some embodiments, the template regions include a barcode sequence. The barcode sequence can act as a spatial barcode and/or as a unique molecular identifier. Template switching olignonucleotides can include deoxyribonucleic acids; ribonucleic acids; modified nucleic acids including 2-aminopurine, 2,6-diaminopurine (2-amino-dA), inverted dT, 5-methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, *e.g.,* UNA-A, UNA-U, UNA-C, UNA-G), Iso-dG, Iso-dC, 2' fluoro bases (e.g., Fluoro C, Fluoro U, Fluoro A, and Fluoro G), or any combination of the foregoing.

In some embodiments, the length of a template switching oligonucleotide can be at least about 2, 10, 20, 50, 75, 100, 150, 200, or 250 nucleotides or longer. In some embodiments, the length of a template switching oligonucleotide can be at most about 2, 10, 20, 50, 100, 150, 200, or 250 nucleotides or longer.

Template switching is described, for example, in U.S. Patent Application No. 16/951,843, entitled "SYSTEMS AND METHODS FOR SPATIAL ANALYSIS OF ANALYTES USING FIDUCIAL ALIGNMENT," filed November 18, 2020; U.S. Patent Application No. 16/951,854, entitled "SYSTEMS AND METHODS FOR TISSUE CLASSIFICATION," filed November 18, 2020; U.S. Patent Application No. 17/039,935, entitled "Systems and Methods for Identifying Morphological Patterns in Tissue Samples," filed September 30, 2020; and U.S. Patent Application No. 16/951,864, entitled "Spatial Analysis of Analytes," filed November 18, 2020.

### Methods for Spatial Analysis of Analytes.

Array-based spatial analysis methods involve the capture of one or more analytes and/or proxies from a biological sample to an array of capture spots on a substrate, each of which is associated with a unique spatial location on the array. Subsequent analysis of the captured analytes and/or proxies includes determining the identity of the analytes and the spatial location of each analyte within the sample. The spatial location of each analyte within the sample is determined based on the capture spot to which each analyte is bound in the array, and the capture spot's relative spatial location within the array.

There are several general methods to associate a spatial barcode with a region of a sample *(e.g.,* one or more neighboring cells in a tissue section), such that the spatial barcode identifies the region of the sample, and/or the contents thereof, as associated with a particular spatial location. One general method is to promote analytes or analyte proxies (e.g., intermediate agents and/or ligation products) out of a cell and towards a spatially-barcoded array (e.g., including spatially-barcoded capture probes). **In** some instances, the spatially-barcoded array populated with capture probes (as described further herein) is contacted with a biological sample, and the biological sample is permeabilized, allowing the analyte to migrate away from the sample and toward the array. The analyte interacts with a capture probe on the spatially-barcoded array. Once the target analyte and/or proxy is captured by the capture probe, the sample is optionally removed from the array and the capture probes are analyzed in order to obtain spatially-resolved analyte information.

Another general method is to cleave spatially-barcoded capture probes from an array and promote the spatially-barcoded capture probes towards and/or into or onto the sample. **In** an exemplary embodiment of this general method, the spatially-barcoded array populated with capture probes (as described further herein) can be contacted with a sample. The spatially-barcoded capture probes are cleaved from the array, and subsequently interact with cells within the provided sample. The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed to obtain spatially-resolved information about the tagged cell.

For instance, one exemplary workflow utilizes a spatially-barcoded array on a substrate (e.g., chip), where spatially-barcoded capture probes are clustered at areas called capture spots. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample, and the sample is permeabilized through application of permeabilization reagents. Permeabilization reagents may be administered by placing the array/sample assembly within a bulk solution. Alternatively, permeabilization reagents may be administered to the sample via a diffusion-resistant medium and/or a physical barrier such as a lid, where the sample is sandwiched between the diffusion-resistant medium and/or barrier and the array-containing substrate. The analytes can migrate toward the spatially-barcoded capture array, or the cleaved spatially-barcoded capture probes migrate toward the sample, using any number of techniques disclosed herein. For example, analyte, proxy, and/or capture probe migration can occur using a diffusion-resistant medium lid and passive migration. As another example, analyte and/or capture probe migration can be active migration, using an electrophoretic transfer system, for example. Once the analytes and/or proxies are in close proximity to the spatially-barcoded capture probes, the capture probes can hybridize or otherwise bind a target analyte and/or proxy. The sample can be optionally removed from the array.

In some embodiments, once the analytes and/or proxies are captured by the capture probes, the captured analytes and/or proxies can be spatially-barcoded by performing a reverse transcriptase first strand cDNA reaction. A first strand cDNA reaction can be optionally performed using template switching oligonucleotides. For example, a template switching oligonucleotide can hybridize to a poly(C) tail added to a 3' end of the cDNA by a reverse transcriptase enzyme. The original mRNA template and template switching oligonucleotide can then be denatured from the cDNA, allowing the spatially-barcoded capture probe to hybridize with the cDNA and a complement of the cDNA to be generated. The first strand cDNA can then be purified and collected for downstream amplification steps. The first strand cDNA can be optionally amplified using PCR, where forward and reverse primers flank the spatial barcode and target analyte or proxy regions of interest, generating a nucleic acid library associated with a particular spatial barcode. In some embodiments, the nucleic acid library preparation can be quantified and/or subjected to quality control to verify the success of the library preparation steps. In some embodiments, the cDNA comprises a sequencing by synthesis (SBS) primer sequence. The nucleic acid library amplicons are sequenced and analyzed to decode spatial information.

In some embodiments, the sample is removed from the spatially-barcoded array and the spatially-barcoded capture probes are removed from the array for barcoded analyte amplification and library preparation. Another embodiment includes performing first strand synthesis using template switching oligonucleotides on the spatially-barcoded array without cleaving the capture probes from the array. In some such embodiments, sample preparation and permeabilization are performed as described elsewhere herein. Once the capture probes capture the target analyte(s), first strand cDNA created by template switching and reverse transcriptase is then denatured, and the second strand is then extended. The second strand cDNA is then denatured from the first strand cDNA and transferred off of the array *(e.g.,* to a microtube). cDNA quantification and amplification can be performed using standard techniques discussed herein. The cDNA can then be subjected to library preparation and indexing, including fragmentation, end-repair, and A-tailing, and indexing PCR steps. The library can also be optionally tested for quality control (QC).

Yet another general method for spatial analysis comprises detecting multiple oligonucleotides that hybridize to an analyte. In some instances, for example, spatial analysis can be performed using RNA-templated ligation (RTL). Methods of RTL have been described previously. *See, e.g.,* Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain *(e.g.,* a poly(A) sequence, a non-homopolymeric sequence). After hybridization to the analyte, a ligase (e.g., T4DNA ligase or SplintR ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes *(e.g.,* instead of direct capture of an analyte) on an array, optionally amplified, and sequenced, thus determining the location and optionally the abundance of the analyte in the biological sample. Advantageously, this method allows for detection of analytes in cases where analyte transfer is difficult. For instance, biological samples prepared using fixed formalin paraffin embedding (FFPE) can experience crosslinking of RNA analytes, which can further undergo degradation over time. In some cases, such cross-linked RNA molecules migrate poorly or not at all, thus hindering analyte capture. The use of intermediate agents such as probes allows indirect capture of the RNA analytes via the migration of probes into the sample and subsequent migration of ligation products to capture probes. *See,* for example, the sections entitled "Definitions: Analytes" and "Definitions: Capture Probes," above.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes or proxies they capture are associated with specific locations in an array of features on a substrate. For example, specific spatial barcodes can be associated with specific array locations prior to array fabrication, and the sequences of the spatial barcodes can be stored *(e.g.,* in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes, analytes, and/or proxies during analysis of spatial information, the locations of the capture probes and/or analytes can be determined by referring to the stored information that uniquely associates each spatial barcode with an array feature location. In this manner, specific capture probes and captured analytes and/or proxies are associated with specific locations in the array of features. Each array feature location represents a position relative to a coordinate reference point *(e.g.,* an array location, a fiducial marker) for the array. Accordingly, each feature location has an "address" or location in the coordinate space of the array.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2020/061108 and/or U.S. Patent Application Serial No. 16/951,864.

For example, in some instances, sample preparation may include placing the sample on a slide, fixing the sample, and/or staining the sample for imaging. The stained sample can be then imaged on the array using both brightfield (to image the sample stain) and/or fluorescence (to image features) modalities. Optionally, the sample can be destained prior to permeabilization. As described above, in some embodiments, analytes are captured by any means disclosed herein. The biological sample and array are then optionally imaged a second time in one or both modalities while the analytes are reverse transcribed into cDNA, and an amplicon library is prepared and sequenced. Images are then spatially-overlaid in order to correlate spatially-identified biological sample information. When the sample and array are not imaged a second time, a spot coordinate file can be supplied instead, where the spot coordinate file replaces the second imaging step.

In some cases, a map of analyte presence and/or level can be aligned to an image of a biological sample using one or more fiducial markers, e.g., objects placed in the field of view of an imaging system which appear in the image produced, as described in WO 2020/123320, PCT Application No. 2020/061066, and/or U.S. Patent Application Serial No. 16/951,843. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

For instance, in some embodiments, a respective image is aligned to a plurality of features on a substrate by a procedure that comprises analyzing an array of pixel values in the respective image to identify a plurality of fiducial markers of the respective image. The fiducial markers are aligned with a corresponding plurality of reference fiducial markers using an alignment algorithm to obtain a transformation between the plurality of fiducial markers of the respective image and the corresponding plurality of reference fiducial markers. The transformation and a coordinate system corresponding to the plurality of reference fiducial markers are then used to locate a corresponding position in the respective image of each feature in a plurality of features.

Depending on the biological sample and the nature of analyte expression within the biological sample, morphological patterns obtained from spatial analysis of analytes can provide valuable insight into the underlying biological sample. For instance, the morphological patterns can be used to determine a disease state of the biological sample. As another example, the morphological pattern can be used to recommend a therapeutic treatment for the donor of the biological sample.

Specifically, in some embodiments, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder. Furthermore, in some embodiments, the methods and compositions described herein can allow for: identification of transcriptome and/or proteome expression profiles *(e.g.,* in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination. Further non-limiting aspects of spatial analysis methodologies and compositions are described herein.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

### Exemplary System Embodiments.

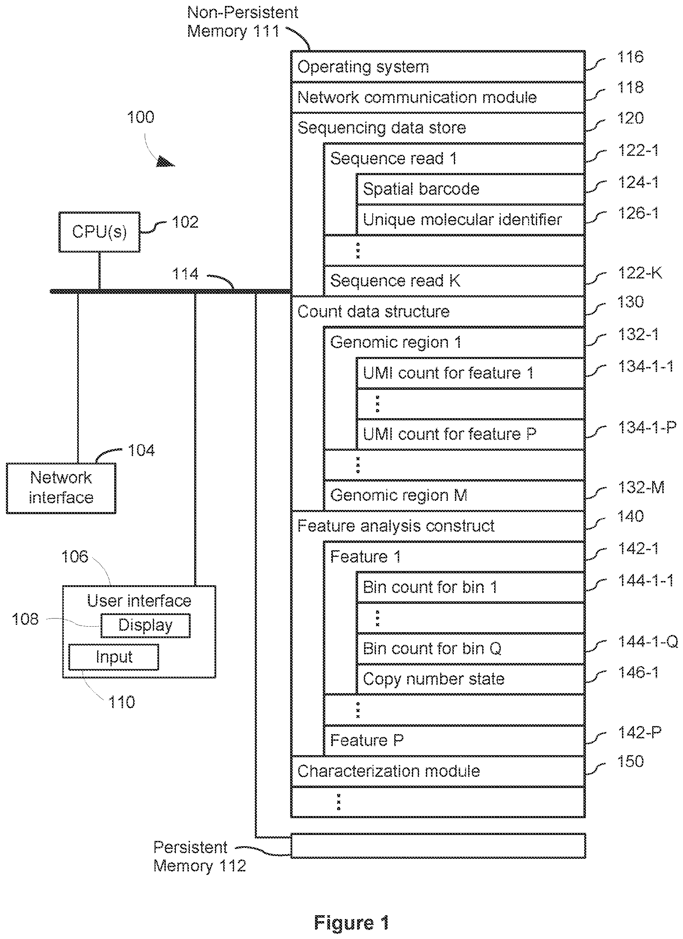

Figure 1 illustrates a block diagram illustrating a system 100 for delineating a tissue sample of a subject into one or more regions that are characterized by an aneuploid state and one or more regions that are characterized by a diploid state, in accordance with some implementations. The device 100 in some implementations includes one or more processing units (CPU(s)) 102 (also referred to as processors), one or more network interfaces 104, a user interface 106 comprising a display 108 and an input module 110, a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:
- an optional operating system 116, which includes procedures for handling various basic system services and for performing hardware dependent tasks;
- an optional network communication module (or instructions) 118 for connecting the system 100 with other devices or a communication network;
- a sequencing data store 120 comprising a plurality of nucleic acid sequence reads 122 *(e.g.,* 122-1, ...122-K), where each respective sequence read includes a corresponding spatial barcode 124 *(e.g.,* 124-1) associating the respective sequence read with a feature in a two-dimensional array of features and a unique molecular identifier 126 *(e.g.,* 126-1), and where the plurality of sequence reads 122 comprises sequence reads of all or portions of a plurality of nucleic acids representing a plurality of different genomic regions 132 *(e.g.,* 132-1, ...132-M) in the genome of the subject;
- a count data structure 130 comprising, for each different genomic region 132 *(e.g.,* 132-1) represented by the plurality of nucleic acids, a respective UMI count 134 *(e.g.,* 134-1-1, ... 134-1-P) for each feature 142 in the two-dimensional array of features;
- a feature analysis construct 140 comprising, for each respective feature 142 *(e.g.,* 142-1, ... 142-P) in the two-dimensional array of features, a respective bin count 144 *(e.g.,* 144-1-1, ... 144-1-Q) for each respective bin in a plurality of bins spanning all or a portion of the genome of the subject corresponding to the respective feature and a respective copy number state 146 *(e.g.,* 146-1) of the respective feature that is determined using the respective bin count for each respective bin in the respective plurality of bins corresponding to the respective feature; and
- a characterization module 150 for identifying the one or more regions of the tissue sample that are characterized by an aneuploid state and the one or more regions of the tissue sample that are characterized by the diploid state, using the respective copy number state of each respective feature in the two-dimensional array of features.

In some implementations, one or more of the above identified elements are stored in one or more of the previously mentioned memory devices and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations. In some implementations, the non-persistent memory 111 optionally stores a subset of the modules and data structures identified above. Furthermore, in some embodiments, the memory stores additional modules and data structures not described above. In some embodiments, one or more of the above identified elements is stored in a computer system, other than that of system 100, that is addressable by system 100 so that system 100 may retrieve all or a portion of such data when needed.

Although Figure 1 depicts a "system 100," the figures are intended more as functional description of the various features that may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112.

Plural instances may be provided for components, operations or structures described herein as a single instance. Finally, boundaries between various components, operations, and data stores may be arbitrary, and particular operations are illustrated in the context of specific illustrative configurations. Other allocations of functionality are envisioned and may fall within the scope of the implementation(s).

### Embodiments for Identifying Regions of Aneuploidy.

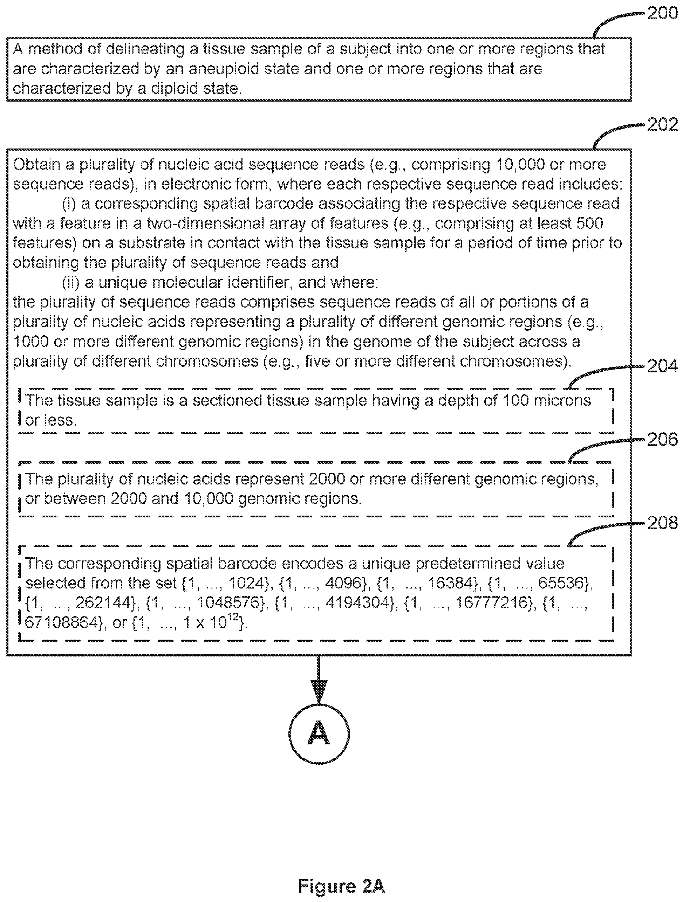

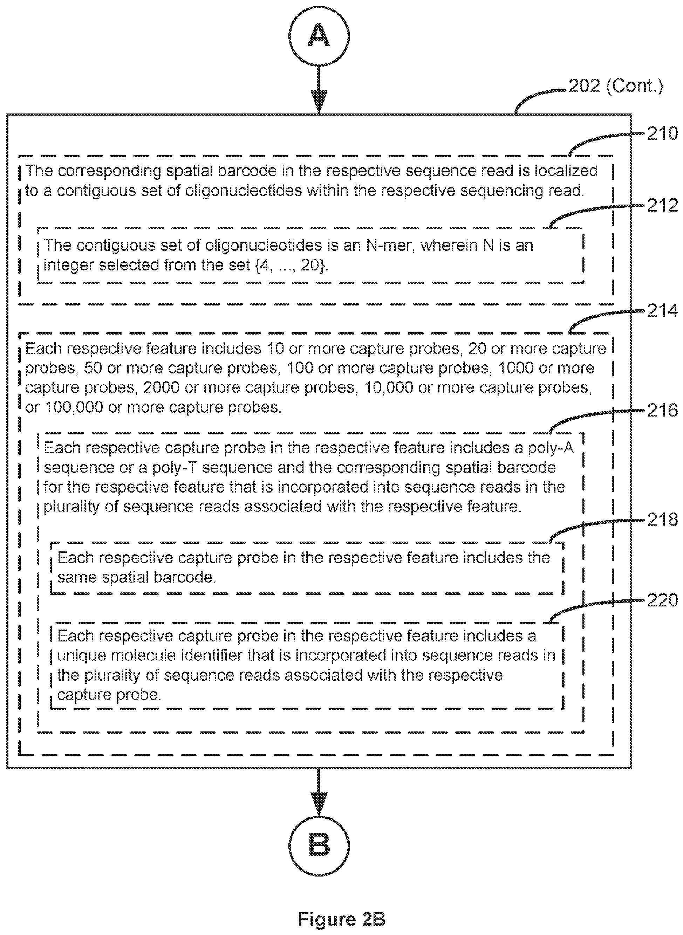

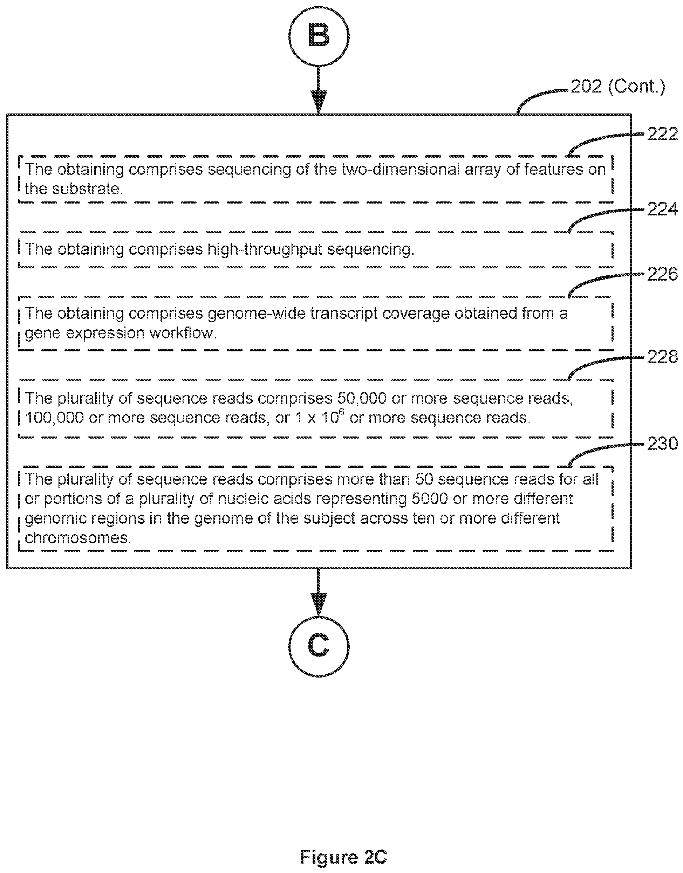

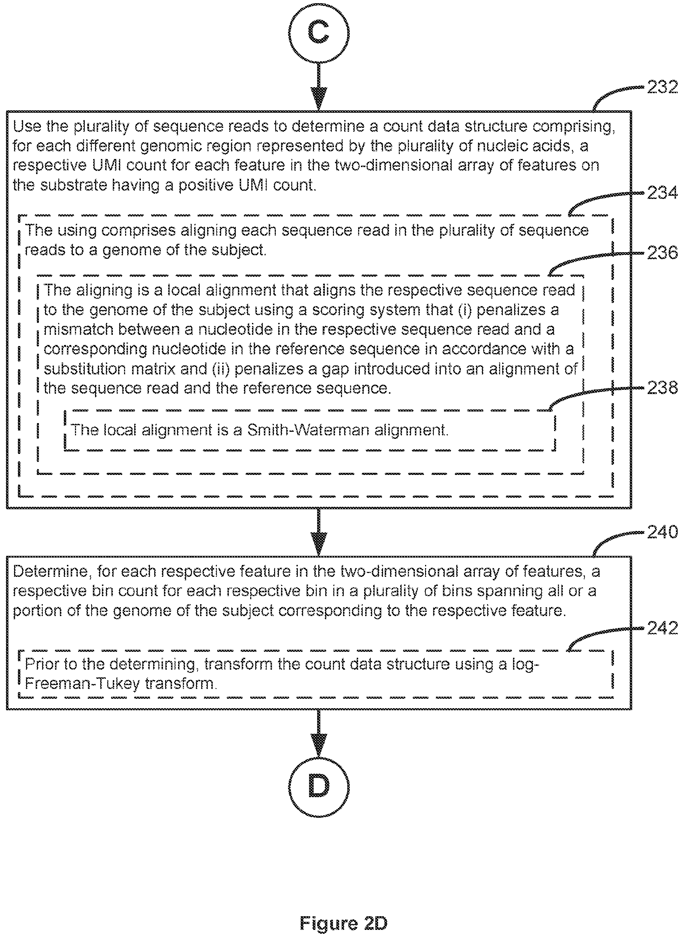

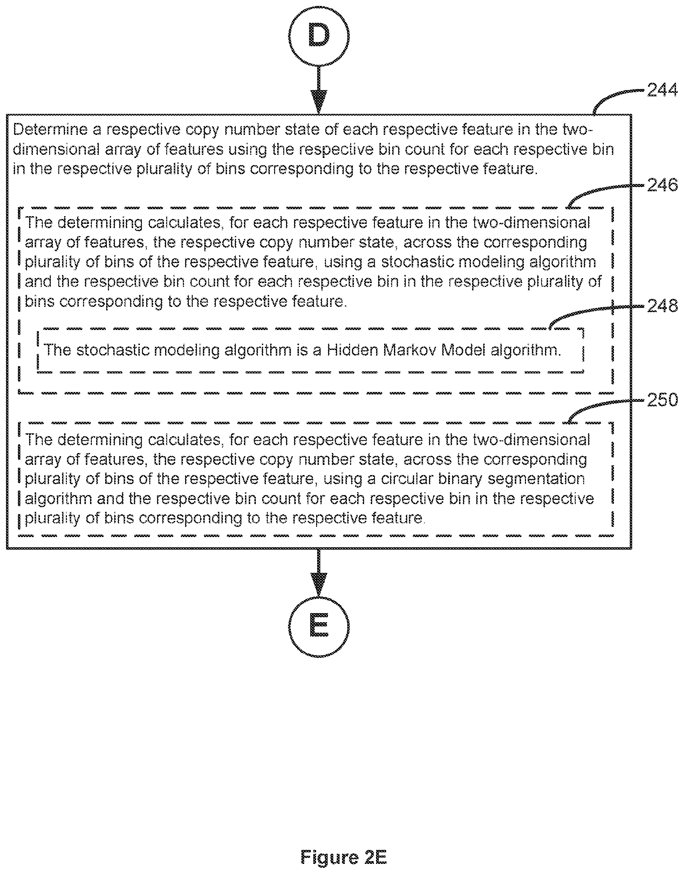

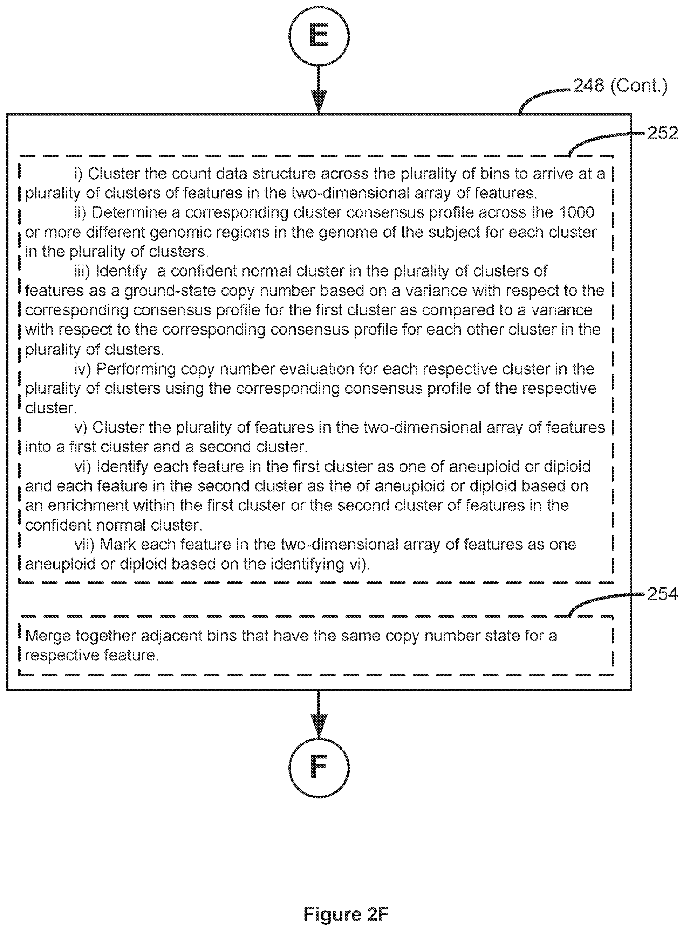

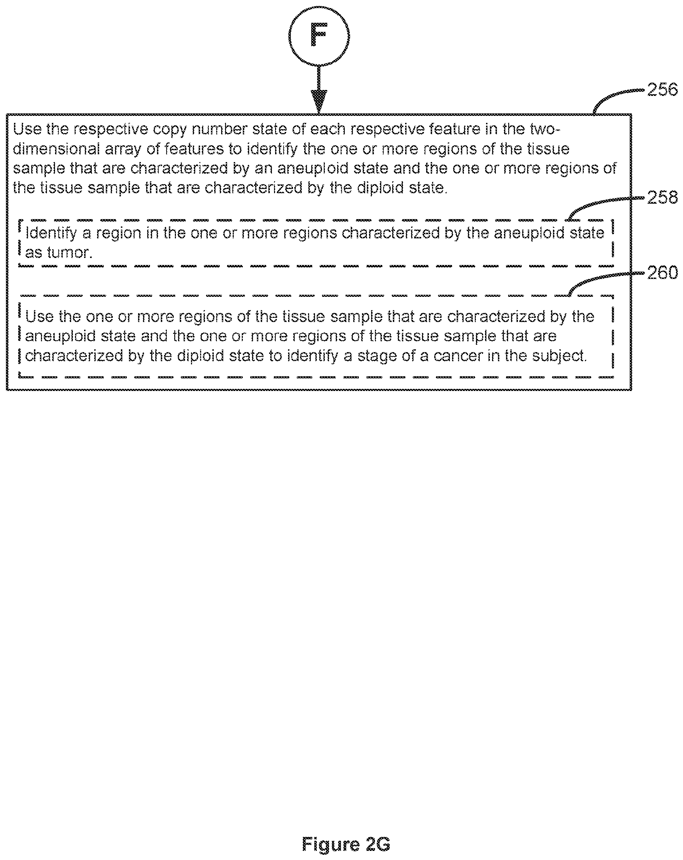

Referring to Figures 2A-G, the present disclosure provides a method 200 for delineating a tissue sample of a subject into one or more regions that are characterized by an aneuploid state and one or more regions that are characterized by a diploid state, at a computer system 100 comprising at least one processor 102 and a memory 111/112 storing at least one program for execution by the at least one processor.

Referring to Block 202, the method 200 comprises obtaining a plurality of nucleic acid sequence reads (e.g., comprising 10,000 or more sequence reads), in electronic form. Each respective sequence read includes (i) a corresponding spatial barcode associating the respective sequence read with a feature in a two-dimensional array of features comprising at least 500 features on a substrate in contact with the tissue sample for a period of time prior to obtaining the plurality of sequence reads and (ii) a unique molecular identifier. The plurality of sequence reads comprises sequence reads of all or portions of a plurality of nucleic acids representing a plurality of different genomic regions (e.g., 1000 or more different genomic regions) in the genome of the subject across a plurality of different chromosomes (e.g., five or more different chromosomes).

Non-limiting embodiments for subjects and samples, including tissue samples, contemplated for use in the present disclosure are described herein. *See,* for example, the sections entitled "Definitions: Subjects" and "Definitions: Biological samples," above.

For instance, referring to Block 204, in some embodiments, the tissue sample is a sectioned tissue sample having a depth of 100 microns or less. In some embodiments, the tissue sample is a sectioned tissue sample having a depth of at least 0.1, at least 0.2, at least 0.3, at least 0.4, at least 0.5, at least 0.7, at least 1.0, at least 1.5, at least 2, at least 3, at least 4, at least 5, at least 6, at least 7, at least 8, at least 9, at least 10, at least 11, at least 12, at least 13, at least 14, at least 15, at least 20, at least 30, at least 40, at least 50, at least 70, at least 80, at least 90, or at least 100 microns. In some embodiments, the tissue sample is a sectioned tissue sample having a depth of no more than 500, no more than 200, no more than 100, no more than 50, no more than 20, no more than 10, or no more than 5 microns. In some embodiments, the tissue sample is a sectioned tissue sample having a depth of from 1 to 100, from 1 to 50, from 10 to 30, from 5 to 25, from 2 to 20, or from 1 to 10 microns. In some embodiments, the tissue sample is a sectioned tissue sample having a depth that falls within another range starting no lower than 0.1 microns and ending no higher than 500 microns.

In some embodiments, the plurality of different genomic regions comprises one or more positions of a genome and/or nucleic acid sequences that can be mapped to a genome (*see*, *e.g*., the section entitled "Definitions: Genome," above). Accordingly, in some embodiments, the plurality of nucleic acids representing the plurality of different genomic regions include nucleic acid molecules that are derived from and/or that can be mapped to one or more genomic regions in the plurality of different genomic regions.

For instance, referring to Block 206, in some embodiments, the plurality of nucleic acids represent 2000 or more different genomic regions, or between 2000 and 10,000 genomic regions. In some embodiments, the plurality of nucleic acids represent at least 10, at least 20, at least 50, at least 100, at least 200, at least 500, at least 1000, at least 2000, at least 5000, at least 8000, at least 10,000, or at least 20,000 different genomic regions. In some embodiments, the plurality of nucleic acids represent no more than 100,000, no more than 50,000, no more than 20,000, no more than 10,000, no more than 5000, no more than 1000, no more than 500, or no more than 200 different genomic regions. In some embodiments, the plurality of nucleic acids represent from 10 to 1000, from 500 to 2000, from 1000 to 10,000, or from 200 to 20,000 different genomic regions. In some embodiments, the plurality of nucleic acids represents a plurality of different genomic regions that falls within another range starting no lower than 10 different genomic regions and ending no higher than 100,000 different genomic regions.

In some embodiments, each respective genomic region in the plurality of different genomic regions corresponds to a respective gene in a plurality of genes. In some embodiments, each respective genomic region in the plurality of different genomic regions corresponds to a respective coding sequence for one or more genes.

In some embodiments, each respective genomic region in the plurality of different genomic regions corresponds to all or a portion of a gene and/or a coding sequence. In some embodiments, each respective genomic region in the plurality of different genomic regions corresponds to one or more genes and/or coding sequences.

In some embodiments, each respective genomic region in the plurality of different genomic regions is a position in a reference sequence (*e.g.,* a locus) spanning one or more nucleotides to which an RNA transcript can be mapped. In some such embodiments, each respective nucleic acid in the plurality of nucleic acids comprises a nucleic acid sequence for an RNA transcript that maps to all or a portion of a respective genomic region in the plurality of different genomic regions. In some embodiments, the reference sequence is a genome for the subject. In some embodiments, the reference sequence is a mammalian genome (*e.g.,* a human genome or a mouse genome).

In some embodiments, each respective nucleic acid in the plurality of nucleic acids represents an analyte from the sample of the subject. In some embodiments, each respective nucleic acid in the plurality of nucleic acids represents an RNA transcript.

In some embodiments, the plurality of different genomic regions comprises a plurality of different chromosomes across five or more different chromosomes. In some embodiments, the plurality of different chromosomes includes at least 3, at least 5, at least 8, at least 10, at least 15, at least 20, or at least 30 different chromosomes. In some embodiments, the plurality of different chromosomes comprises no more than 50, no more than 30, no more than 20, no more than 10, or no more than 5 different chromosomes. In some embodiments, the plurality of different chromosomes comprises from 3 to 10, from 5 to 20, from 10 to 30, or from 5 to 40 different chromosomes. In some embodiments, the plurality of different chromosomes falls within another range starting no lower than 3 different chromosomes and ending no higher than 50 different chromosomes.

In some embodiments, each respective nucleic acid in the plurality of nucleic acids represents a different genomic region in the plurality of different genomic regions. In some embodiments, each respective nucleic acid in the plurality of nucleic acids represents all or a subset of a respective genomic region in the plurality of different genomic regions. In some embodiments, each respective nucleic acid in the plurality of nucleic acids represents a set of genomic regions in the plurality of different genomic regions.

In some embodiments, the obtaining the plurality of sequence reads comprises performing a direct capture of analytes and/or analyte capture moieties from the tissue sample, each respective nucleic acid in the plurality of nucleic acids representing the different genomic regions is an analyte and/or analyte capture moiety that is directly captured from the tissue sample (*e.g.,* an RNA transcript), and the respective sequence read comprises the nucleic acid sequence of all or portions of the respective analyte and/or analyte capture moiety. Non-limiting methods for direct analyte capture (*e.g.*, using capture probes) suitable for use in the present disclosure are described, for example, in the sections entitled "Definitions: Analytes," "Definitions: Capture Probes," and "Methods for Spatial Analysis," above.

In some embodiments, the plurality of nucleic acids representing the different genomic regions represents all or a portion of a transcriptome. In some such embodiments, each respective nucleic acid in the plurality of nucleic acids representing the different genomic regions is all or a portion of a gene and/or a transcript thereof. In some embodiments, each respective nucleic acid in the plurality of nucleic acids representing the different genomic regions comprises one or more genes and/or transcripts thereof. In some embodiments, each respective nucleic acid in the plurality of nucleic acids representing the different genomic regions comprises one or more coding sequences for a respective one or more genes.

In some embodiments, the plurality of nucleic acids representing the different genomic regions represents at least 5%, at least 10%, at least 20%, at least 30%, at least 40%, at least 50%, at least 60%, at least 70%, at least 80%, at least 90%, at least 95%, at least 98%, at least 99%, or 100% of a transcriptome. In some embodiments, the plurality of nucleic acids representing the different genomic regions represents no more than 99%, no more than 90%, no more than 80%, no more than 50%, no more than 30%, or no more than 20% of a transcriptome. In some embodiments, the plurality of nucleic acids representing the different genomic regions represents from 10% to 80%, from 50% to 99%, from 70% to 99%, or from 80% to 100% of a transcriptome. In some embodiments, the plurality of nucleic acids represents a portion of a transcriptome that falls within another range starting no lower than 5% and ending no higher than 100%. In some embodiments, the plurality of nucleic acids representing the different genomic regions represents a plurality of genes comprising at least 10, at least 50, at least 100, at least 200, at least 500, at least 1000, at least 2000, at least 5000, at least 10,000, or at least 15,000 genes. In some embodiments, the plurality of nucleic acids representing the different genomic regions represents a plurality of genes comprising no more than 30,000, no more than 20,000, no more than 10,000, no more than 5000, no more than 1000, no more than 500, or no more than 100 genes. In some embodiments, the plurality of nucleic acids representing the different genomic regions represents a plurality of genes comprising from 100 to 1000, from 500 to 5000, from 2000 to 10,000, or from 10,000 to 20,000 genes. In some embodiments, the plurality of nucleic acids representing the different genomic regions represents a plurality of genes that falls within another range starting no lower than 10 genes and ending no higher than 30,000 genes.

In some embodiments, the transcriptome is obtained from the subject. In some embodiments, the transcriptome is obtained from a mammal. In some embodiments, the transcriptome is a human transcriptome or a mouse transcriptome.

In some implementations, the obtaining the plurality of sequence reads comprises indirect capture of analytes from the tissue sample, each respective nucleic acid in the plurality of nucleic acids representing the different genomic regions corresponds to an analyte that is indirectly captured from the tissue sample (*e.g.,* an RNA transcript), and the respective sequence read comprises a nucleic acid sequence that maps to all or a portion of the respective analyte. For instance, in some embodiments, the obtaining the plurality of sequence reads comprises indirect capture of analytes using RNA templated ligation, and the respective sequence read comprises the nucleic acid sequence of an intermediate agent (*e.g.,* a probe) that maps to (*e.g.*, hybridizes to) all or a portion of the respective analyte. Non-limiting methods for indirect analyte capture including RNA templated ligation are further described, for example, in the sections entitled "Definitions: Analytes," "Definitions: Capture Probes," and "Methods for Spatial Analysis," above.

In some embodiments, each respective nucleic acid in the plurality of nucleic acids representing the different genomic regions is a probe in a plurality of probes that represents all or a portion of a transcriptome. In some such embodiments, each respective probe in the plurality of probes maps to (*e.g.*, hybridizes to) all or a portion of a gene and/or a transcript thereof. In some embodiments, each respective probe in the plurality of probes maps to (*e.g.*, hybridizes to) one or more genes and/or transcripts thereof. In some embodiments, each respective probe in the plurality of probes maps to (*e.g.*, hybridizes to) one or more coding sequences for a respective one or more genes.

In some embodiments, the plurality of probes represents (*e.g.*, targets nucleic acid sequences spanning) at least 5%, at least 10%, at least 20%, at least 30%, at least 40%, at least 50%, at least 60%, at least 70%, at least 80%, at least 90%, at least 95%, at least 98%, at least 99%, or 100% of a transcriptome. In some embodiments, the plurality of probes represents no more than 99%, no more than 90%, no more than 80%, no more than 50%, no more than 30%, or no more than 20% of a transcriptome. In some embodiments, the plurality of probes represents from 10% to 80%, from 50% to 99%, from 70% to 99%, or from 80% to 100% of a transcriptome. In some embodiments, the plurality of probes represents a portion of a transcriptome that falls within another range starting no lower than 5% and ending no higher than 100%.

In some embodiments, the plurality of probes targets a plurality of genes comprising at least 10, at least 50, at least 100, at least 200, at least 500, at least 1000, at least 2000, at least 5000, at least 10,000, or at least 15,000 genes. In some embodiments, the plurality of probes targets a plurality of genes comprising no more than 30,000, no more than 20,000, no more than 10,000, no more than 5000, no more than 1000, no more than 500, or no more than 100 genes. In some embodiments, the plurality of probes targets a plurality of genes comprising from 100 to 1000, from 500 to 5000, from 2000 to 10,000, or from 10,000 to 20,000 genes. In some embodiments, the plurality of probes targets a plurality of genes that falls within another range starting no lower than 10 genes and ending no higher than 30,000 genes.

In some embodiments, the transcriptome is obtained from the subject. In some embodiments, the transcriptome is obtained from a mammal. In some embodiments, the transcriptome is a human transcriptome or a mouse transcriptome.

In some embodiments, each respective gene in the transcriptome is represented (*e.g.,* targeted) by one or more probes. In some embodiments, each respective gene in the transcriptome is represented (*e.g.*, targeted) by a set of probes. In some embodiments, each genomic region in the plurality of genomic regions is represented (*e.g.*, targeted) by one or more probes. In some embodiments, each genomic region in the plurality of genomic regions is represented (*e.g.*, targeted) by a set of probes.

For example, in some implementations, each respective nucleic acid in the plurality of nucleic acids representing the different genomic regions corresponds to one or more probes, in a plurality of probes, that target a respective analyte in a plurality of analytes. As described further herein, in some embodiments, the plurality of probes comprises, for each respective genomic region in the plurality of genomic regions, a corresponding set of probes. Each respective probe in the corresponding set of probes for the respective genomic region maps (*e.g.*, hybridizes) to all or a portion of a respective analyte that represents the respective genomic region (*e.g.,* an RNA transcript for a respective locus in a genome). In some embodiments, each respective nucleic acid in the plurality of nucleic acids is a ligation product that is obtained by a procedure comprising (i) hybridizing each respective probe in the corresponding set of probes to the respective analyte (*e.g.*, RNA transcript) that represents the respective genomic region and (ii) ligating the corresponding set of hybridized probes. In some embodiments, each respective nucleic acid in the plurality of nucleic acids comprises a ligation product obtained from a ligation of all of the probes in a respective set of probes.

In some embodiments, the plurality of probes comprises at least 50, at least 100, at least 200, at least 500, at least 1000, at least 2000, at least 5000, at least 10,000, at least 20,000, or at least 50,000 unique sets of probes (*e.g.*, representing a respective different genomic region in the plurality of genomic regions). In some embodiments, the plurality of probes comprises no more than 100,000, no more than 50,000, no more than 30,000, no more than 20,000, no more than 10,000, no more than 5000, no more than 1000, or no more than 500 unique sets of probes. In some embodiments, the plurality of probes comprises from 1000 to 5000, from 5000 to 10,000, from 20,000 to 100,000, or from 10,000 to 40,000 unique sets of probes. In some embodiments, the plurality of probes falls within another range starting no lower than 50 unique sets of probes and ending no higher than 100,000 unique sets of probes.

In some embodiments, each genomic region in the plurality of genomic regions is represented by a corresponding one or more unique sets of probes.

In some embodiments, a corresponding set of probes for a respective genomic region comprises at least 1, at least 2, at least 3, at least 4, at least 5, at least 6, at least 7, at least 8, at least 9, or at least 10 unique probes. In some embodiments, a corresponding set of probes for a respective genomic region comprises no more than 20, no more than 10, or no more than 5 unique probes. In some embodiments, a corresponding set of probes for a respective genomic region comprises from 2 to 5, from 1 to 10, from 1 to 4, or from 3 to 15 unique probes. In some embodiments, a corresponding set of probes for a respective genomic region comprises another range of probes starting no lower than 1 unique probe and ending no higher than 20 unique probes.

In some embodiments, each respective set of probes comprises a plurality of copies for each respective unique probe. For instance, in some embodiments where a corresponding set of probes for a respective genomic region comprises a first probe *(e.g.,* a "left" probe) and a second probe *(e.g.,* a "right" probe), the corresponding set of probes comprises a first plurality of copies of the first probe and a second plurality of copies of the second probe. Accordingly, in some embodiments, each respective set of probes comprises, for each respective unique probe, at least 100,000, at least 500,000, at least 1 million, at least 2 million, at least 3 million, at least 5 million, or at least 10 million copies. In some embodiments, each respective set of probes comprises, for each respective unique probe, no more than 50 million, no more than 10 million, no more than 5 million, or no more than 2 million copies. In some embodiments, each respective set of probes comprises, for each respective unique probe, from 100,000 to 1 million copies, from 500,000 to 5 million copies, or from 1 million to 10 million copies. In some embodiments, each respective set of probes comprises, for each respective unique probe, a plurality of copies that falls within another range starting no lower than 100,000 copies and ending no higher than 50 million copies.

Non-limiting methods for intermediate agents, including probes, are further described, for example, in the sections entitled "Definitions: Analytes," "Definitions: Capture Probes," and "Methods for Spatial Analysis," above.

In some embodiments, capture probes are used to capture the plurality of nucleic acids representing the plurality of different genomic regions. For instance, in some embodiments, a plurality of capture probes are designed to hybridize to all or a portion of the nucleic acids.

In some embodiments, where the plurality of nucleic acids comprises analytes derived directly from the sample of the subject (*e.g.*, RNA transcripts), the capture probes are designed to hybridize to all or a portion of each respective analyte (*e.g.,* all or a portion of the nucleic acid sequence of the RNA transcript).

In some embodiments, where the plurality of nucleic acids comprises intermediate agents corresponding to analytes that are indirectly captured from the tissue sample (*e.g.*, probes that hybridize to RNA transcripts and/or are ligated), the capture probes are designed to hybridize to all or a portion of each respective intermediate agent.

Non-limiting methods for obtaining analyte data using capture probes and embodiments thereof are further described, for example, in the sections entitled "Definitions: Analytes," "Definitions: Barcodes," "Definitions: Capture Probes," "Definitions: Capture Spots," "Definitions: Capture Spot Arrays," and "Methods for Spatial Analysis," above. For example, in some embodiments, the method comprises capturing the plurality of nucleic acids representing the plurality of different genomic regions using a plurality of capture probes, each respective capture probe comprising a spatial barcode. Generally, as further described herein, barcodes are feature-specific (*e.g.*, specific to a capture probe and/or specific to a feature on a substrate to which multiple capture probes are affixed). Barcodes therefore allow for the tracking of spatial information of the captured nucleic acid (*e.g.*, analyte and/or intermediate agent). In some such embodiments, the nucleic acid sequence of the barcode is appended to the nucleic acid sequence of the captured nucleic acid (*e.g.,* by first strand cDNA synthesis and/or PCR extension), such that a sequencing of the barcoded nucleic acid molecule indicates the origin (*e.g.*, location on a two-dimensional array) of the capture probe where the nucleic acid molecule was captured.

Referring to Block 208, in some embodiments, the corresponding spatial barcode encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 x 10¹²}. Referring to Block 210, in some embodiments, the corresponding spatial barcode in the respective sequence read is localized to a contiguous set of oligonucleotides within the respective sequencing read. Referring to Block 212, in some embodiments, the contiguous set of oligonucleotides is an N-mer, wherein N is an integer selected from the set {4, ..., 20}.

As described above, in some embodiments, the spatial barcode associates the respective sequence read with a feature in a two-dimensional array of features. In some implementations, a respective feature (interchangeably, "capture spot") in the two-dimensional array of features includes a corresponding set of capture probes, and each respective capture probe in the corresponding set of capture probes comprises a respective spatial barcode that is uniquely associated with the respective feature. Thus, each respective capture probe in the plurality of capture probes on the two-dimensional array of features can be traced back to its originating position on the array and, subsequently, each respective sequence read obtained from a sequencing of each respective nucleic acid captured by each respective capture probe can be traced back to its originating position at the time of capture.

Referring to Block 214, in some embodiments, each respective feature includes 10 or more capture probes, 20 or more capture probes, 50 or more capture probes, 100 or more capture probes, 1000 or more capture probes, 2000 or more capture probes, 10,000 or more capture probes, or 100,000 or more capture probes. In some embodiments, each respective feature includes at least 500,000, at least 1 million, at least 2 million, or at least 5 million capture probes. In some embodiments, each respective feature includes no more than 10 million, no more than 5 million, no more than 1 million, no more than 100,000, no more than 10,000 or no more than 1000 capture probes. In some embodiments, each respective feature comprises from 1000 to 10,000, from 5000 to 100,000, from 10,000 to 500,000, or from 20,000 to 5 million capture probes. In some embodiments, each respective feature includes a plurality of capture probes that falls within another range starting no lower than 10 capture probes and ending no higher than 10 million capture probes. *See, e.g.,* the sections entitled "Definitions: Capture Spots," and "Definitions: Capture Spot Arrays," above.

Referring to Block 216, in some embodiments, each respective capture probe in the respective feature includes a poly-A sequence or a poly-T sequence and the corresponding spatial barcode for the respective feature that is incorporated into sequence reads in the plurality of sequence reads associated with the respective feature. Referring to Block 218, in some embodiments, each respective capture probe in the respective feature includes the same spatial barcode. In some embodiments, a first capture probe in a respective feature has a different spatial barcode than a second capture probe in the respective feature.

In some embodiments, the unique molecular identifier (UMI) for each respective sequence read associates the respective sequence read with a unique molecule (*e.g.,* a unique nucleic acid molecule from which the respective sequence read was obtained via sequencing). For instance, in some embodiments, the plurality of nucleic acid sequence reads comprises, for each respective nucleic acid in the plurality of nucleic acids representing the plurality of different genomic regions, a corresponding set of nucleic acid sequence reads derived from the respective nucleic acid, where each respective sequence read in the corresponding set of nucleic acid sequence reads has the same UMI, thus linking the respective sequence read to its originating nucleic acid molecule.

In some embodiments, the method comprises capturing the plurality of nucleic acids representing the plurality of different genomic regions using a plurality of capture probes, each respective capture probe comprising a unique molecular identifier that is specific to the respective capture probe. In some such embodiments, the nucleic acid sequence of the unique molecular identifier is appended to the nucleic acid sequence of the captured nucleic acid (*e.g.,* by first strand cDNA synthesis and/or PCR extension). Accordingly, referring to Block 220, each respective capture probe in the respective feature includes a unique molecule identifier that is incorporated into sequence reads in the plurality of sequence reads associated with the respective capture probe. Non-limiting embodiments for UMIs are described in further detail in, *e.g.*, the sections entitled "Definitions: Capture Probes," and "Methods for Spatial Analysis of Analytes," above.

In some embodiments, the two-dimensional array of features comprises at least 50, at least 100, at least 200, at least 500, at least 1000, at least 2000, at least 5000, at least 10,000, at least 100,000, at least 500,000, or at least 1 million features. In some embodiments, the two-dimensional array of features comprises no more than 5 million, no more than 1 million, no more than 100,000, no more than 10,000, no more than 1000, or no more than 500 features. In some embodiments, the two-dimensional array of features comprises from 100 to 10,000, from 300 to 5000, from 2000 to 100,000, or from 50,000 to 500,000 features. In some embodiments, the two-dimensional array of features includes a plurality of features that falls within another range starting no lower than 50 features and ending no higher than 5 million features.

In some embodiments, each feature corresponds to a plurality of cells in the tissue sample of the subject. In some such embodiments, each respective feature in the plurality of features in the two-dimensional array of features corresponds to a different plurality of cells in the tissue sample of the subject.

In some embodiments, each feature corresponds to at least 1, at least 2, at least 3, at least 4, at least 5, at least 10, at least 20, at least 30, at least 50, at least 100, or at least 200 cells. In some embodiments, each feature corresponds to no more than 500, no more than 200, no more than 100, no more than 50, no more than 20, or no more than 10 cells. In some embodiments, each feature corresponds to between 1 and 10 cells, between 3 and 8 cells, between 5 and 20 cells, or between 2 and 100 cells. In some embodiments, each feature corresponds to another range of cells starting no lower than 1 cell and ending no higher than 500 cells.

In some embodiments, each feature corresponds to a single cell in the tissue sample of the subject. In some such embodiments, each respective feature in the plurality of features in the two-dimensional array of features corresponds to a different respective cell in the tissue sample of the subject.

In some embodiments, each cell in the tissue sample of the subject corresponds to one or more features in the two-dimensional array of features. In some such embodiments, each respective cell in the tissue sample of the subject corresponds to a different set of features. In some embodiments, each cell in the tissue sample of the subject corresponds to a single feature in the two-dimensional array of features. In some embodiments, each cell in the tissue sample of the subject corresponds to a different respective feature in the two-dimensional array of features.

In other words, in some embodiments, the two-dimensional array of features is arranged such that a respective cell in the tissue sample of the subject can be contacted by one or more features and/or a respective feature in the two-dimensional array of features contacts one or more cells. In some embodiments, at least one cell in the tissue sample of the subject is not contacted by a respective feature and/or at least one feature in the two-dimensional array of features does not contact a respective cell.

Referring to Block 222, in some embodiments, the obtaining the plurality of nucleic acid sequence reads comprises sequencing of the two-dimensional array of features on the substrate.

A wide variety of different sequencing methods can be used to obtain the plurality of nucleic acid sequence reads. In general, sequence reads can be obtained from, for example, nucleic acid molecules such as deoxyribonucleic acid (DNA) or ribonucleic acid (RNA), including variants or derivatives thereof (*e.g.*, single stranded DNA or DNA/RNA hybrids, and nucleic acid molecules with a nucleotide analog). Sequencing can be performed by various commercial systems. More generally, sequencing can be performed using nucleic acid amplification, polymerase chain reaction (PCR) (*e.g.,* digital PCR and droplet digital PCR (ddPCR), quantitative PCR, real time PCR, multiplex PCR, PCR-based singleplex methods, emulsion PCR), and/or isothermal amplification.

Other examples of methods for sequencing include, but are not limited to, DNA hybridization methods (*e.g.*, Southern blotting), restriction enzyme digestion methods, Sanger sequencing methods, next-generation sequencing methods (*e.g.*, single-molecule real-time sequencing, nanopore sequencing, and Polony sequencing), ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and any combinations thereof.

Referring to Block 224, in some embodiments, the obtaining the plurality of nucleic acid sequence reads comprises high-throughput sequencing.

Referring to Block 226, in some embodiments, the obtaining the plurality of nucleic acid sequence reads comprises genome-wide transcript coverage obtained from a gene expression workflow.

Referring to Block 228, in some embodiments, the plurality of sequence reads comprises 50,000 or more sequence reads, 100,000 or more sequence reads, or 1 x 10⁶ or more sequence reads. In some embodiments, the plurality of sequence reads comprises at least 10,000, at least 20,000, at least 50,000, at least 100,000, at least 200,000, at least 300,000, at least 400,000, at least 500,000, at least 600,000, at least 700,000, at least 800,000, at least 900,000, at least 1 million, at least 2 million, at least 3 million, at least 5 million, at least 10 million, at least 50 million, or at least 100 million sequence reads. In some embodiments, the plurality of sequence reads comprises no more than 200 million, no more than 50 million, no more than 10 million, no more than 5 million, no more than 1 million, no more than 500,000, no more than 100,000, no more than 50,000, or no more than 10,000 sequence reads. In some embodiments, the plurality of sequence reads comprises from 10,000 to 100,000, from 50,000 to 500,000, from 100,000 to 2 million, or from 500,000 to 10 million sequence reads. In some embodiments, the plurality of sequence reads falls within another range starting no lower than 10,000 sequence reads and ending no higher than 200 million sequence reads.

Referring to Block 230, in some embodiments, the plurality of sequence reads comprises more than 50 sequence reads for all or portions of a plurality of nucleic acids representing 5000 or more different genomic regions in the genome of the subject across ten or more different chromosomes. In some embodiments, for each respective genomic region in the plurality of different genomic regions, the plurality of sequence reads comprises at least 1, at least 2, at least 3, at least 4, at least 5, at least 10, at least 20, at least 30, at least 40, at least 50, at least 100, at least 200, or at least 500 sequence reads for all or portions of a set of nucleic acids that represent the respective genomic region. In some embodiments, for each respective genomic region in the plurality of different genomic regions, the plurality of sequence reads comprises no more than 1000, no more than 500, no more than 100, no more than 50, or no more than 20 sequence reads for all or portions of a set of nucleic acids that represent the respective genomic region.

In some embodiments, the obtaining the plurality of nucleic acid sequence reads comprises sparse transcript coverage, such that one or more transcripts in the transcriptome of the subject are not represented in the plurality of nucleic acid sequence reads.

In some embodiments, the sequencing generates the plurality of nucleic acid sequence reads including the spatial barcode, the UMI, and a nucleic acid sequence for a captured nucleic acid that represents one or more genomic regions in the plurality of genomic regions. In some embodiments where the nucleic acid is an analyte, the sequencing of the captured nucleic acid comprises obtaining all or a portion of the nucleic acid sequence of the analyte, as well as the sequence of the barcode and the UMI. In some embodiments where the nucleic acid is an intermediate agent (*e.g.,* a probe and/or a ligation product obtained from two or more probes) designed to target a respective analyte, the sequencing of the captured nucleic acid comprises obtaining all or a portion of the nucleic acid sequence of the intermediate agent, where the intermediate agent is at least partially complementary (*e.g.*, hybridizable) to the nucleic acid sequence of the target analyte. Accordingly, the sequencing of the captured nucleic acid comprises obtaining a nucleic acid sequence that can be mapped to the target nucleic acid analyte, as well as the sequence of the barcode and the UMI.

In some embodiments, the plurality of sequence reads are mapped to a reference sequence. In some such embodiments, the mapping the plurality of sequence reads to the reference sequence comprises mapping the portion of the sequence read that corresponds to (*e.g.,* that includes and/or is hybridizable to) the nucleic acid sequence of a target analyte to the reference sequence. In some embodiments, the mapping is an alignment. In some embodiments, the reference sequence is a genome of the subject. In some embodiments, the reference sequence is a mammalian genome (*e.g.,* a human genome and/or a mouse genome).

In some embodiments, the plurality of sequence reads are preprocessed prior to further analysis (*e.g.*, determining counts).

In some embodiments, the preprocessing comprises performing a procedure where, for each respective genomic region, a corresponding subset of sequence reads that maps to the respective genomic region is identified. In some embodiments, the genomic region is retrieved from a lookup table, file or data structure. In some embodiments, the sequence reads are obtained in an electronic data file (*e.g.,* in a BAM file format) that provides, as input, sequence reads mapped to the reference sequence, such that the corresponding genomic regions are known *a priori.* In some embodiments, a filtering step is performed such that sequence reads that do not map to any genomic regions are removed from the plurality of nucleic acid sequence reads. In some examples, where sequence reads represent RNA analytes (*e.g.*, RNA transcripts), the sequence reads that overlap splice sites are removed from the plurality of nucleic acid sequence reads during the filtering step. For instance, the removal of RNA sequence reads that overlay splice sites prevents alignment of exon-exon reads with genomic sequences that span long introns. Other preprocessing steps are contemplated, as will be apparent to one skilled in the art.

Referring to Block 232, the method further includes using the plurality of sequence reads to determine a count data structure comprising, for each different genomic region represented by the plurality of nucleic acids, a respective UMI count for each feature in the two-dimensional array of features on the substrate having a positive UMI count. In some embodiments, the UMI count is a tally of the number of unique UMIs that map to a respective genomic region (*e.g.*, determined by a mapping of the nucleic acid sequence read to a reference sequence) at each respective feature (*e.g.*, determined using the spatial barcode).

Accordingly, referring to Block 234, in some embodiments, the using the plurality of sequence reads to determine a count data structure comprises aligning each sequence read in the plurality of sequence reads to a genome of the subject.

In some embodiments, the alignment is a local alignment. Local alignment or local sequence alignment is used to determine regions of nucleic acid sequences that are similar by recursively comparing two sequences at all possible lengths and optimizing a similarity score for all possible matches/mismatches, insertions or deletions. The local alignment aligns the sequence read to a reference sequence. In some such embodiments, the sequence reads have already been mapped to a genomic region (*e.g.,* a locus). A mapping algorithm will try to locate a location (*e.g.*, ideally a unique location) in the reference sequence that matches the sequence read, while tolerating a certain amount of mismatch to allow subsequence variation detection. Examples of programs that can serve to map sequence reads to genomic regions include, but are not limited to SARUMAN, GPU-RMAP, BarraCUDA, SOAP3, SOAP3-dp, CUSHAW, CUSHAW2-GPU, Burrows-Wheeler transform algorithm, a hashing algorithm, pigeonhole, MAQ, RMAP, SOAP, Hobbes, ZOOM, FastHASH, RazerS, RazerS 3, BFAST SEME, SHRiMP, BWT-SW, BWA, Botie, BLASR, Bowtie 2, BWA-SW, GEM, or SOAP2. For further discussion of these mapping algorithms, *see* Canzar and Stazberg, 2018, "Short Read Mapping: An Algorithmic Tour," Proc IEEE Inst. Electr Electron Eng., 105(3), 436-458.

Referring to Block 236, in some embodiments, the aligning is a local alignment that aligns the respective sequence read to the genome of the subject using a scoring system that (i) penalizes a mismatch between a nucleotide in the respective sequence read and a corresponding nucleotide in the reference sequence in accordance with a substitution matrix and (ii) penalizes a gap introduced into an alignment of the sequence read and the reference sequence. Referring to Block 238, in some embodiments, the local alignment is a Smith-Waterman alignment.

For instance, in some embodiments, the alignment scoring system penalizes a mismatch between a nucleotide in the sequence read and a corresponding nucleotide in the reference sequence in accordance with a substitution matrix. The scoring system also penalizes a gap introduced into an alignment of the sequence read and the reference sequence. Examples where such scoring is used are the local sequence alignment algorithms of Smith-Waterman (*see, for example,* Smith and Waterman, J Mol. Biol., 147(1):195-97 (1981), which is incorporated herein by reference), Lalign (*see, for example,* Huang and Miller, Adv. Appl. Math, 12:337-57 (1991), and PatternHunter (*see, for example,* Ma B. et al., Bioinformatics, 18(3):440-45 (2002).

In some embodiments, the aligning further comprises determining a count of the number of nucleic acid sequence reads that map to each respective genomic region in the plurality of genomic regions.

In some embodiments, the determining the count is performed based on a count of the number of unique UMIs corresponding to the plurality of sequence reads that map to the respective genomic region.

In some embodiments, a sequence read is deemed to map to a respective genomic region when all or a subset of the portion of the sequence read that corresponds to (*e.g.,* that includes and/or is hybridizable to) the nucleic acid sequence of a target analyte aligns to the respective genomic region. For instance, in some embodiments, a sequence read is deemed to map to a respective genomic region when at least 40%, at least 50%, at least 60%, at least 70%, at least 80%, at least 90%, or at least 95% of the portion of the sequence read that corresponds to the target analyte aligns to the respective genomic region.

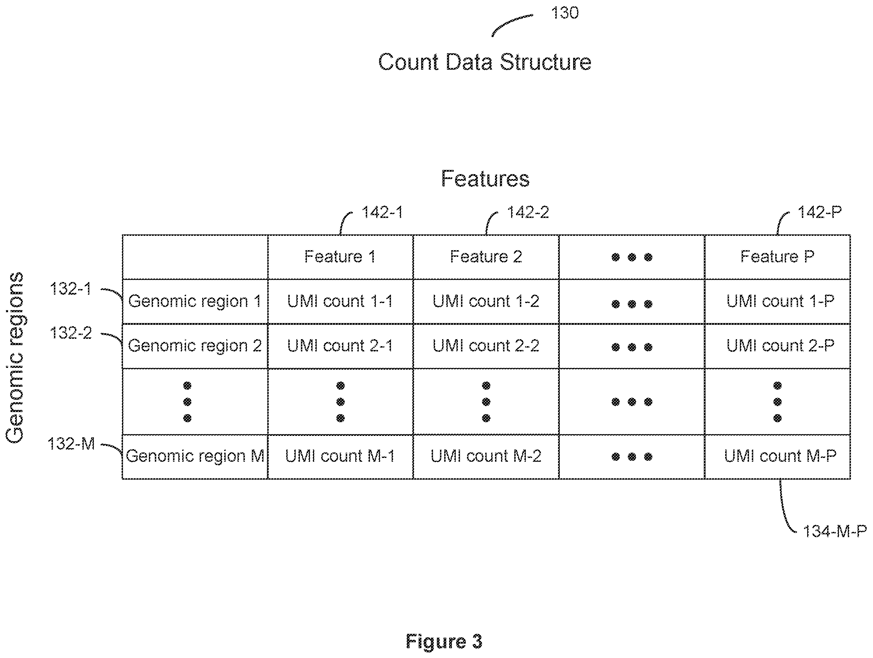

In some embodiments, the method comprises, after the aligning, generating the count matrix including, for each respective genomic region in the plurality of genomic regions, a respective UMI count indicating the number of unique UMIs that aligned to the respective genomic region for each respective feature in the plurality of features. An exemplary count matrix is depicted in Figure 3 (*e.g.,* Count Data Structure 130). The count matrix 130 is dimensioned by genomic regions 132 (*e.g.,* genes and/or loci) along a first axis and by a plurality of features 142 (*e.g.*, represented by spatial barcodes) along a second axis. In count matrix 130, each feature 142 in a plurality of P features represents a different plurality of capture probes, where P is a positive integer. Each row in count matrix 130 represents a different genomic region 132 in the plurality of M different genomic regions, where M is a positive integer. Thus, each element in count matrix 130 is a count of the number of unique nucleic acids captured at a respective feature (*e.g.*, location on a two-dimensional array of features) that can be mapped to a respective genomic region, where each unique nucleic acid is determined by its corresponding unique molecular identifier.

In some embodiments, the count matrix is a gene expression matrix. In some embodiments, the gene expression matrix is a count of the number of nucleic acid sequence reads that map to the respective genomic region, based on an alignment.

Referring to Block 240, the method further includes determining, for each respective feature in the two-dimensional array of features, a respective bin count for each respective bin in a plurality of bins spanning all or a portion of the genome of the subject corresponding to the respective feature. In some embodiments, each respective bin in the plurality of bins comprises a respective subset of the plurality of different genomic regions. Thus, in some embodiments, each respective bin in the plurality of bins comprises multiple genomic regions in the plurality of different genomic regions.

In some embodiments, the respective bin count for each respective bin is determined using the UMI count for each respective genomic region in the respective subset of the plurality of different genomic regions. In some embodiments, the respective bin count for each respective bin is a sum of the UMI counts for each respective genomic region in the respective subset of the plurality of different genomic regions.

In some embodiments, binning the plurality of genomic regions to obtain bin counts allows the UMI counts corresponding to individual genomic regions to be applied to larger regions encompassing multiple genomic regions. In this way, it is possible to obtain analyte capture information even when one or more individual genomic regions are poorly represented or not represented in the plurality of sequence reads (*e.g.*, where nucleic acids representing the one or more individual genomic regions are sparsely captured or not captured by the two-dimensional array of features). Moreover, in some embodiments, binning the plurality of genomic regions to obtain bin counts provides information relating to larger chromosomal mutations, such as copy number variations, which can be difficult to determine when analyzing expression data on a gene-by-gene basis.

In some embodiments, each bin comprises at least 0.1 megabases (MB), at least 0.5 MB, at least 1 MB, at least 1.5 MB, at least 2 MB at least 3 MB, or at least 5 MB of a corresponding reference sequence for the subject. In some embodiments, each bin comprises no more than 10 MB, no more than 5 MB, no more than 1 MB, or no more than 0.5 MB of a corresponding reference sequence for the subject. In some embodiments, each bin comprises from 0.1 to 0.5 MB, from 0.2 to 1 MB, from 1 to 10 MB, or from 3 to 8 MB. In some embodiments, each bin falls within another range starting no lower than 0.1 MB and ending no higher than 10 MB.

In some embodiments, the method includes preprocessing the count data structure prior to the determining the respective bin count for each respective bin. In some such embodiments, the method includes stabilizing variance in the plurality of UMI counts. For instance, referring to Block 242, in some embodiments, the method further comprises, prior to the determining the respective bin count for each respective bin, transforming the count data structure using a log-Freeman-Tukey transform. In some embodiments, the method includes smoothing outliers in the plurality of UMI counts. For instance, in some embodiments, the method comprises, prior to the determining the respective bin count for each respective bin, performing a polynomial dynamic linear modeling (DLM) across the plurality of UMI counts in the count data structure.

Referring to Block 244, the method further includes determining a respective copy number state of each respective feature in the two-dimensional array of features using the respective bin count for each respective bin in the respective plurality of bins corresponding to the respective feature.

Any suitable method for determining copy number state is contemplated for use in the present disclosure. For example, referring to Block 246, in some embodiments, the determining the respective copy number state calculates, for each respective feature in the two-dimensional array of features, the respective copy number state, across the corresponding plurality of bins of the respective feature, using a stochastic modeling algorithm and the respective bin count for each respective bin in the respective plurality of bins corresponding to the respective feature. Referring to Block 248, in some embodiments, the stochastic modeling algorithm is a Hidden Markov Model algorithm.

As another example, referring to Block 250, in some embodiments, the determining the respective copy number state calculates, for each respective feature in the two-dimensional array of features, the respective copy number state, across the corresponding plurality of bins of the respective feature, using a circular binary segmentation algorithm and the respective bin count for each respective bin in the respective plurality of bins corresponding to the respective feature.

In some embodiments, the determining the respective copy number state is performed using a single sample approach (e.g., without a reference or a control sample). In some such embodiments, sequence reads are mapped to a reference genome to form a plurality of genomic regions. Genomic regions are binned into variable-sized bins and read coverage is determined for each bin. For coverage normalization, the variable-sized bins are selected to contain a constant number of mappable positions (such an approach can smooth stochastic sampling noise). For an exemplary reference sequence, the mappability for various sequencing methodologies (*e.g.*, fragment or mate pair) and read lengths can be determined. This can be used to predict, for each position in the reference sequence, whether it is likely to be capable of having reads uniquely map there or not based on the degree of homology or repetitiveness elsewhere in the reference sequence. Within these bins, coverage can be further normalized based on predicted mappability and GC content of the bins. In various embodiments, a Hidden Markov Model (HMM) can be used for segmentation, applying empirically derived filters to one or more contiguous bins to call copy number states. In some such embodiments, the copy number states of the bins are determined, and any copy number variations present can be detected for each genomic region.

In some embodiments, the determining the respective copy number state is performed using a paired-sample approach. In some such embodiments, rather than comparing to the predicted mappability of the reference sequence, the coverage of the sample of the subject can be normalized by comparing it to the coverage of a control sample. Using such an approach can, in some instances, address systematic issues such as mappability and/or GC content, which may be expected to be similar between both samples, thus simplifying normalization. In some such embodiments, nucleic acid sequence reads are obtained for the sample of the subject and a control sample. For each sample, the plurality of nucleic acid sequence reads is aligned to a reference sequence and the aligned reads form a plurality of genomic regions. In various embodiments, the subject sample and the control sample nucleic acid sequence reads can be stored in a single nucleic acid sequence data file. Nucleic acid sequence read coverage is determined for each base position of the plurality of genomic regions of the subject sample and the control sample. Each of the plurality of genomic regions of the subject sample and the control sample is binned into one or more nonoverlapping fixed-size bins. In various embodiments, the bin size can be variable and determined, for example, by fixing the number of positions of a control sample with coverage. Nucleic acid sequence read coverage for each bin is determined and, to adjust for coverage differences in the samples, coverage of each bin is normalized by the mean coverage of the respective sample. Nucleic acid sequence read coverage ratios for each bin of the subject sample is determined by dividing the read coverage of each bin of the subject sample with the read coverage of a corresponding bin of the control sample. In some embodiments, a stochastic modeling algorithm (*e.g.*, a Hidden Markov Modeling (HMM) algorithm) is used to convert the normalized nucleic acid sequence read coverage ratios for each bin of the subject sample to discrete copy number states. In some embodiments, the discrete copy number states of each bin of the subject sample is utilized to identify copy number variation in the genomic regions of the subject sample. In various embodiments, adjacent bins with the same copy number are merged into segments for CNV reporting purposes. In various embodiments, bins are filtered before they are merged into a segment to meet minimum segment length requirements and/or window region mappability thresholds. *See, e.g.,* United States Patent Application US17/225,833, filed April 8, 2021.

In some embodiments, the determining the respective copy number state comprises a read count approach, a paired-end approach, and/or an assembly approach.

Read count approaches are generally performed by counting the number of nucleic acid sequence reads that are mapped to a genomic region within each frame of a nonoverlapping sliding window. Read count values are used to identify regions with copy number variations. Paired-end approaches are typically used with paired-end next-generation sequencing methodologies and identify genomic aberrations based on distances between paired reads. For instance, in paired-end sequencing data, sequence reads are obtained for each of the two ends of genomic regions. The distance between pairs of paired-end reads is used as an indicator of a genomic aberration, such that genomic aberrations are detected when the distance is significantly different from the predetermined average insert size. Assembly approaches assemble genomic regions by connecting overlapping short reads (contigs). Copy number variations are detected by comparing the assembled contigs to the reference genome. Unlike read count approaches, assembly approaches do not perform an alignment of the sequence reads to the reference genome prior to assembly.

In some embodiments, the determining the respective copy number state comprises performing a segmentation step. In some embodiments, the segmentation step is performed using circular binary segmentation.

In some implementations, the determining the respective copy number state is performed using a copy number variation detection tool. Examples of copy number variation detection tools contemplated for use in the present disclosure include, but are not limited to, ADTEx, CONTRA, cn.MOPS, ExomeCNV, VarScan2, and/or CoNVEX. *See, e.g.,* Zare et al., "An evaluation of copy number variation detection tools for cancer using whole exome sequencing data," BMC Bioinformatics (2017) 18286.

As another example, referring to Block 252, in some embodiments, the method further comprises i) clustering the count data structure across the plurality of bins to arrive at a plurality of clusters of features in the two-dimensional array of features; ii) determining a corresponding cluster consensus profile across the plurality of genomic regions (e.g., 1000 or more different genomic regions) in the genome of the subject for each cluster in the plurality of clusters; iii) identifying a confident normal cluster in the plurality of clusters of features as a ground-state copy number based on a variance with respect to the corresponding consensus profile for the first cluster as compared to a variance with respect to the corresponding consensus profile for each other cluster in the plurality of clusters; iv) performing copy number evaluation for each respective cluster in the plurality of clusters using the corresponding consensus profile of the respective cluster; v) clustering the plurality of features in the two-dimensional array of features into a first cluster and a second cluster; vi) identifying each feature in the first cluster as one of aneuploid or diploid and each feature in the second cluster as the of aneuploid or diploid based on an enrichment within the first cluster or the second cluster of features in the confident normal cluster; and vii) marking each feature in the two-dimensional array of features as one aneuploid or diploid based on the identifying vi).

In some embodiments, the i) clustering is hierarchical clustering, and the method further comprises estimating the variance of each cluster of features in the plurality of clusters of features. In some embodiments, the variance is estimated using a Gaussian mixture model. In some embodiments, the ii) corresponding cluster consensus profile across the plurality of genomic regions is determined by, for each respective bin in the plurality of bins, pooling the plurality of bin counts within each respective cluster of features.

In some embodiments, the iii) confident normal cluster is identified by selecting the cluster of features having the minimum estimated variance across the plurality of clusters of features. In some embodiments, the iii) confident normal cluster is identified on a per-feature basis, where a mixture of three Gaussian models of UMI counts for each respective feature is deemed to represent genomic gains, losses, and neutral states, and where a respective feature is identified as confident normal when at least a threshold percentage of genomic regions in the plurality of genomic regions exhibit a neutral state. In some such embodiments, the threshold percentage is at least 60%, at least 70%, at least 80%, at last 90%, at least 95%, at least 98%, or at least 99%.

In some embodiments, the iv) performing copy number evaluation comprises, for each cluster of features, identifying consensus chromosome breakpoints based on the corresponding consensus profile of each respective cluster in the plurality of clusters of features. In some such embodiments, the consensus chromosome breakpoints are identified by integrating a Poisson-gamma model and Markov Chain Monte Carlo iterations to generate posterior means per bin and applying Kolmogorov-Smirnov tests to join adjacent bins with similar means. Accordingly, referring to Block 254, in some embodiments, the method further comprises merging together adjacent bins that have the same copy number state for a respective feature. In some embodiments, the consensus chromosome breakpoints from each feature cluster are merged to form a union of consensus genomic breakpoints for all of the features in the plurality of features. In some implementations, final copy number values for each bin are calculated as the posterior averages of all genomic regions spanning across the adjacent chromosome breakpoints in each feature.

In some embodiments, the iv) performing copy number evaluation comprises any of the copy number variation detection methods disclosed herein.

In some embodiments, the v) clustering is hierarchical clustering of the copy number states of each respective feature in the plurality of features, and the vi) identifying each feature in the first cluster as one of aneuploid or diploid and each feature in the second cluster as the of aneuploid or diploid is performed based on a distance between the copy number states of features in the confident normal cluster and all other features in the plurality of features. For instance, in some embodiments, the copy number states of features in the confident normal cluster are deemed to be diploid and features having copy number states that are highly distant from the features in the confident normal cluster are deemed to be aneuploid. In some embodiments, the vi) identifying is performed on a per-feature basis using a mixture of three Gaussian models. *See, e.g.,* Gao et al., "Delineating copy number and clonal substructure in human tumors from single-cell transcriptomes," Nature Biotechnology (2021), doi: 10.1038/s41587-020-00795-2.

Referring to Block 256, the method further includes using the respective copy number state of each respective feature in the two-dimensional array of features to identify the one or more regions of the tissue sample that are characterized by an aneuploid state and the one or more regions of the tissue sample that are characterized by the diploid state.

In some embodiments, the method further comprises using the one or more regions of the tissue sample that are characterized by an aneuploid state to identify a clinical condition. In some embodiments, the aneuploid state is a first clinical condition and the diploid state is a second clinical condition. In some embodiments, the aneuploid state is a first clinical condition and the diploid state is a healthy and/or normal condition.

In some embodiments, a respective clinical condition (*e.g.,* a first clinical condition and/or a second clinical condition) is a cancer.

In some embodiments, a respective clinical condition (*e.g.,* a first clinical condition and/or a second clinical condition) is selected from the group consisting of: Prader-Willi and Angelman syndromes (PWS/AS), Williams-Beuren syndrome (WBS), DiGeorge/Velocardiofacial syndromes, multiple congenital anomalies (MCA) (*e.g.,* intellectual disability (ID), developmental delay (DD), dysmorphic features, cardiac defects, limb and digital abnormalities and/or seizures), neuropsychiatric diseases (*e.g.*, autism spectrum disorders (ASD) and/or schizophrenia (SZ)), Parkinson's disease (PD), Alzheimer's disease (AD), susceptibility to HIV infection, autoimmune diseases (*e.g.*, systemic lupus erythromatosus (SLE), rheumatoid arthiritis (RA), and/or Crohn's disease), type 2 diabetes, obesity, drug metabolism and toxicity disorders, skin disorders (*e.g.*, Psoriasis, Hidradenitis Supparativa/Acne Inversa and/or dermatophytosis), Charcot-Marie Tooth disease type 1A/Hereditary Neuropathy with Pressure Palsies (CMT1A/HNPP), and Sotos syndrome. In some embodiments, a respective clinical condition is any of the copy number variation disorders disclosed in Shaikh, "Copy Number Variation Disorders," Curr Genet Med Rep. 2017 Dec; 5(4): 183-190.

In some embodiments, a respective clinical condition is cancer, where the cancer is selected from the group consisting of: Acute Lymphoblastic Leukemia (ALL), Acute Myeloid Leukemia (AML), Adolescents, Cancer in, Adrenocortical Carcinoma, AIDS-Related Cancers, Kaposi Sarcoma (Soft Tissue Sarcoma), AIDS-Related Lymphoma (Lymphoma), Primary CNS Lymphoma (Lymphoma), Anal Cancer, Appendix Cancer, Astrocytomas, Childhood (Brain Cancer), Atypical Teratoid/Rhabdoid Tumor, Childhood, Central Nervous System (Brain Cancer), Basal Cell Carcinoma of the Skin, Bile Duct Cancer, Bladder Cancer, Bone Cancer (includes Ewing Sarcoma and Osteosarcoma and Malignant Fibrous Histiocytoma), Brain Tumors, Breast Cancer, Bronchial Tumors (Lung Cancer), Burkitt Lymphoma, Carcinoid Tumor (Gastrointestinal), Carcinoma of Unknown Primary, Cardiac (Heart) Tumors, Childhood, Central Nervous System, Atypical Teratoid/Rhabdoid Tumor, Childhood (Brain Cancer), Medulloblastoma and Other CNS Embryonal Tumors, Childhood (Brain Cancer), Germ Cell Tumor, Childhood (Brain Cancer), Primary CNS Lymphoma, Cervical Cancer, Childhood Cancers, Cancers of Childhood, Unusual, Cholangiocarcinoma, Chordoma, Childhood (Bone Cancer), Chronic Lymphocytic Leukemia (CLL), Chronic Myelogenous Leukemia (CML), Chronic Myeloproliferative Neoplasms, Colorectal Cancer, Craniopharyngioma, Childhood (Brain Cancer), Cutaneous T-Cell Lymphoma, Ductal Carcinoma In Situ (DCIS), Childhood (Brain Cancer), Endometrial Cancer (Uterine Cancer), Ependymoma, Childhood (Brain Cancer), Esophageal Cancer, Esthesioneuroblastoma (Head and Neck Cancer), Ewing Sarcoma (Bone Cancer), Extracranial Germ Cell Tumor, Childhood, Extragonadal Germ Cell Tumor, Eye Cancer, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Gastrointestinal Carcinoid Tumor, Gastrointestinal Stromal Tumors (GIST) (Soft Tissue Sarcoma), Germ Cell Tumors, Childhood Central Nervous System Germ Cell Tumors (Brain Cancer), Childhood Extracranial Germ Cell Tumors, Extragonadal Germ Cell Tumors, Ovarian Germ Cell Tumors, Testicular Cancer, Gestational Trophoblastic Disease, Hairy Cell Leukemia, Head and Neck Cancer, Heart Tumors, Childhood, Hepatocellular (Liver) Cancer, Histiocytosis, Langerhans Cell, Hodgkin Lymphoma, Hypopharyngeal Cancer (Head and Neck Cancer), Intraocular Melanoma, Islet Cell Tumors, Pancreatic Neuroendocrine Tumors, Kaposi Sarcoma (Soft Tissue Sarcoma), Kidney (Renal Cell) Cancer, Langerhans Cell Histiocytosis, Laryngeal Cancer (Head and Neck Cancer), Leukemia, Lip and Oral Cavity Cancer (Head and Neck Cancer), Liver Cancer, Lung Cancer (Non-Small Cell, Small Cell, Pleuropulmonary Blastoma, and Tracheobronchial Tumor), Lymphoma, Male Breast Cancer, Malignant Fibrous Histiocytoma of Bone and Osteosarcoma, Melanoma, Melanoma, Intraocular (Eye), Merkel Cell Carcinoma (Skin Cancer), Mesothelioma, Malignant, Metastatic Cancer, Metastatic Squamous Neck Cancer with Occult Primary (Head and Neck Cancer), Midline Tract Carcinoma With NUT Gene Changes, Mouth Cancer (Head and Neck Cancer), Multiple Endocrine Neoplasia Syndromes, Multiple Myeloma/Plasma Cell Neoplasms, Mycosis Fungoides (Lymphoma), Myelodysplastic Syndromes, Myelodysplastic/Myeloproliferative Neoplasms, Myelogenous Leukemia, Chronic (CML), Myeloid Leukemia, Acute (AML), Myeloproliferative Neoplasms, Chronic, Nasal Cavity and Paranasal Sinus Cancer (Head and Neck Cancer), Nasopharyngeal Cancer (Head and Neck Cancer), Neuroblastoma, Non-Hodgkin Lymphoma, Non-Small Cell Lung Cancer, Oral Cancer, Lip and Oral Cavity Cancer and Oropharyngeal Cancer (Head and Neck Cancer), Osteosarcoma and Malignant Fibrous Histiocytoma of Bone, Ovarian Cancer, Pancreatic Cancer, Pancreatic Neuroendocrine Tumors (Islet Cell Tumors), Papillomatosis (Childhood Laryngeal), Paraganglioma, Paranasal Sinus and Nasal Cavity Cancer (Head and Neck Cancer), Parathyroid Cancer, Penile Cancer, Pharyngeal Cancer (Head and Neck Cancer), Pheochromocytoma, Pituitary Tumor, Plasma Cell Neoplasm/Multiple Myeloma, Pleuropulmonary Blastoma (Lung Cancer), Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Rhabdomyosarcoma, Childhood (Soft Tissue Sarcoma), Salivary Gland Cancer (Head and Neck Cancer), Childhood Rhabdomyosarcoma (Soft Tissue Sarcoma), Childhood Vascular Tumors (Soft Tissue Sarcoma), Ewing Sarcoma (Bone Cancer), Kaposi Sarcoma (Soft Tissue Sarcoma), Osteosarcoma (Bone Cancer), Soft Tissue Sarcoma, Uterine Sarcoma, Sézary Syndrome (Lymphoma), Skin Cancer, Small Cell Lung Cancer, Small Intestine Cancer, Soft Tissue Sarcoma, Squamous Cell Carcinoma of the Skin, Squamous Neck Cancer with Occult Primary, Metastatic (Head and Neck Cancer), Stomach (Gastric) Cancer, T-Cell Lymphoma, Lymphoma (Mycosis Fungoides and Sèzary Syndrome), Testicular Cancer, Throat Cancer (Head and Neck Cancer), Nasopharyngeal Cancer, Oropharyngeal Cancer, Hypopharyngeal Cancer, Thymoma and Thymic Carcinoma, Thyroid Cancer, Tracheobronchial Tumors (Lung Cancer), Transitional Cell Cancer of the Renal Pelvis and Ureter (Kidney (Renal Cell) Cancer), Ureter and Renal Pelvis, Transitional Cell Cancer (Kidney (Renal Cell) Cancer, Urethral Cancer, Uterine Cancer, Endometrial, Uterine Sarcoma, Vaginal Cancer, Vascular Tumors (Soft Tissue Sarcoma), and/or Vulvar Cancer.

In some embodiments, the cancer is selected from brain non-glioma (ependymoma, hemangioblastoma, medulloblastoma, meningioma), breast (breast ductal, breast lobular), colon, endometrial (endometrial, endometrial serous, endometrial stromal sarcoma), gastroesophageal (esophageal adenocarcinoma, gastric), gastrointestinal stromal tumor, glioma (Glioma, oligodendroglioma), head and neck adenocarcinoma, hematological (acute lymphoblastic leukemia, acute myeloid leukemia, b cell lymphoma, chronic lymphocytic leukemia, chronic myeloid leukemia, rosai dorfman, T-cell lymphoma), hepatobiliary (cholangiocarcinoma, gallbladder, liver), lung adenocarcinoma, melanoma, mesothelioma, neuroendocrine (gastrointestinal neuroendocrine, high grade neuroendocrine lung, low grade neuroendocrine lung, pancreatic neuroendocrine, skin neuroendocrine), ovarian (ovarian clear cell, ovarian granulosa, ovarian serous), pancreas, prostate, renal (renal chromophobe, renal clear cell, renal papillary), sarcoma (chondrosarcoma, chordoma, ewing sarcoma, fibrous sarcoma, leiomyosarcoma, liposarcoma, osteosarcoma, rhabdomyosarcoma, synovial sarcoma, vascular sarcoma), squamous (cervical, esophageal squamous, head and neck squamous, lung squamous, skin squamous/basal), thymic, thyroid, and/or urothelial cancers.

In some embodiments, the cancer is any one or more entries of the ICD-10-CM, or the International Classification of Disease. The ICD provides a method of classifying diseases, injuries, and causes of death. The World Health Organization (WHO) publishes the ICDs to standardize the methods of recording and tracking instances of diagnosed disease, including cancer. For example, in some embodiments, the cancer is selected from the classifications from any chapter of the ICD or cancers from Chapter 2, C and D codes. C codes include Neoplasm of Lip, Oral Cavity and Pharynx (C00-C14), Neoplasm of Digestive Organs (C15-C26), Neoplasm of Respiratory System and Intrathoracic Organs (C30-C39), Neoplasm of Mesothelial and Soft Tissue (C45), Neoplasm of Bones, Joints and Articular Cartilage (C40-C41), Neoplasm of Skin (Melanoma, Merkel Cell, and Other Skin Histologies) (C43, C44, C4a), Kaposi Sarcoma (46), Neoplasm of Peripheral Nerves and Autonomic nervous system, Retroperitoneum, Peritoneum, and Soft Tissues (C47, C48, C49), Neoplasm of Breast and Female Genital Organs (C50 - C58), Neoplasm of Male Genital Organs (C60-C63), Neoplasm of Urinary Tract (C64-C68), Neoplasms of Eye, Brain and Other Parts of the Central Nervous System (C69-C72), Neoplasm of Thyroid, Other Endocrine Glands, and Ill-defined Sites (C73-C76), Malignant Neuroendocrine Tumors (C7a._), Secondary Neuroendocrine Tumors (C7B), Neoplasm of other and ill- defined sites (C76-80), Secondary and unspecified malignant neoplasm of lymph nodes (C77), Secondary Cancers of respiratory and digestive organs, other and unspecified sites (C78-80), Malignant Neoplasm without specification of site (C80), Malignant neoplasms of lymphoid, and/or hematopoietic and related tissue (C81-C96).

In some embodiments, the cancer is any broadly construed categorization to a cohort class. Exemplary cohort classes include, but are not limited to, Blood Cancer, Bone Cancer, Brain Cancer, Bladder Cancer, Breast Cancer, Colon and Rectal Cancer, Endometrial Cancer, Kidney Cancer, Leukemia, Liver Cancer, Lung Cancer, Melanoma, Non-Hodgkin Lymphoma, Pancreatic Cancer, Prostate Cancer, Thyroid Cancer, and/or other tissue-based or organ-based classifications.

In some embodiments, the cancer is a site of biopsy for a biopsy specimen (e.g., a sample from a subject) such as one or more ICD-03 codes, including but not limited to lip, base of tongue, tongue, gum, floor of mouth, other mouth, salivary gland, oropharynx, nasopharynx, posterior wall of nasopharynx, hypopharynx, pharynx, esophagus, stomach, small intestine, large intestine, appendix, rectum, anal canal and/or anus, liver, intrahepatic bile ducts, gallbladder and/or extrahepatic bile ducts, pancreas, unspecified digestive organs, nasal cavity (including nasal cartilage), middle ear, sinuses, accessory sinus, nose, larynx, trachea, lung and/or bronchus, thymus, heart, mediastinum, pleura, respiratory, bones and/or joints, bones of skull and face, mandible, blood, bone marrow, hematopoietic system, spleen, reticulo-endothelial, skin, peripheral nerves, retroperitoneum and/or peritoneum, connective and/or soft tissue, breast, vagina and/or labia, vulva, cervix uteri, corpus uteri, uterus, ovary, fallopian tube, other female genital, placenta, penis, prostate gland, testis, epididymis, spermatic cord, male genital, scrotum, kidney, renal pelvis, ureter, urinary bladder, other urinary organs, orbit and/or lacrimal gland, retina, eyeball, eye, nose, meninges (*e.g.*, cerebral and spinal), brain, cranial nerves, spinal cord, ventricle, cerebellum, other nervous system, thyroid gland, adrenal glands, parathyroid gland, pituitary gland, craniopharyngeal duct, pineal gland, other endocrine glands, and/or lymph nodes.

In some embodiments, the cancer is one of a plurality of tumor and/or tissue types having common cell lineages. In some embodiments, the cancer is one of a plurality of metastasis sites and/or a metastasis site of origin (*e.g.,* a liver metastasis of pancreatic origin, upper gastrointestinal origin, or cholangio origin; a breast metastasis of salivary gland origin, squamous origin, or ductile origin; a brain metastasis of glioblastoma, oligodendroglioma, astrocytoma, or medulloblastoma; a lung metastasis of NSCLC adenocarcinoma or squamous, *etc*.).

For example, in some embodiments, referring to Block 258, the method further comprises identifying a region in the one or more regions characterized by the aneuploid state as tumor. In some embodiments, the method further comprises using the region identified as tumor to diagnose a cancer condition (*e.g.,* a presence or absence of cancer, a stage of cancer, a cancer type, a cancer subtype, a tissue of origin, a cancer grade, and/or a histopathological grade).

In some embodiments, referring to Block 260, the method further comprises using the one or more regions of the tissue sample that are characterized by an aneuploid state and the one or more regions of the tissue sample that are characterized by the diploid state to identify a stage of a cancer in the subject. In some embodiments, the method further comprises using the one or more regions of the tissue sample that are characterized by an aneuploid state and the one or more regions of the tissue sample that are characterized by the diploid state to determine whether a cancer has metastasized.

In some embodiments, the method further comprises using the one or more regions of the tissue sample that are characterized by an aneuploid state and the one or more regions of the tissue sample that are characterized by the diploid state to perform a subclonal analysis and/or gene expression analysis on identified populations. *See, e.g.,* Gao et al., "Delineating copy number and clonal substructure in human tumors from single-cell transcriptomes," Nature Biotechnology (2021), doi: 10.1038/s41587-020-00795-2.

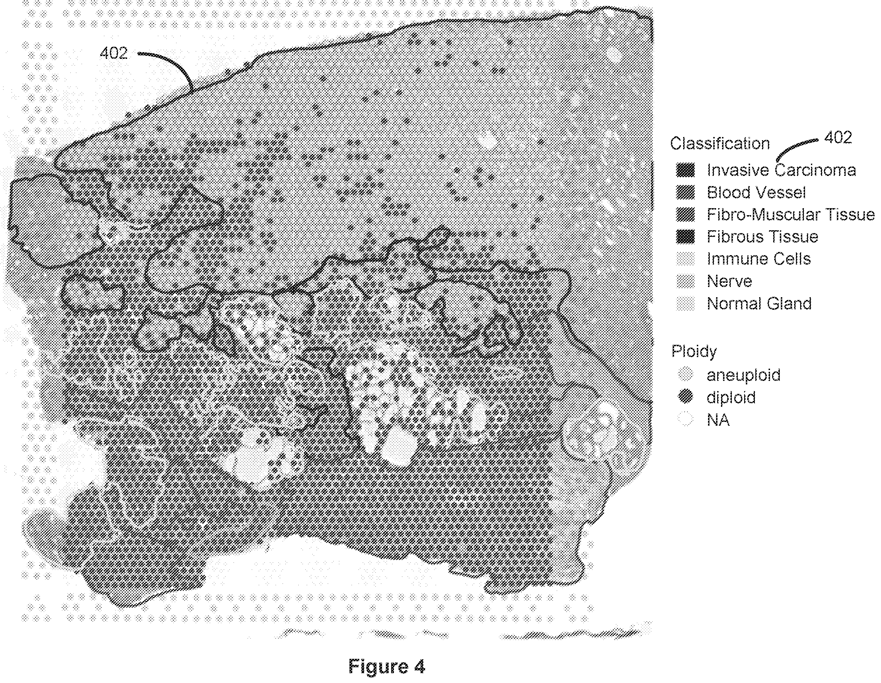

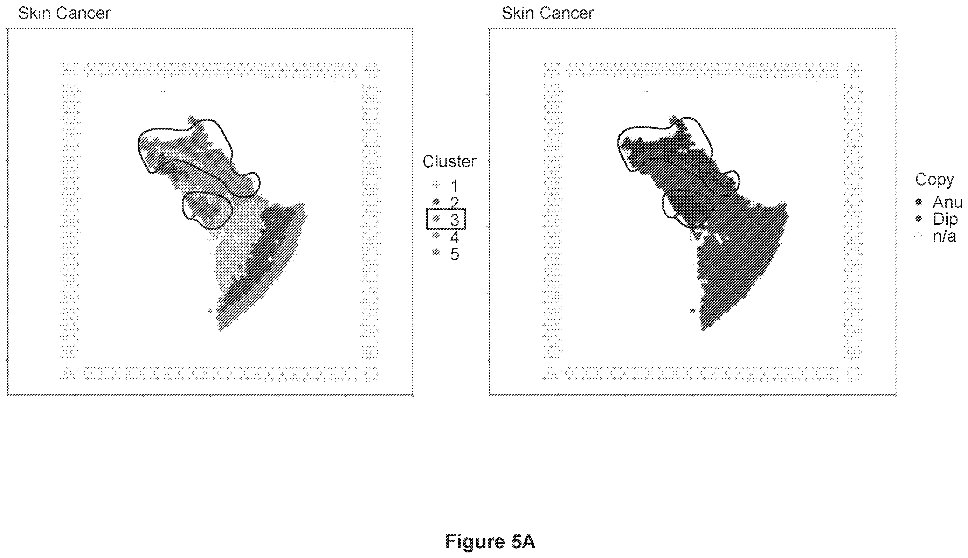

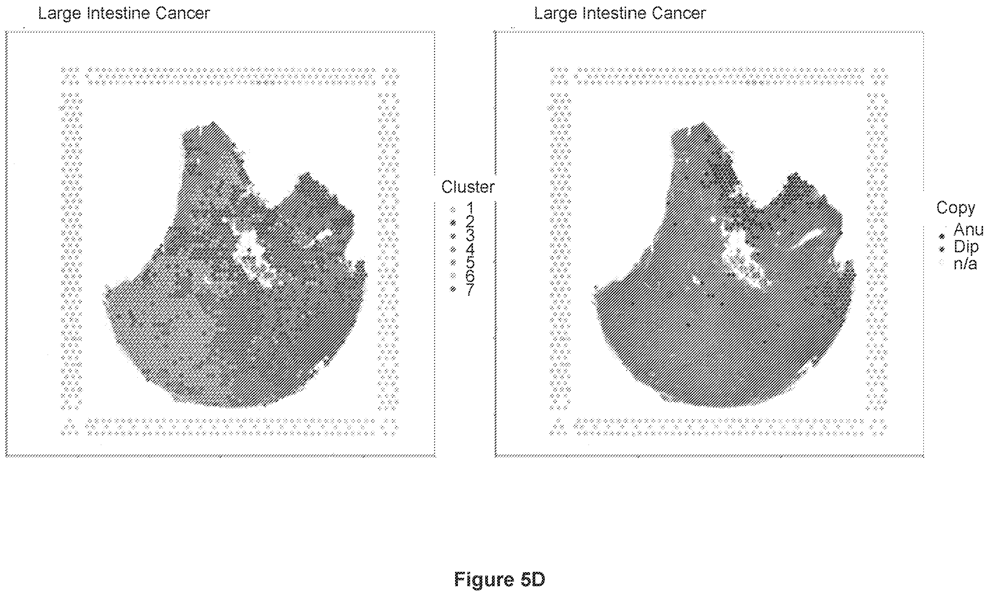

In some embodiments, the method further comprises visualizing, on a display, each respective feature in the plurality of features, where each respective feature in the two-dimensional array of features that is characterized by an aneuploid state has a first indicia and each respective feature that is characterized by the diploid state has a second indicia. For instance, as illustrated in Figures 4 and 5A-G, features characterized by an aneuploid state are indicated by a first coloring (*e.g.,* light shading (green)), while features characterized by a diploid state are indicated by a second coloring (*e.g.*, dark shading (red)).

Generally, the information types described above can be presented on a user interface of a computing device in an interactive manner, such that the user interface can receive user input instructing the user interface to modify representation of the information. Various combinations of information can be displayed concurrently in response to user input. Using the information visualization methods described herein, previously unknown patterns and relationships can be discovered from the regions of the tissue sample that are characterized by an aneuploid state or a diploid state. In this way, biological samples can be characterized.

In some embodiments, the method further comprises, on the display, performing a spatial overlay of an image of the tissue sample of the subject (*e.g.,* a brightfield microscopy image) with the visualization of each respective feature in the two-dimensional array of features, where each respective feature that is characterized by an aneuploid state has a first indicia and each respective feature that is characterized by the diploid state has a second indicia. In some embodiments, the method further includes annotating one or both of the image of the tissue sample of the subject and the visualization of the two-dimensional array of features. In some embodiments, the annotation is performed by user interaction with a user affordance on the display (*e.g.,* drawing, lassoing, selecting, highlighting, *etc*.). In some embodiments, the annotation is performed by a trained pathologist.

Additional embodiments for obtaining images, preparing biological samples for staining and/or imaging, and visualization and overlay of two-dimensional arrays and/or images are contemplated for use in the present disclosure, as described, *e.g.*, in the sections entitled "Definitions: Biological Samples" and "Methods for Spatial Analysis of Analytes," above.

### Additional Embodiments.

Another aspect of the present disclosure provides a computer system for delineating a tissue sample of a subject into one or more regions that are characterized by an aneuploid state and one or more regions that are characterized by a diploid state, the computer system comprising one or more processors and memory addressable by the one or more processors, the memory storing at least one program for execution by the one or more processors. The at least one program comprises instructions for a method including obtaining a plurality of nucleic acid sequence reads (*e.g.*, comprising 10,000 or more sequence reads), in electronic form. Each respective sequence read includes (i) a corresponding spatial barcode associating the respective sequence read with a feature in a two-dimensional array of features (*e.g.*, comprising at least 500 features) on a substrate in contact with the tissue sample for a period of time prior to obtaining the plurality of sequence reads and (ii) a unique molecular identifier. The plurality of sequence reads comprises sequence reads of all or portions of a plurality of nucleic acids representing 1000 or more different genomic regions in the genome of the subject across five or more different chromosomes.

The method further includes using the plurality of sequence reads to determine a count data structure comprising, for each different genomic region represented by the plurality of nucleic acids, a respective UMI count for each feature in the two-dimensional array of features on the substrate having a positive UMI count. For each respective feature in the two-dimensional array of features, a respective bin count is determined for each respective bin in a plurality of bins spanning all or a portion of the genome of the subject corresponding to the respective feature. A respective copy number state of each respective feature in the two-dimensional array of features is determined using the respective bin count for each respective bin in the respective plurality of bins corresponding to the respective feature. The respective copy number state of each respective feature in the two-dimensional array of features is used to identify the one or more regions of the tissue sample that are characterized by an aneuploid state and the one or more regions of the tissue sample that are characterized by the diploid state.

Another aspect of the present disclosure provides a non-transitory computer readable storage medium, where the non-transitory computer readable storage medium stores instructions, which when executed by a computer system, cause the computer system to perform a method for delineating a tissue sample of a subject into one or more regions that are characterized by an aneuploid state and one or more regions that are characterized by a diploid state. The method comprises obtaining a plurality of nucleic acid sequence reads (*e.g.*, comprising 10,000 or more sequence reads), in electronic form. Each respective sequence read includes (i) a corresponding spatial barcode associating the respective sequence read with a feature in a two-dimensional array of features (*e.g.*, comprising at least 500 features) on a substrate in contact with the tissue sample for a period of time prior to obtaining the plurality of sequence reads and (ii) a unique molecular identifier. The plurality of sequence reads comprises sequence reads of all or portions of a plurality of nucleic acids representing 1000 or more different genomic regions in the genome of the subject across five or more different chromosomes.

The plurality of sequence reads is used to determine a count data structure comprising, for each different genomic region represented by the plurality of nucleic acids, a respective UMI count for each feature in the two-dimensional array of features on the substrate having a positive UMI count. For each respective feature in the two-dimensional array of features, a respective bin count is determined for each respective bin in a plurality of bins spanning all or a portion of the genome of the subject corresponding to the respective feature. A respective copy number state of each respective feature in the two-dimensional array of features is determined using the respective bin count for each respective bin in the respective plurality of bins corresponding to the respective feature. The respective copy number state of each respective feature in the two-dimensional array of features is used to identify the one or more regions of the tissue sample that are characterized by an aneuploid state and the one or more regions of the tissue sample that are characterized by the diploid state.

Another aspect of the present disclosure provides a computer system for delineating a tissue sample of a subject into one or more regions that are characterized by an aneuploid state and one or more regions that are characterized by a diploid state, the computer system comprising one or more processors and memory addressable by the one or more processors, the memory storing at least one program for execution by the one or more processors, the at least one program comprising instructions for performing any of the methods and/or embodiments disclosed herein.

Another aspect of the present disclosure provides a non-transitory computer readable storage medium, where the non-transitory computer readable storage medium stores instructions, which when executed by a computer system, cause the computer system to perform any of the methods and/or embodiments disclosed herein.

### EXAMPLES

### Example 1 - Detecting Regions of Aneuploidy in Clinical Samples

Figures 4 and 5A-G illustrate exemplary tissue samples delineated into one or more regions that were characterized by an aneuploid state and one or more regions that were characterized by a diploid state, in accordance with some embodiments of the present disclosure.

Figure 4 shows a tissue section obtained from sample of prostate cancer. The tissue section was contacted with a two-dimensional array of features and used to obtain a plurality of nucleic acid sequence reads. A respective copy number state of each respective feature in the two-dimensional array of features was then determined, in accordance with an embodiment of the present disclosure, and each respective feature was classified as having an aneuploid state or a diploid state. An overlay of an image of the tissue section with the two-dimensional array of features was then performed to identify the one or more regions of the tissue sample that are characterized by an aneuploid state and the one or more regions of the tissue sample that are characterized by the diploid state, as indicated by the legend (*e.g.,* Ploidy: "Aneuploid," "Diploid," and "NA"). Regions of the tissue section that extended beyond the fiducial markers bordering the two-dimensional array are depicted but were not overlaid with features.

The image of the tissue section was further annotated by a trained pathologist, thus delineating the tissue into regions of classification (*e.g.*, Classification: "Invasive Carcinoma," "Blood Vessel," "Fibro-Muscular Tissue," "Fibrous Tissue," "Immune Cells," "Nerve," and "Normal Gland"). As depicted in Figure 4, regions of the tissue section corresponding to Invasive Carcinoma 402 by expert pathology were enriched in features that were characterized by an aneuploid state (*e.g.,* light shading), whereas all other non-cancerous regions of the tissue section were enriched in features that were characterized by a diploid state (*e.g.,* dark shading). These results show concordance between expert annotations of clinical conditions (*e.g.,* cancer or non-cancer) and feature ploidy (*e.g.,* aneuploid or diploid) and further highlight the ability of the systems and methods of the present disclosure to accurately discriminate between clinical conditions by determining aneuploidy in tissues.

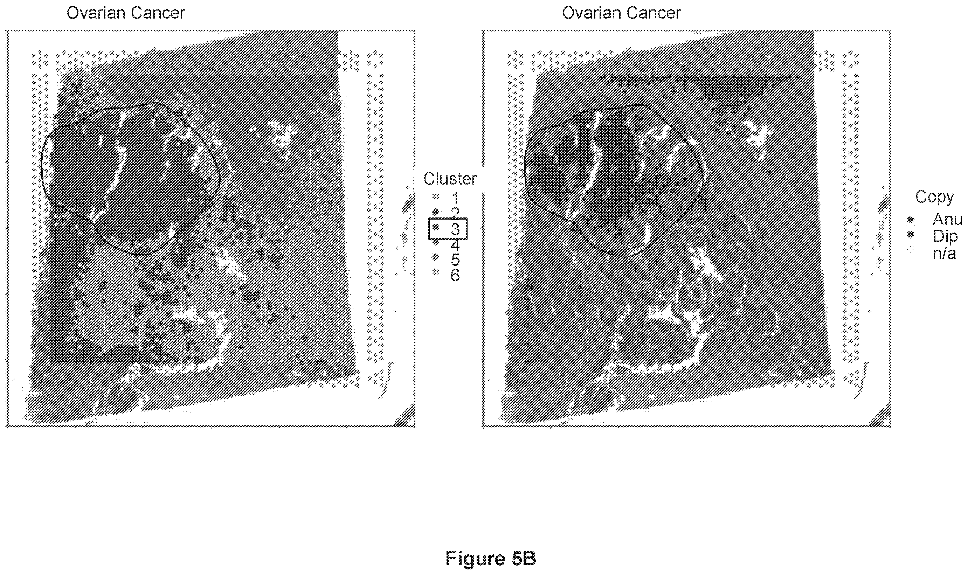

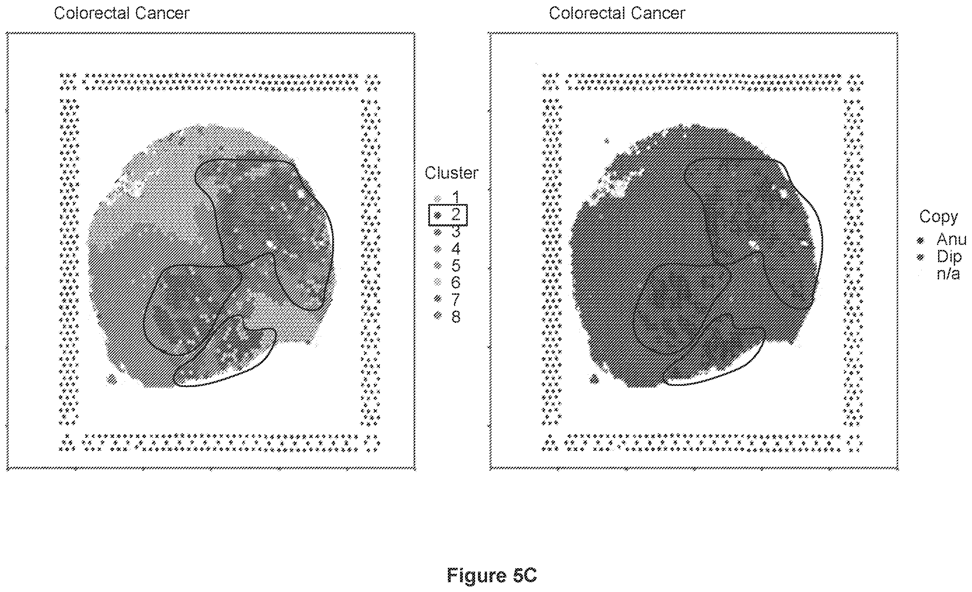

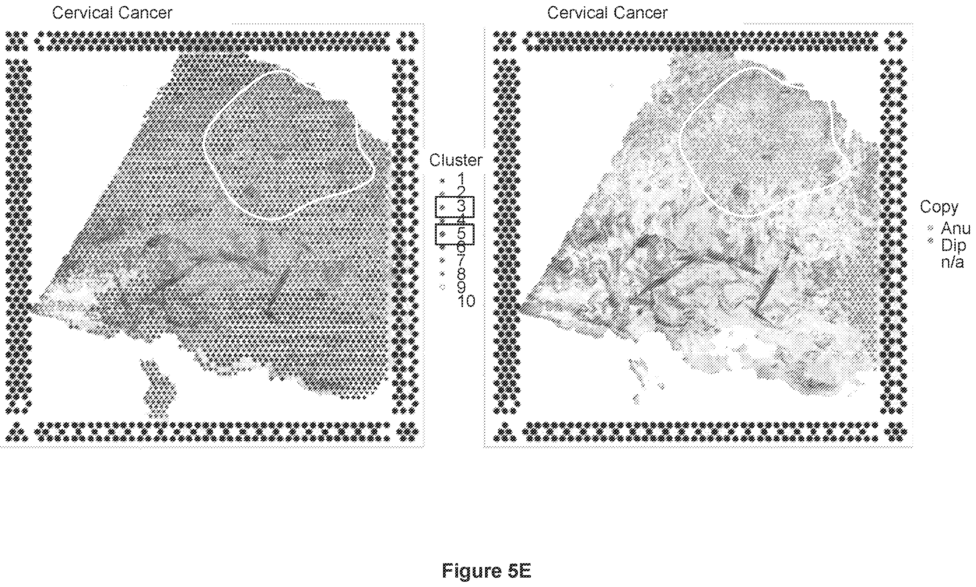

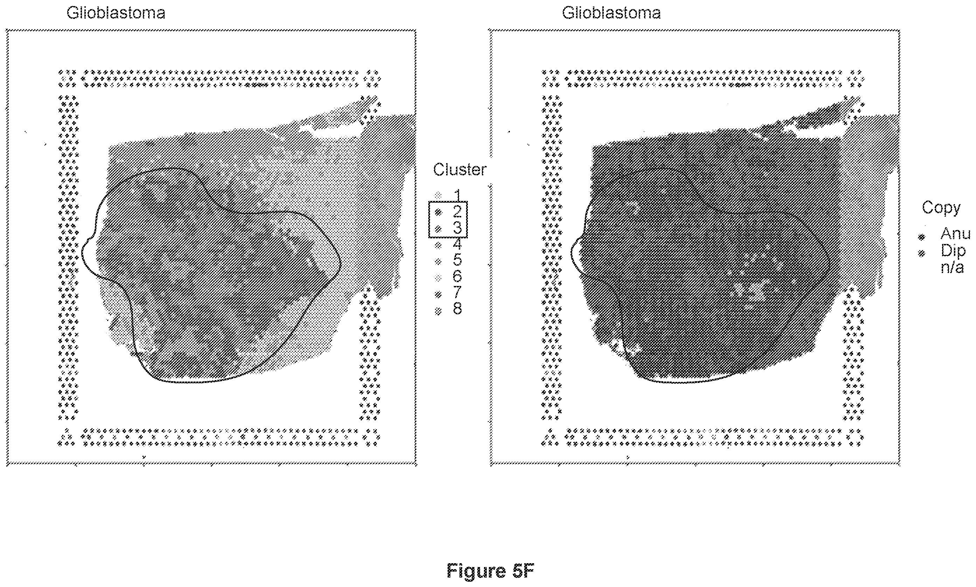

Figures 5A-G illustrate a method in accordance with some embodiments of the present disclosure performed on tissue sections obtained from samples of: 5A: skin cancer, 5B: ovarian cancer, 5C: colorectal cancer, 5D: large intestine cancer, 5E: cervical cancer, and 5F: glioblastoma. In each sample, regions enriched in aneuploidy are encircled in the right panel (*e.g.,* Copy: "Anu," "Dip," "n/a"), and corresponding clusters of features in the two-dimensional array of features are similarly encircled in the left panel (*e.g.,* Cluster). Generally, clusters of features could be used to discriminate between regions of aneuploidy and regions of diploidy, as particularly illustrated in Figures 5A, 5B, 5C, 5E, and 5F. These examples show that the systems and methods of the present disclosure provide a useful tool for performing digital pathology independent of or concurrently with analysis by a trained pathologist.

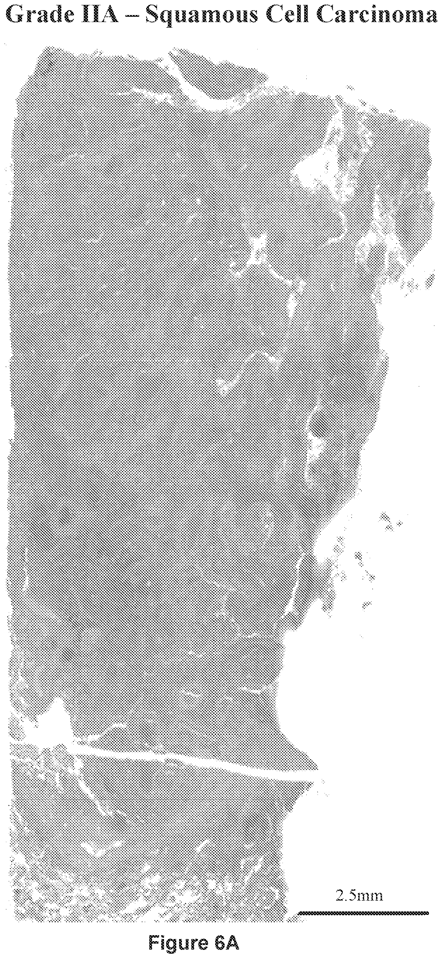

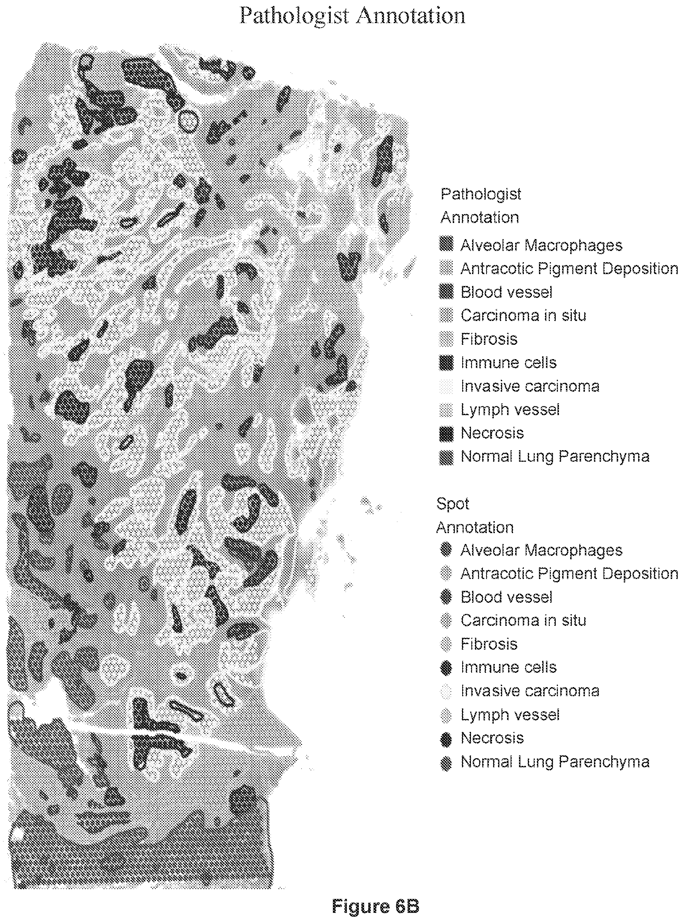

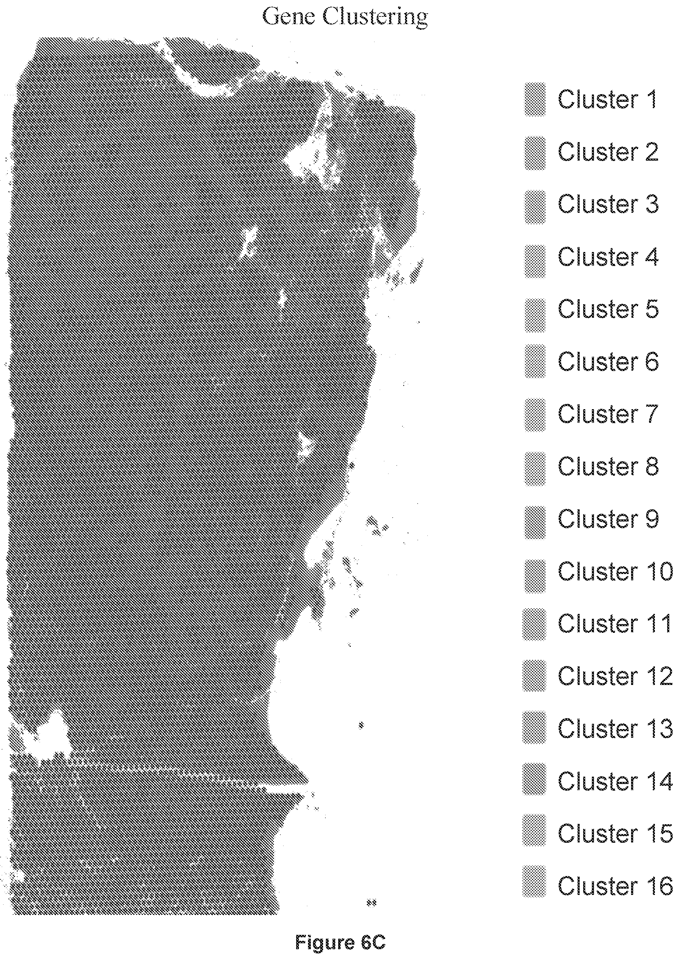

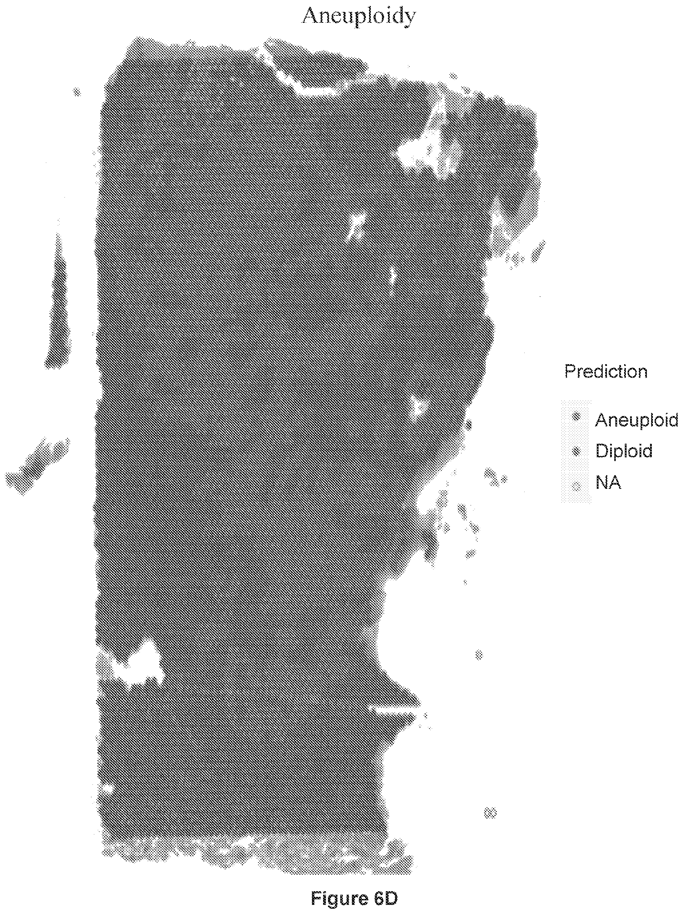

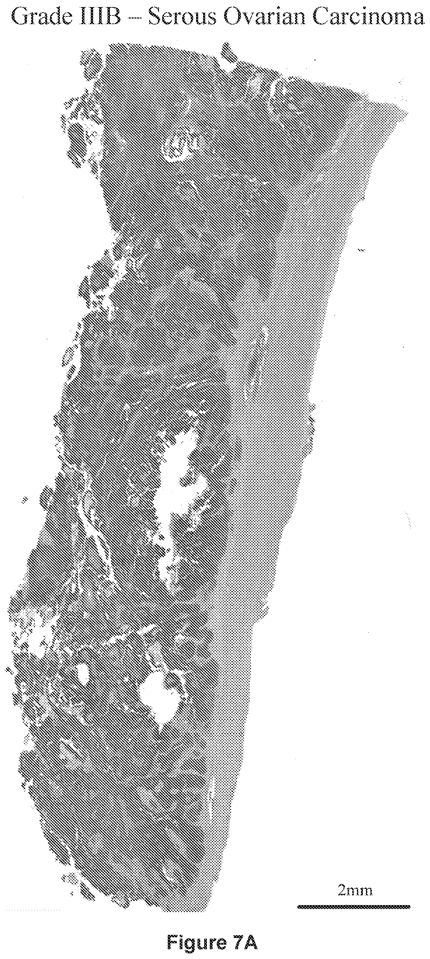

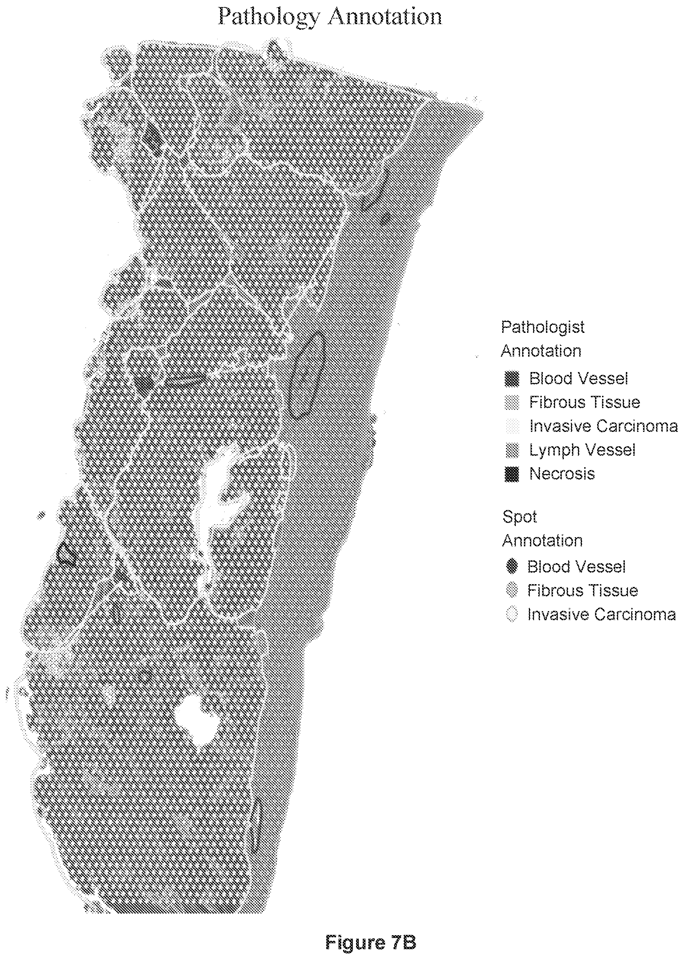

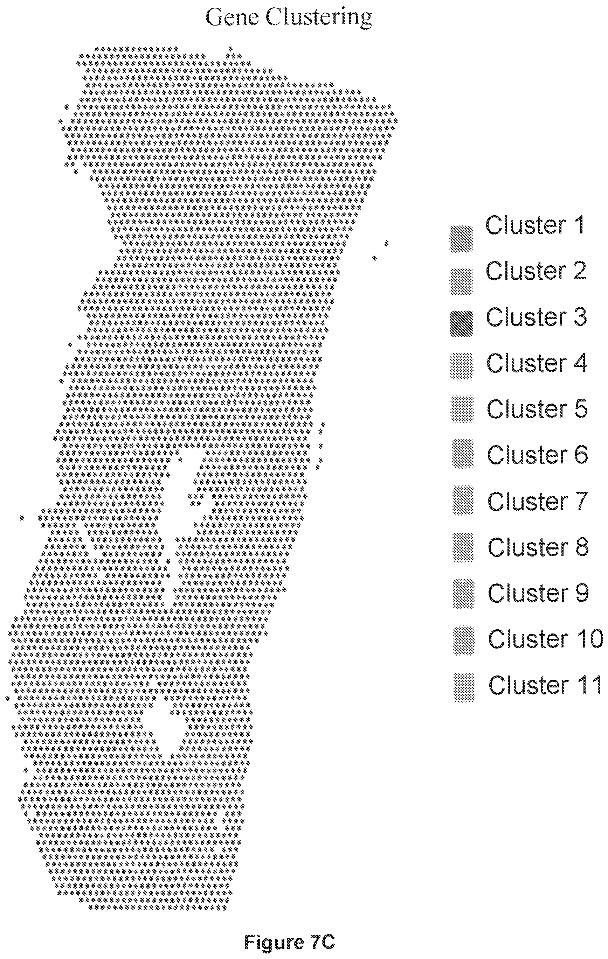

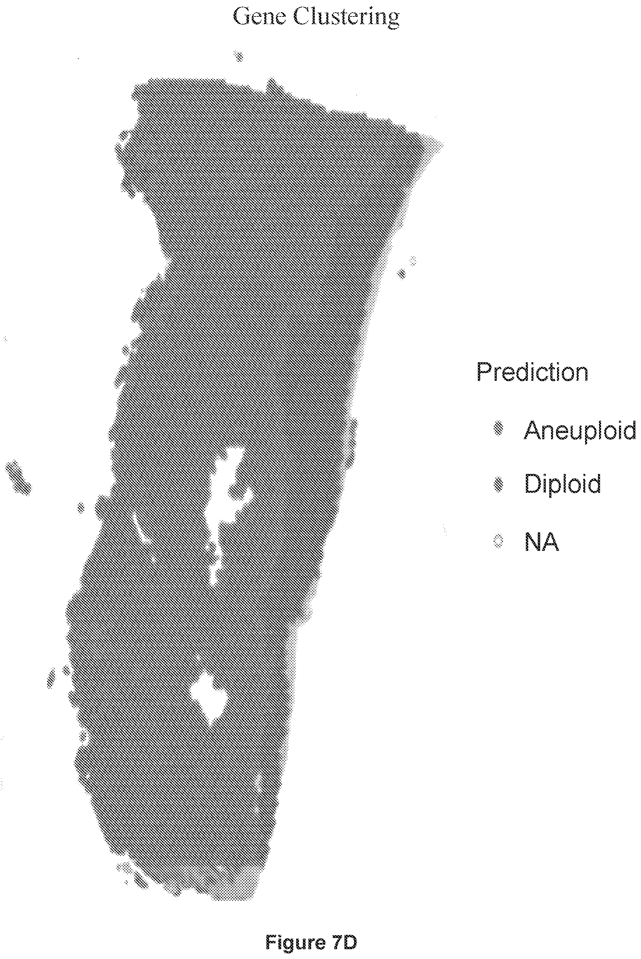

Figures 6A, 6B, 6C, 6D, 7A, 7B, 7C, and 7D illustrate a method in accordance with some embodiments of the present disclosure performed on tissue sections obtained from samples of human lung cancer (Figs. 6A, 6B, 6C, and 6D) and human ovarian cancer (Figs. 7A, 7B, 7C, and 7D).

The human tumor FFPE tissue sections (Grade IIA squamous cell carcinoma of the lung and Grade IIIB serous ovarian carcinoma) were spatially profiled using Visium CytAssist and Visium CytAssist Spatial Gene Expression Kit for FFPE (10x Genomics User Guide CG000495). Briefly, tissue sections were mounted on glass slides, H&E stained, and imaged to select the target region for whole transcriptome analysis. Human probe panels comprising about 3 pairs of specific probes for each targeted gene were added to the tissue sections and left to hybridize in the presence of a ligase overnight. Following probe hybridization and ligation, the tissue sections were prepared for ligation product transfer to spatially barcoded Visium slides with 6.5 or 11 (mm²) Capture Areas. The ligated probes were transferred to the Visium array and captured via the capture domain of spatially barcoded capture probes. The barcoded ligation products were then amplified followed by a clean up SPRIselect step. The amplified ligation products were indexed via sample index PCR generating sequencing-ready libraries. The resulting sequencing reads from the sequencing libraries were aligned and overlaid with the corresponding tissue section image, enabling analysis of mRNA (see, gene clustering). Data visualizations were performed with the 10x Loupe Browser v6.3 desktop software.

Copy number variations (CNV) are known to drive tumorgenesis. Here, copy number karyotyping was performed using a modifed version of CopyKAT. CopyKAT has been used previously to analyze single cell RNA-seq data (see, Gao, Ruli, et al., "Delineating copy number and clonal substructure in human tumors from single-cell transcriptomes," Nature Biotechnology 39.5 (2021): 599-608). However, the input was modified to use the spatial barcode matrix in place of single cell RNA barcode matrix data. This modification in the inputs allows the identification of aneuploid regions within the studied tissue and allows for the differentiation of tumor and normal regions in each tissue section.

As is demonstrated in FIGS. 6 and 7, the regions marked as aneuploid were in high concordance with the pathologist annotated invasive carcinoma regions. These examples show that the systems and methods of the present disclosure provide a useful tool for performing digital pathology independent of or concurrently with analysis by a trained pathologist.

### REFERENCES CITED AND ALTERNATIVE EMBODIMENTS

The present invention can be implemented as a computer program product that comprises a computer program mechanism embedded in a nontransitory computer readable storage medium. For instance, the computer program product could contain the program modules shown in Figure 1. These program modules can be stored on a CD-ROM, DVD, magnetic disk storage product, USB key, or any other non-transitory computer readable data or program storage product.

The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims.

## Claims

1. A method of delineating a tissue sample of a subject into one or more regions that are **characterized by** an aneuploid state and one or more regions that are **characterized by** a diploid state, the method comprising:
at a computer system (100) comprising at least one processor (102) and a memory (111/112) storing at least one program for execution by the at least one processor, the at least one program comprising instructions for:
A) obtaining a plurality of nucleic acid sequence reads comprising 10,000 or more sequence reads, in electronic form, wherein:
each respective sequence read (122) includes (i) a corresponding spatial barcode (124) associating the respective sequence read with a feature (142) in a two-dimensional array of features comprising at least 500 features on a substrate in contact with the tissue sample for a period of time prior to obtaining the plurality of sequence reads and (ii) a unique molecular identifier (126), and
the plurality of sequence reads comprises sequence reads of all or portions of a plurality of nucleic acids representing 1000 or more different genomic regions in the genome of the subject across five or more different chromosomes;
B) using the plurality of sequence reads to determine a count data structure (130) comprising, for each different genomic region (132) represented by the plurality of nucleic acids, a respective UMI count (134) for each feature (142) in the two-dimensional array of features on the substrate having a positive UMI count;
C) determining, for each respective feature in the two-dimensional array of features, a respective bin count (144) for each respective bin in a plurality of bins spanning all or a portion of the genome of the subject corresponding to the respective feature, wherein each respective bin in the plurality of bins comprises multiple genomic regions in the 1000 or more different genomic regions;
D) determining a respective copy number state (146) of each respective feature in the two-dimensional array of features using the respective bin count for each respective bin in the respective plurality of bins corresponding to the respective feature; and
E) using the respective copy number state of each respective feature in the two-dimensional array of features to identify the one or more regions of the tissue sample that are **characterized by** an aneuploid state and the one or more regions of the tissue sample that are **characterized by** the diploid state.

2. The method of claim 1, wherein the obtaining A) comprises sequencing of the two-dimensional array of features on the substrate.

3. The method claim 1 or 2, wherein
the plurality of nucleic acids represent 2000 or more different genomic regions, or between 2000 and 10,000 genomic regions, or
the plurality of sequence reads comprises 50,000 or more sequence reads, 100,000 or more sequence reads, or 1 x 10⁶ or more sequence reads.

4. The method of any one of claims 1-3, wherein
each respective feature includes 10 or more capture probes, 20 or more capture probes, 50 or more capture probes, 100 or more capture probes, 1000 or more capture probes, 2000 or more capture probes, 10,000 or more capture probes, or 100,000 or more capture probes,
optionally wherein each respective capture probe in the respective feature includes a poly-A sequence or a poly-T sequence and the corresponding spatial barcode for the respective feature that is incorporated into sequence reads in the plurality of sequence reads associated with the respective feature.

5. The method of claim 4, wherein
each respective capture probe in the respective feature includes the same spatial barcode, or
each respective capture probe in the respective feature includes a unique molecule identifier that is incorporated into sequence reads in the plurality of sequence reads associated with the respective capture probe.

6. The method of any one of claims 1-5, wherein
the tissue sample is a sectioned tissue sample having a depth of 100 microns or less, the obtaining A) comprises genome-wide transcript coverage obtained from a gene expression workflow, or
the method further comprises, prior to the determining C), transforming the count data structure using a log-Freeman-Tukey transform.

7. The method of any one of claims 1-6, the method further comprising:
i) clustering the count data structure across the plurality of bins to arrive at a plurality of clusters of features in the two-dimensional array of features,
ii) determining a corresponding cluster consensus profile across the 1000 or more different genomic regions in the genome of the subject for each cluster in the plurality of clusters,
iii) identifying a confident normal cluster in the plurality of clusters of features as a ground-state copy number based on a variance with respect to the corresponding consensus profile for the first cluster as compared to a variance with respect to the corresponding consensus profile for each other cluster in the plurality of clusters,
iv) performing copy number evaluation for each respective cluster in the plurality of clusters using the corresponding consensus profile of the respective cluster,
v) clustering the plurality of features in the two-dimensional array of features into a first cluster and a second cluster,
vi) identifying each feature in the first cluster as one of aneuploid or diploid and each feature in the second cluster as the of aneuploid or diploid based on an enrichment within the first cluster or the second cluster of features in the confident normal cluster, and
vii) marking each feature in the two-dimensional array of features as one aneuploid or diploid based on the identifying vi).

8. The method of any one of claims 1-7, wherein the determining D) calculates, for each respective feature in the two-dimensional array of features, the respective copy number state, across the corresponding plurality of bins of the respective feature, using a stochastic modeling algorithm and the respective bin count for each respective bin in the respective plurality of bins corresponding to the respective feature, optionally wherein the stochastic modeling algorithm is a Hidden Markov Model algorithm.

9. The method of any one of claims 1-7, wherein the determining D) calculates, for each respective feature in the two-dimensional array of features, the respective copy number state, across the corresponding plurality of bins of the respective feature, using a circular binary segmentation algorithm and the respective bin count for each respective bin in the respective plurality of bins corresponding to the respective feature.

10. The method of any one of claims 1-9, the method further comprising merging together adjacent bins that have the same copy number state for a respective feature.

11. The method of any one of claims 1-10, the method further comprising identifying a region in the one or more regions **characterized by** the aneuploid state as tumor.

12. The method of any one of claims 1-11, the method further comprising using the one or more regions of the tissue sample that are **characterized by** the aneuploid state and the one or more regions of the tissue sample that are **characterized by** the diploid state to identify a stage of a cancer in the subject.

13. The method of any one of claims 1-12, wherein the plurality of sequence reads comprises more than 50 sequence reads for all or portions of a plurality of nucleic acids representing 5000 or more different genomic regions in the genome of the subject across ten or more different chromosomes.

14. A computer system (100) for delineating a tissue sample of a subject into one or more regions that are **characterized by** an aneuploid state and one or more regions that are **characterized by** a diploid state, the computer system comprising:
one or more processors (102); and
memory (111/112) addressable by the one or more processors, the memory storing at least one program for execution by the one or more processors, the at least one program comprising instructions for:
A) obtaining a plurality of nucleic acid sequence reads comprising 10,000 or more sequence reads, in electronic form, wherein:
each respective sequence read (122) includes (i) a corresponding spatial barcode (124) associating the respective sequence read with a feature (142) in a two-dimensional array of features comprising at least 500 features on a substrate in contact with the tissue sample for a period of time prior to obtaining the plurality of sequence reads and (ii) a unique molecular identifier (126), and
the plurality of sequence reads comprises sequence reads of all or portions of a plurality of nucleic acids representing 1000 or more different genomic regions in the genome of the subject across five or more different chromosomes;
B) using the plurality of sequence reads to determine a count data structure (130) comprising, for each different genomic region (132) represented by the plurality of nucleic acids, a respective UMI count (134) for each feature (142) in the two-dimensional array of features on the substrate having a positive UMI count;
C) determining, for each respective feature in the two-dimensional array of features, a respective bin count (144) for each respective bin in a plurality of bins spanning all or a portion of the genome of the subject corresponding to the respective feature, wherein each respective bin in the plurality of bins comprises multiple genomic regions in the 1000 or more different genomic regions;
D) determining a respective copy number state (146) of each respective feature in the two-dimensional array of features using the respective bin count for each respective bin in the respective plurality of bins corresponding to the respective feature; and
E) using the respective copy number state of each respective feature in the two-dimensional array of features to identify the one or more regions of the tissue sample that are **characterized by** an aneuploid state and the one or more regions of the tissue sample that are **characterized by** the diploid state.

15. A non-transitory computer readable storage medium, wherein the non-transitory computer readable storage medium stores instructions, which when executed by a computer system (100), cause the computer system to perform a method for delineating a tissue sample of a subject into one or more regions that are **characterized by** an aneuploid state and one or more regions that are **characterized by** a diploid state, the method comprising:
A) obtaining a plurality of nucleic acid sequence reads comprising 10,000 or more sequence reads, in electronic form, wherein:
each respective sequence read (122) includes (i) a corresponding spatial barcode (124) associating the respective sequence read with a feature (142) in a two-dimensional array of features comprising at least 500 features on a substrate in contact with the tissue sample for a period of time prior to obtaining the plurality of sequence reads and (ii) a unique molecular identifier (126), and
the plurality of sequence reads comprises sequence reads of all or portions of a plurality of nucleic acids representing 1000 or more different genomic regions in the genome of the subject across five or more different chromosomes;
B) using the plurality of sequence reads to determine a count data structure (130) comprising, for each different genomic region (132) represented by the plurality of nucleic acids, a respective UMI count (134) for each feature (142) in the two-dimensional array of features on the substrate having a positive UMI count;
C) determining, for each respective feature in the two-dimensional array of features, a respective bin count (144) for each respective bin in a plurality of bins spanning all or a portion of the genome of the subject corresponding to the respective feature, wherein each respective bin in the plurality of bins comprises multiple genomic regions in the 1000 or more different genomic regions;
D) determining a respective copy number state (146) of each respective feature in the two-dimensional array of features using the respective bin count for each respective bin in the respective plurality of bins corresponding to the respective feature; and
E) using the respective copy number state of each respective feature in the two-dimensional array of features to identify the one or more regions of the tissue sample that are **characterized by** an aneuploid state and the one or more regions of the tissue sample that are **characterized by** the diploid state.

## Patentansprüche

1. Verfahren zum Abgrenzen einer Gewebeprobe eines Probanden in eine oder mehrere Regionen, die durch einen aneuploiden Zustand gekennzeichnet sind, und eine oder mehrere Regionen, die durch einen diploiden Zustand gekennzeichnet sind, das Verfahren umfassend:
an einem Computersystem (100), das mindestens einen Prozessor (102) und einen Speicher (111/112), der mindestens ein Programm zum Ausführen durch den mindestens einen Prozessor speichert, umfasst, wobei das mindestens eine Programm Anweisungen umfasst für:
A) das Erhalten einer Mehrzahl von Nukleinsäuresequenz-Reads, umfassend 10.000 oder mehr Sequenz-Reads, in elektronischer Form, wobei:
jeder jeweilige Sequenz-Read (122) (i) einen entsprechenden räumlichen Barcode (124), der den jeweiligen Sequenz-Read mit einem Merkmal (142) in einem zweidimensionalen Array von Merkmalen assoziiert, das mindestens 500 Merkmale auf einem Substrat umfasst, das vor dem Erhalten der Mehrzahl von Sequenz-Reads für einen Zeitraum mit der Gewebeprobe in Kontakt kommt, und (ii) eine eindeutige molekulare Kennung (126) aufweist, und
wobei die Mehrzahl von Sequenz-Reads Sequenz-Reads von allen oder Teilen einer Mehrzahl von Nukleinsäuren umfasst, die 1000 oder mehr verschiedene genomische Regionen in dem Genom des Probanden für fünf oder mehr verschiedene Chromosomen darstellen;
B) das Verwenden der Mehrzahl von Sequenz-Reads, um eine Zähldatenstruktur (130) zu bestimmen, die für jede unterschiedliche genomische Region (132), die durch die Mehrzahl von Nukleinsäuren dargestellt wird, eine jeweilige UMI-Zählung (134) für jedes Merkmal (142) in dem zweidimensionalen Array von Merkmalen auf dem Substrat, das eine positive UMI-Zählung aufweist, umfasst;
C) das Bestimmen, für jedes jeweilige Merkmal in dem zweidimensionalen Array von Merkmalen, einer jeweiligen Bin-Zählung (144) für jeden jeweiligen Bin in einer Mehrzahl von Bins, die das gesamte oder einen Teil des Genoms des Probanden, der dem jeweiligen Merkmal entspricht, abdecken, wobei jeder jeweilige Bin in der Mehrzahl von Bins mehrere genomische Regionen in den 1000 oder mehr verschiedenen genomischen Regionen umfasst;
D) das Bestimmen eines jeweiligen Kopienzahlzustands (146) jedes jeweiligen Merkmals in dem zweidimensionalen Array von Merkmalen unter Verwendung der jeweiligen Bin-Zählung für jeden jeweiligen Bin in der jeweiligen Mehrzahl von Bins, die dem jeweiligen Merkmal entsprechen; und
E) das Verwenden des jeweiligen Kopienzahlzustands jedes jeweiligen Merkmals in dem zweidimensionalen Array von Merkmalen zum Identifizieren der einen oder der mehreren Regionen der Gewebeprobe, die durch einen aneuploiden Zustand gekennzeichnet sind, und der einen oder der mehreren Regionen der Gewebeprobe, die durch den diploiden Zustand gekennzeichnet sind.

2. Verfahren nach Anspruch 1, wobei das Erhalten von A) das Sequenzieren des zweidimensionalen Arrays von Merkmalen auf dem Substrat umfasst.

3. Verfahren nach Anspruch 1 oder 2, wobei:
die Mehrzahl von Nukleinsäuren 2000 oder mehr verschiedene genomische Regionen oder zwischen 2000 und 10.000 genomische Regionen darstellen, oder
die Mehrzahl von Sequenz-Reads 50.000 oder mehr Sequenz-Reads, 100.000 oder mehr Sequenz-Reads oder 1 x 10⁶ oder mehr Sequenz-Reads umfasst.

4. Verfahren nach einem der Ansprüche 1 bis 3, wobei
jedes jeweilige Merkmal 10 oder mehr Fängersonden, 20 oder mehr Fängersonden, 50 oder mehr Fängersonden, 100 oder mehr Fängersonden, 1000 oder mehr Fängersonden, 2000 oder mehr Fängersonden, 10.000 oder mehr Fängersonden oder 100.000 oder mehr Fängersonden aufweist,
optional, wobei jede jeweilige Fängersonde in dem jeweiligen Merkmal eine Poly-A-Sequenz oder eine Poly-T-Sequenz und den entsprechenden räumlichen Barcode für das jeweilige Merkmal aufweist, der in Sequenz-Reads in der Mehrzahl von Sequenz-Reads, die mit dem jeweiligen Merkmal assoziiert sind, eingebunden ist.

5. Verfahren nach Anspruch 4, wobei
jede jeweilige Fängersonde in dem jeweiligen Merkmal den gleichen räumlichen Barcode aufweist, oder
wobei jede jeweilige Fängersonde in dem jeweiligen Merkmal eine eindeutige molekulare Kennung aufweist, die in Sequenz-Reads in der Mehrzahl von Sequenz-Reads, die mit der jeweiligen Fängersonde assoziiert ist, eingebunden ist.

6. Verfahren nach einem der Ansprüche 1 bis 5, wobei
die Gewebeprobe eine geschnittene Gewebeprobe mit einer Tiefe von 100 Mikrometern oder weniger ist, das Erhalten von A) eine genomweite Transkriptabdeckung umfasst, die aus einem Genexpressions-Arbeitsablauf erhalten wird, oder
das Verfahren ferner vor dem Bestimmen von C) das Transformieren der Zähldatenstruktur unter Verwendung einer Log-Freeman-Tukey-Transformation umfasst.

7. Verfahren nach einem der Ansprüche 1 bis 6, das Verfahren ferner umfassend:
i) Clustern der Zähldatenstruktur für die Mehrzahl von Bins zum Erreichen einer Mehrzahl von Clustern der Merkmale in dem zweidimensionalen Array von Merkmalen,
ii) Bestimmen eines entsprechenden Konsensus-Profils des Clusters für die 1000 oder mehr unterschiedlichen genomischen Regionen in dem Genom des Probanden für jeden Cluster in der Mehrzahl von Clustern,
iii) Identifizieren eines zuverlässigen normalen Clusters in der Mehrzahl von Clustern der Merkmale als ein Grundzustand der Kopienzahl basierend auf einer Varianz in Bezug auf das entsprechende Konsensus-Profil für den ersten Cluster im Vergleich zu einer Varianz in Bezug auf das entsprechende Konsensus-Profil für jeden anderen Cluster in der Mehrzahl von Clustern,
iv) Durchführen einer Kopienzahlbewertung für jeden jeweiligen Cluster in der Mehrzahl von Clustern unter Verwendung des entsprechenden Konsensus-Profils des jeweiligen Clusters,
v) Clustern der Mehrzahl von Merkmalen in dem zweidimensionalen Array von Merkmalen zu einem ersten Cluster und einem zweiten Cluster,
vi) Identifizieren jedes Merkmals in dem ersten Cluster als aneuploid oder diploid und jedes Merkmals in dem zweiten Cluster als aneuploid oder diploid basierend auf einer Anreicherung innerhalb des ersten Clusters oder des zweiten Clusters von Merkmalen in dem zuverlässigen normalen Cluster, und
vii) Markieren jedes Merkmals in dem zweidimensionalen Array von Merkmalen als entweder aneuploid oder diploid basierend auf der Identifizierung von vi).

8. Verfahren nach einem der Ansprüche 1 bis 7, wobei das Bestimmen von D) für jedes jeweilige Merkmal in dem zweidimensionalen Array von Merkmalen den jeweiligen Kopienzahlzustand, für die entsprechende Mehrzahl von Bins des jeweiligen Merkmals, unter Verwendung eines stochastischen Modellierungsalgorithmus und der jeweiligen Bin-Zählung für jedes jeweilige Bin in der jeweiligen Mehrzahl von Bins, die dem jeweiligen Merkmal entspricht, berechnet, wobei der stochastische Modellierungsalgorithmus optional ein Hidden-Markov-Model-Algorithmus ist.

9. Verfahren nach einem der Ansprüche 1 bis 7, wobei das Bestimmen von D) für jedes jeweilige Merkmal in dem zweidimensionalen Array von Merkmalen den jeweiligen Kopienzahlzustand, für die entsprechende Mehrzahl von Bins des jeweiligen Merkmals, unter Verwendung eines zirkulären binären Segmentierungsalgorithmus und der jeweiligen Bin-Zählung für jedes jeweilige Bin in der jeweiligen Mehrzahl von Bins, die dem jeweiligen Merkmal entspricht, berechnet.

10. Verfahren nach einem der Ansprüche 1 bis 9, wobei das Verfahren ferner das Zusammenführen benachbarter Bins umfasst, die den gleichen Kopienzahlzustand für ein jeweiliges Merkmal aufweisen.

11. Verfahren nach einem der Ansprüche 1 bis 10, wobei das Verfahren ferner das Identifizieren einer Region in der einen oder den mehreren Regionen, die durch den aneuploiden Zustand gekennzeichnet sind, als Tumor umfasst.

12. Verfahren nach einem der Ansprüche 1 bis 11, wobei das Verfahren ferner die Verwendung der einen oder der mehreren Regionen der Gewebeprobe, die durch den aneuploiden Zustand gekennzeichnet sind, und der einen oder der mehreren Regionen der Gewebeprobe, die durch den diploiden Zustand gekennzeichnet sind, umfasst, um ein Krebsstadium bei dem Probanden zu identifizieren.

13. Verfahren nach einem der Ansprüche 1-12, wobei die Mehrzahl von Sequenz-Reads mehr als 50 Sequenz-Reads für alle oder Teile einer Mehrzahl von Nukleinsäuren umfasst, die 5000 oder mehr verschiedene genomische Regionen in dem Genom des Probanden für zehn oder mehr verschiedene Chromosomen darstellen.

14. Computersystem (100) zum Abgrenzen einer Gewebeprobe eines Probanden in eine oder mehrere Regionen, die durch einen aneuploiden Zustand gekennzeichnet sind, und eine oder mehrere Regionen, die durch einen diploiden Zustand gekennzeichnet sind, das Computersystem umfassend:
einen oder mehrere Prozessoren (102); und
einen Speicher (111/112), der durch den einen oder die mehreren Prozessoren adressierbar ist, wobei der Speicher mindestens ein Programm zum Ausführen durch den einen oder die mehreren Prozessoren speichert, wobei das mindestens eine Programm Anweisungen umfasst für:
A) das Erhalten einer Mehrzahl von Nukleinsäuresequenz-Reads, umfassend 10.000 oder mehr Sequenz-Reads, in elektronischer Form, wobei:
jeder jeweilige Sequenz-Read (122) (i) einen entsprechenden räumlichen Barcode (124), der den jeweiligen Sequenz-Read mit einem Merkmal (142) in einem zweidimensionalen Array von Merkmalen assoziiert, das mindestens 500 Merkmale auf einem Substrat umfasst, das vor dem Erhalten der Mehrzahl von Sequenz-Reads für einen Zeitraum mit der Gewebeprobe in Kontakt kommt, und (ii) eine eindeutige molekulare Kennung (126) aufweist, und
wobei die Mehrzahl von Sequenz-Reads Sequenz-Reads von allen oder Teilen einer Mehrzahl von Nukleinsäuren umfasst, die 1000 oder mehr verschiedene genomische Regionen in dem Genom des Probanden für fünf oder mehr verschiedene Chromosomen darstellen;
B) das Verwenden der Mehrzahl von Sequenz-Reads, um eine Zähldatenstruktur (130) zu bestimmen, die für jede unterschiedliche genomische Region (132), die durch die Mehrzahl von Nukleinsäuren dargestellt wird, eine jeweilige UMI-Zählung (134) für jedes Merkmal (142) in dem zweidimensionalen Array von Merkmalen auf dem Substrat, das eine positive UMI-Zählung aufweist, umfasst;
C) das Bestimmen, für jedes jeweilige Merkmal in dem zweidimensionalen Array von Merkmalen, einer jeweiligen Bin-Zählung (144) für jeden jeweiligen Bin in einer Mehrzahl von Bins, die das gesamte oder einen Teil des Genoms des Probanden, der dem jeweiligen Merkmal entspricht, abdecken, wobei jeder jeweilige Bin in der Mehrzahl von Bins mehrere genomische Regionen in den 1000 oder mehr verschiedenen genomischen Regionen umfasst;
D) das Bestimmen eines jeweiligen Kopienzahlzustands (146) jedes jeweiligen Merkmals in dem zweidimensionalen Array von Merkmalen unter Verwendung der jeweiligen Bin-Zählung für jeden jeweiligen Bin in der jeweiligen Mehrzahl von Bins, die dem jeweiligen Merkmal entsprechen; und
E) das Verwenden des jeweiligen Kopienzahlzustands jedes jeweiligen Merkmals in dem zweidimensionalen Array von Merkmalen zum Identifizieren der einen oder der mehreren Regionen der Gewebeprobe, die durch einen aneuploiden Zustand gekennzeichnet sind, und der einen oder der mehreren Regionen der Gewebeprobe, die durch den diploiden Zustand gekennzeichnet sind.

15. Nichtflüchtiges computerlesbares Speichermedium, wobei das nichtflüchtige computerlesbare Speichermedium Anweisungen speichert, die, wenn sie durch ein Computersystem (100) ausgeführt werden, das Computersystem veranlassen, ein Verfahren zum Abgrenzen einer Gewebeprobe eines Probanden in eine oder mehrere Regionen, die durch einen aneuploiden Zustand gekennzeichnet sind, und eine oder mehrere Regionen, die durch einen diploiden Zustand gekennzeichnet sind, durchzuführen, das Verfahren umfassend:
A) Erhalten einer Mehrzahl von Nukleinsäuresequenz-Reads, umfassend 10.000 oder mehr Sequenz-Reads, in elektronischer Form, wobei:
jeder jeweilige Sequenz-Read (122) (i) einen entsprechenden räumlichen Barcode (124), der den jeweiligen Sequenz-Read mit einem Merkmal (142) in einem zweidimensionalen Array von Merkmalen assoziiert, das mindestens 500 Merkmale auf einem Substrat umfasst, das vor dem Erhalten der Mehrzahl von Sequenz-Reads für einen Zeitraum mit der Gewebeprobe in Kontakt kommt, und (ii) eine eindeutige molekulare Kennung (126) aufweist, und
wobei die Mehrzahl von Sequenz-Reads Sequenz-Reads von allen oder Teilen einer Mehrzahl von Nukleinsäuren umfasst, die 1000 oder mehr verschiedene genomische Regionen in dem Genom des Probanden für fünf oder mehr verschiedene Chromosomen darstellen;
B) Verwenden der Mehrzahl von Sequenz-Reads, um eine Zähldatenstruktur (130) zu bestimmen, die für jede unterschiedliche genomische Region (132), die durch die Mehrzahl von Nukleinsäuren dargestellt wird, eine jeweilige UMI-Zählung (134) für jedes Merkmal (142) in dem zweidimensionalen Array von Merkmalen auf dem Substrat, das eine positive UMI-Zählung aufweist, umfasst;
C) Bestimmen, für jedes jeweilige Merkmal in dem zweidimensionalen Array von Merkmalen, einer jeweiligen Bin-Zählung (144) für jeden jeweiligen Bin in einer Mehrzahl von Bins, die das gesamte oder einen Teil des Genoms des Probanden, der dem jeweiligen Merkmal entspricht, abdecken, wobei jeder jeweilige Bin in der Mehrzahl von Bins mehrere genomische Regionen in den 1000 oder mehr verschiedenen genomischen Regionen umfasst;
D) Bestimmen eines jeweiligen Kopienzahlzustands (146) jedes jeweiligen Merkmals in dem zweidimensionalen Array von Merkmalen unter Verwendung der jeweiligen Bin-Zählung für jeden jeweiligen Bin in der jeweiligen Mehrzahl von Bins, die dem jeweiligen Merkmal entsprechen; und
E) Verwenden des jeweiligen Kopienzahlzustands jedes jeweiligen Merkmals in dem zweidimensionalen Array von Merkmalen zum Identifizieren der einen oder der mehreren Regionen der Gewebeprobe, die durch einen aneuploiden Zustand gekennzeichnet sind, und der einen oder der mehreren Regionen der Gewebeprobe, die durch den diploiden Zustand gekennzeichnet sind.

## Revendications

1. Procédé de délimitation d'un échantillon de tissu d'un sujet en une ou plusieurs régions qui sont **caractérisées par** un état aneuploïde et une ou plusieurs régions qui sont **caractérisées par** un état diploïde, le procédé comprenant :
au niveau d'un système informatique (100) comprenant au moins un processeur (102) et une mémoire (111/112) stockant au moins un programme pour l'exécution par l'au moins un processeur, l'au moins un programme comprenant des instructions pour :
A) l'obtention d'une pluralité de lectures de séquences d'acides nucléiques comprenant 10 000 lectures de séquences ou plus, sous forme électronique, dans lequel :
chaque lecture de séquence (122) respective comprend (i) un code à barres spatial (124) correspondant associant la lecture de séquence respective à une caractéristique (142) dans un réseau bidimensionnel de caractéristiques comprenant au moins 500 caractéristiques sur un substrat en contact avec l'échantillon de tissu pendant une période de temps avant l'obtention de la pluralité de lectures de séquence et (ii) un identifiant moléculaire unique (126), et
la pluralité de lectures de séquence comprend des lectures de séquence de la totalité ou de portions d'une pluralité d'acides nucléiques représentant 1000 régions génomiques différentes ou plus dans le génome du sujet sur cinq chromosomes différents ou plus ;
B) l'utilisation de la pluralité de lectures de séquence pour déterminer une structure de données de comptage (130) comprenant, pour chaque région génomique différente (132) représentée par la pluralité d'acides nucléiques, un comptage d'UMI (134) respectif pour chaque caractéristique (142) dans le réseau bidimensionnel de caractéristiques sur le substrat ayant un comptage d'UMI positif ;
C) la détermination, pour chaque caractéristique respective dans le réseau bidimensionnel de caractéristiques, d'un comptage de cases (144) respectif pour chaque case respective dans une pluralité de cases couvrant la totalité ou une portion du génome du sujet correspondant à la caractéristique respective, dans lequel chaque case respective dans la pluralité de cases comprend de multiples régions génomiques dans les 1000 régions génomiques différentes ou plus ;
D) la détermination d'un état de nombre de copies (146) respectif de chaque caractéristique respective dans le réseau bidimensionnel de caractéristiques en utilisant le comptage de cases respectif pour chaque case respective dans la pluralité respective de cases correspondant à la caractéristique respective ; et
E) l'utilisation de l'état de nombre de copies respectif de chaque caractéristique respective dans le réseau bidimensionnel de caractéristiques pour identifier les une ou plusieurs régions de l'échantillon de tissu qui sont **caractérisées par** un état aneuploïde et les une ou plusieurs régions de l'échantillon de tissu qui sont **caractérisées par** l'état diploïde.

2. Procédé selon la revendication 1, dans lequel l'obtention A) comprend le séquençage du réseau bidimensionnel de caractéristiques sur le substrat.

3. Procédé selon la revendication 1 ou 2, dans lequel
la pluralité d'acides nucléiques représente 2000 régions génomiques différentes ou plus, ou entre 2000 et 10 000 régions génomiques, ou
la pluralité de lectures de séquence comprend 50 000 lectures de séquence ou plus, 100 000 lectures de séquence ou plus, ou 1 x 10⁶ lectures de séquence ou plus.

4. Procédé selon l'une quelconque des revendications 1 à 3, dans lequel
chaque caractéristique respective comprend 10 sondes de capture ou plus, 20 sondes de capture ou plus, 50 sondes de capture ou plus, 100 sondes de capture ou plus, 1000 sondes de capture ou plus, 2000 sondes de capture ou plus, 10 000 sondes de capture ou plus, ou 100 000 sondes de capture ou plus,
facultativement dans lequel chaque sonde de capture respective dans la caractéristique respective comporte une séquence poly-A ou une séquence poly-T et le code à barres spatial correspondant pour la caractéristique respective qui est incorporé en lectures de séquence dans la pluralité de lectures de séquence associées à la caractéristique respective.

5. Procédé selon la revendication 4, dans lequel
chaque sonde de capture respective dans la caractéristique respective comporte le même code à barres spatial, ou
chaque sonde de capture respective dans la caractéristique respective comporte un identifiant moléculaire unique qui est incorporé dans des lectures de séquence dans la pluralité de lectures de séquence associées à la sonde de capture respective.

6. Procédé selon l'une quelconque des revendications 1 à 5, dans lequel
l'échantillon de tissu est un échantillon de tissu sectionné ayant une profondeur de 100 microns ou moins, l'obtention A) comprend une couverture de transcrits à l'échelle du génome obtenue à partir d'un flux de travail d'expression génique, ou
le procédé comprend en outre, avant la détermination C), la transformation de la structure de données de comptage en utilisant une transformation log-Freeman-Tukey.

7. Procédé selon l'une quelconque des revendications 1 à 6, le procédé comprenant en outre :
i) le regroupement de la structure de données de comptage sur la pluralité de cases pour arriver à une pluralité de groupes de caractéristiques dans le réseau bidimensionnel de caractéristiques,
ii) la détermination d'un profil consensuel de groupe correspondant sur les 1000 régions génomiques différentes ou plus dans le génome du sujet pour chaque groupe dans la pluralité de groupes,
iii) l'identification d'un groupe normal fiable dans la pluralité de groupes de caractéristiques comme un nombre de copies à l'état fondamental sur la base d'une variance par rapport au profil consensuel correspondant pour le premier groupe comparativement à une variance par rapport au profil consensuel correspondant pour chaque autre groupe dans la pluralité de groupes,
iv) la réalisation d'une évaluation du nombre de copies pour chaque groupe respectif dans la pluralité de groupes en utilisant le profil consensuel correspondant du groupe respectif,
v) le regroupement de la pluralité de caractéristiques dans le réseau bidimensionnel de caractéristiques en un premier groupe et un deuxième groupe,
vi) l'identification de chaque caractéristique dans le premier groupe comme l'un parmi aneuploïde ou diploïde et de chaque caractéristique dans le deuxième groupe comme l'un parmi aneuploïde ou diploïde sur la base d'un enrichissement, au sein du premier groupe ou du deuxième groupe de caractéristiques dans le groupe normal fiable, et
vii) le marquage de chaque caractéristique dans le réseau bidimensionnel de caractéristiques comme aneuploïde ou diploïde sur la base de l'identification vi).

8. Procédé selon l'une quelconque des revendications 1 à 7, dans lequel la détermination D) calcule, pour chaque caractéristique respective dans le réseau bidimensionnel de caractéristiques, l'état de nombre de copies respectif, sur la pluralité correspondante de cases de la caractéristique respective, en utilisant un algorithme de modélisation stochastique et le comptage de cases respectif pour chaque case respective dans la pluralité respective de cases correspondant à la caractéristique respective, facultativement dans lequel l'algorithme de modélisation stochastique est un algorithme de modèle de Markov caché.

9. Procédé selon l'une quelconque des revendications 1 à 7, dans lequel la détermination D) calcule, pour chaque caractéristique respective dans le réseau bidimensionnel de caractéristiques, l'état de nombre de copies respectif, sur la pluralité correspondante de cases de la caractéristique respective, en utilisant un algorithme de segmentation binaire circulaire et le comptage de cases respectif pour chaque case respective dans la pluralité respective de cases correspondant à la caractéristique respective.

10. Procédé selon l'une quelconque des revendications 1 à 9, le procédé comprenant en outre la fusion de cases adjacentes qui ont le même état de nombre de copies pour une caractéristique respective.

11. Procédé selon l'une quelconque des revendications 1 à 10, le procédé comprenant en outre l'identification d'une région, dans les une ou plusieurs régions **caractérisées par** l'état aneuploïde, comme tumeur.

12. Procédé selon l'une quelconque des revendications 1 à 11, le procédé comprenant en outre l'utilisation des une ou plusieurs régions de l'échantillon de tissu qui sont **caractérisées par** l'état aneuploïde et des une ou plusieurs régions de l'échantillon de tissu qui sont **caractérisées par** l'état diploïde pour identifier un stade d'un cancer chez le sujet.

13. Procédé selon l'une quelconque des revendications 1 à 12, dans lequel la pluralité de lectures de séquence comprend plus de 50 lectures de séquence de la totalité ou de portions d'une pluralité d'acides nucléiques représentant 5 000 régions génomiques différentes ou plus dans le génome du sujet sur dix chromosomes différents ou plus.

14. Système informatique (100) de délimitation d'un échantillon de tissu d'un sujet en une ou plusieurs régions qui sont **caractérisées par** un état aneuploïde et une ou plusieurs régions qui sont **caractérisées par** un état diploïde, le système informatique comprenant :
un ou plusieurs processeurs (102) ; et
la mémoire (111/112) adressable par les un ou plusieurs processeurs, la mémoire stockant au moins un programme pour exécution par les un ou plusieurs processeurs, l'au moins un programme comprenant des instructions pour :
A) l'obtention d'une pluralité de lectures de séquences d'acides nucléiques comprenant 10 000 lectures de séquences ou plus, sous forme électronique, dans lequel :
chaque lecture de séquence (122) respective comprend (i) un code à barres spatial (124) correspondant associant la lecture de séquence respective à une caractéristique (142) dans un réseau bidimensionnel de caractéristiques comprenant au moins 500 caractéristiques sur un substrat en contact avec l'échantillon de tissu pendant une période de temps avant l'obtention de la pluralité de lectures de séquence et (ii) un identifiant moléculaire unique (126), et
la pluralité de lectures de séquence comprend des lectures de séquence de la totalité ou de portions d'une pluralité d'acides nucléiques représentant 1000 régions génomiques différentes ou plus dans le génome du sujet sur cinq chromosomes différents ou plus ;
B) l'utilisation de la pluralité de lectures de séquence pour déterminer une structure de données de comptage (130) comprenant, pour chaque région génomique différente (132) représentée par la pluralité d'acides nucléiques, un comptage d'UMI (134) respectif pour chaque caractéristique (142) dans le réseau bidimensionnel de caractéristiques sur le substrat ayant un comptage d'UMI positif ;
C) la détermination, pour chaque caractéristique respective dans le réseau bidimensionnel de caractéristiques, d'un comptage de cases (144) respectif pour chaque case respective dans une pluralité de cases couvrant la totalité ou une portion du génome du sujet correspondant à la caractéristique respective, dans lequel chaque case respective dans la pluralité de cases comprend de multiples régions génomiques dans les 1000 régions génomiques différentes ou plus ;
D) la détermination d'un état de nombre de copies (146) respectif de chaque caractéristique respective dans le réseau bidimensionnel de caractéristiques en utilisant le comptage de cases respectif pour chaque case respective dans la pluralité respective de cases correspondant à la caractéristique respective ; et
E) l'utilisation de l'état de nombre de copies respectif de chaque caractéristique respective dans le réseau bidimensionnel de caractéristiques pour identifier les une ou plusieurs régions de l'échantillon de tissu qui sont **caractérisées par** un état aneuploïde et les une ou plusieurs régions de l'échantillon de tissu qui sont **caractérisées par** l'état diploïde.

15. Support de stockage non transitoire lisible par ordinateur, dans lequel le support de stockage non transitoire lisible par ordinateur stocke des instructions qui, lorsqu'elles sont exécutées par un système informatique (100), amènent le système informatique à réaliser un procédé de délimitation d'un échantillon de tissu d'un sujet en une ou plusieurs régions qui sont **caractérisées par** un état aneuploïde et une ou plusieurs régions qui sont **caractérisées par** un état diploïde, le procédé comprenant :
A) l'obtention d'une pluralité de lectures de séquences d'acides nucléiques comprenant 10 000 lectures de séquences ou plus, sous forme électronique, dans lequel :
chaque lecture de séquence (122) respective comprend (i) un code à barres spatial (124) correspondant associant la lecture de séquence respective à une caractéristique (142) dans un réseau bidimensionnel de caractéristiques comprenant au moins 500 caractéristiques sur un substrat en contact avec l'échantillon de tissu pendant une période de temps avant l'obtention de la pluralité de lectures de séquence et (ii) un identifiant moléculaire unique (126), et
la pluralité de lectures de séquence comprend des lectures de séquence de la totalité ou de portions d'une pluralité d'acides nucléiques représentant 1000 régions génomiques différentes ou plus dans le génome du sujet sur cinq chromosomes différents ou plus ;
B) l'utilisation de la pluralité de lectures de séquence pour déterminer une structure de données de comptage (130) comprenant, pour chaque région génomique différente (132) représentée par la pluralité d'acides nucléiques, un comptage d'UMI (134) respectif pour chaque caractéristique (142) dans le réseau bidimensionnel de caractéristiques sur le substrat ayant un comptage d'UMI positif ;
C) la détermination, pour chaque caractéristique respective dans le réseau bidimensionnel de caractéristiques, d'un comptage de cases (144) respectif pour chaque case respective dans une pluralité de cases couvrant la totalité ou une portion du génome du sujet correspondant à la caractéristique respective, dans lequel chaque case respective dans la pluralité de cases comprend de multiples régions génomiques dans les 1000 régions génomiques différentes ou plus ;
D) la détermination d'un état de nombre de copies (146) respectif de chaque caractéristique respective dans le réseau bidimensionnel de caractéristiques en utilisant le comptage de cases respectif pour chaque case respective dans la pluralité respective de cases correspondant à la caractéristique respective ; et
E) l'utilisation de l'état de nombre de copies respectif de chaque caractéristique respective dans le réseau bidimensionnel de caractéristiques pour identifier les une ou plusieurs régions de l'échantillon de tissu qui sont **caractérisées par** un état aneuploïde et les une ou plusieurs régions de l'échantillon de tissu qui sont **caractérisées par** l'état diploïde.
